# EUROPEAN PATENT APPLICATION

(11) **EP 4 071 163 A1**
(43) Date of publication of application: **12.10.2022**
(21) Application number: 21747841.1
(22) Date of filing: 28.01.2021
(51) Int. Cl.: C07K 9/00, A61K 31/7088, A61K 47/54, A61K 48/00, C12N 15/113

(54) **NUCLEIC ACID COMPLEX AND PHARMACEUTICAL COMPOSITION CONTAINING SAME**

(30) Priority: 30.01.2020 JP 2020013997
(71) Applicant: Eisai R&D Management Co., Ltd, Bunkyo-ku, Tokyo 112-8088 (JP)
(72) Inventor: SUZUKI Yuta, Tsukuba-shi, Ibaraki 300-2635 (JP); YAMAZAKI Kazuto, Tsukuba-shi, Ibaraki 300-2635 (JP); KUBARA Kenji, Tsukuba-shi, Ibaraki 300-2635 (JP); TAMURA Tomohiko, Yokohama-shi, Kanagawa 236-0004 (JP); KUROTAKI Daisuke, Kumamoto-shi, Kumamoto 860-8555 (JP)
(74) Representative: Hoffmann Eitle
(86) International application number: PCT/JP2021/003085
(87) International publication number: WO 2021/153687

(57) **Abstract**

Disclosed is a nucleic acid complex or a pharmaceutically acceptable salt thereof, the nucleic acid complex represented by the formula (I), wherein X is CH₂ or O; Y is a sugar ligand having mannose or GalNAc; n is an integer of 1 to 8; and Z is a group comprising an oligonucleotide.

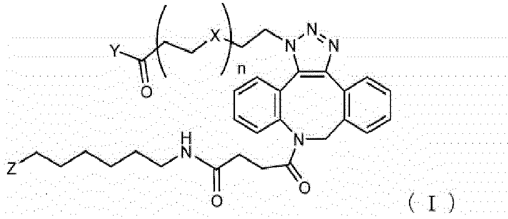

## Description

### Technical Field

The present invention relates to a nucleic acid complex, a pharmaceutical composition comprising the nucleic acid complex, and others.

### Background Art

Known as nucleic acid drugs are, for example, antisense drugs, decoy nucleic acids, ribozymes, aptamers, siRNA, miRNA, and messenger RNA (mRNA). Among them, nucleic acid drugs, which act on mRNA, are expected to be promising for clinical application to diseases previously considered to be intractable because of their high versatility that allows all kinds of genes in cells to be regulated. In other words, nucleic acid drugs are expected to be promising as next-generation drugs following small molecules and antibody drugs. However, nucleic acid drugs suffer from a problem of difficulty in delivering into cells, which are sites of action for nucleic acid drugs, because of, for example, the relatively large size of nucleic acid drugs, significantly low cell membrane permeability due to negative charges in the phosphate backbone, and decomposition of siRNA by nucleases in the blood (Non Patent Literature 1).

For the problem, a method of applying a delivery means to nucleic acid drugs is employed as a solution. Nucleic acid complexes (conjugated nucleic acids) formed of a target ligand and a nucleic acid have been reported as one of delivery means. Examples of the target ligand include a form that binds to a receptor expressed on cell surfaces. For example, several nucleic acid complexes have been reported, the nucleic acid complexes using N-acetyl-D-galactosamine (GalNAc) or the like as a ligand for the asialoglycoprotein receptor (ASGPR), which is highly expressed on liver parenchymal cells (Patent Literature 1, Patent Literature 2, Patent Literature 3, Non Patent Literature 2). In addition, a mannose conjugate and a mannosylated nucleic acid complex have been reported as drug carriers for delivery to the mannose receptor (CD206), which is highly expressed on immunocytes such as macrophages and dendritic cells (Patent Literature 4). Further, it has been reported that a nucleic acid drug is modified with a liposoluble compound such as cholesterol and fatty acid to enhance the affinity with lipoproteins and the like in the plasma, thereby achieving delivery to cells expressing the corresponding lipoprotein receptors (LDL receptor, HDL receptor, scavenger receptor SRB1) (Non Patent Literature 3).

### Citation List

### Patent Literature

Patent Literature 1: WO 2009/073809
Patent Literature 2: WO 2014/179620
Patent Literature 3: WO 2016/100401
Patent Literature 4: WO 2018/004004

### Non-Patent Literature

Non Patent Literature 1: Nature Reviews Drug Discovery. 8, 129-138 (2009).
Non Patent Literature 2: J. Am. Chem. Soc. 2014,136,16958-16961.
Non Patent Literature 3: Nucleic Acids Research, 2019, Vol.47, No.3, 1082-1096.

### Summary of Invention

### Technical Problem

While many target ligands and nucleic acid complexes comprising a ligand have been previously proposed, sufficiently satisfactory ones in terms of improvement of specific binding to receptors and enhancement of cellular uptake of a nucleic acid drug have not been found yet in the current situation.

### Solution to Problem

The present inventors diligently studied to solve the problem, and eventually found a nucleic acid complex that is taken up in a cell-selective manner. Specifically, the present invention relates to [1] to [27] in the following.
[1] A nucleic acid complex represented by the formula (I): 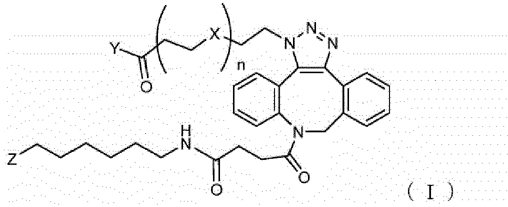 wherein X is CH₂ or O;
   Y is a sugar ligand having mannose or GalNAc;
   n is an integer of 1 to 8; and
   Z is a group containing an oligonucleotide,
   or a pharmaceutically acceptable salt thereof.
[2] A nucleic acid complex represented by the formula (II): 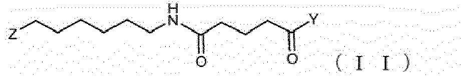 wherein Y is a sugar ligand having mannose or GalNAc; and
   Z is a group containing an oligonucleotide,
   or a pharmaceutically acceptable salt thereof.
[3] A nucleic acid complex represented by the formula (III): 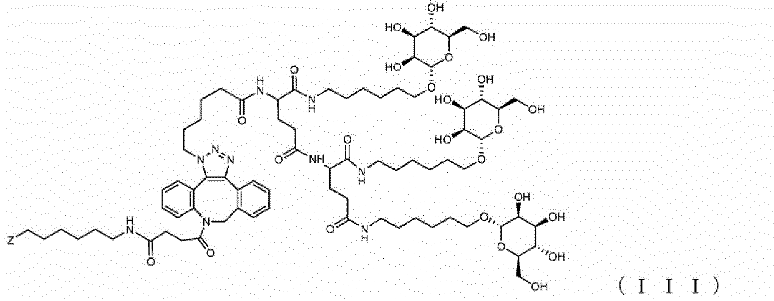 wherein Z is a group containing an oligonucleotide,
   or a pharmaceutically acceptable salt thereof.
[4] A nucleic acid complex represented by the formula (IV): 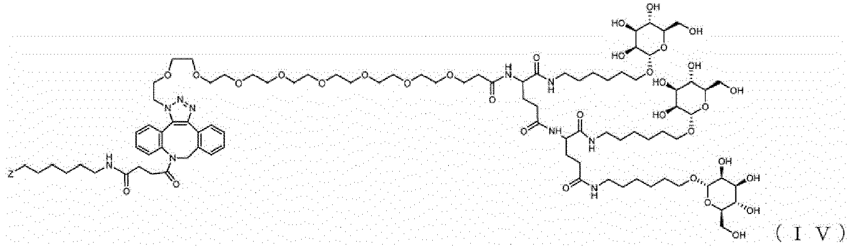 wherein Z is a group containing an oligonucleotide,
   or a pharmaceutically acceptable salt thereof.
[5] A nucleic acid complex represented by the formula (V): 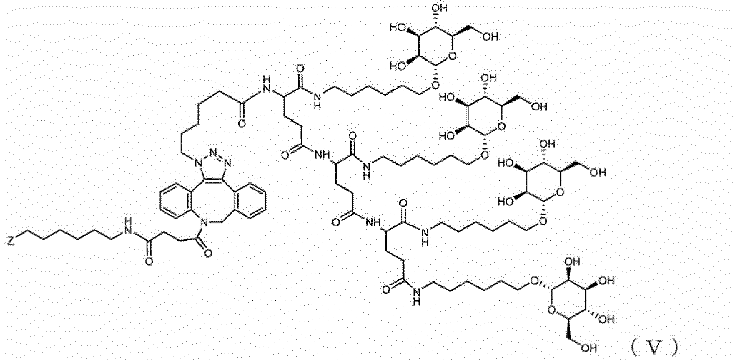 wherein Z is a group containing an oligonucleotide,
   or a pharmaceutically acceptable salt thereof.
[6] A nucleic acid complex represented by the formula (VI): 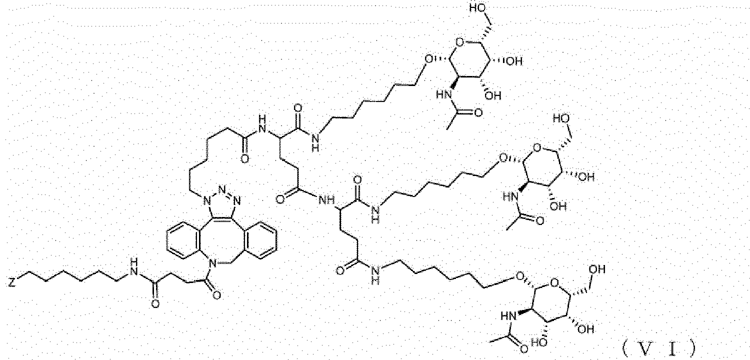 wherein Z is a group containing an oligonucleotide,
   or a pharmaceutically acceptable salt thereof.
[7] A nucleic acid complex represented by the formula (VII): 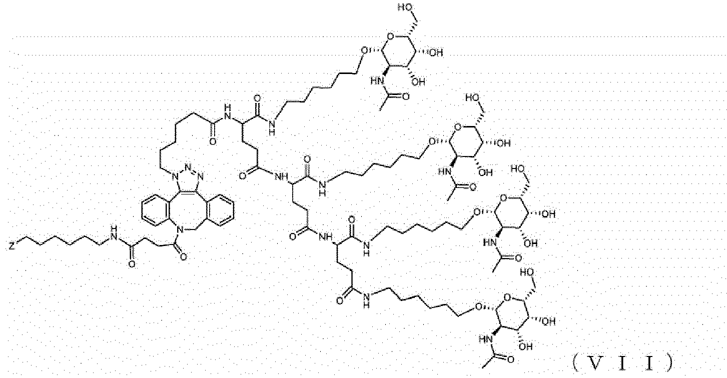 wherein Z is a group containing an oligonucleotide,
   or a pharmaceutically acceptable salt thereof.
[8] A nucleic acid complex represented by the formula (VIII): 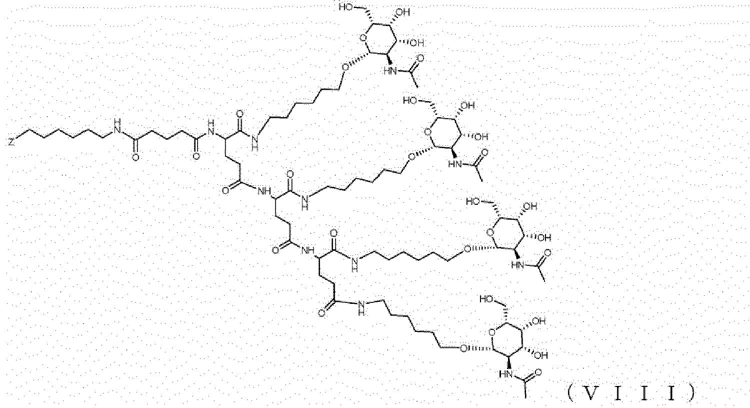 wherein Z is a group containing an oligonucleotide,
   or a pharmaceutically acceptable salt thereof.
[9] A nucleic acid complex represented by the formula (IX): 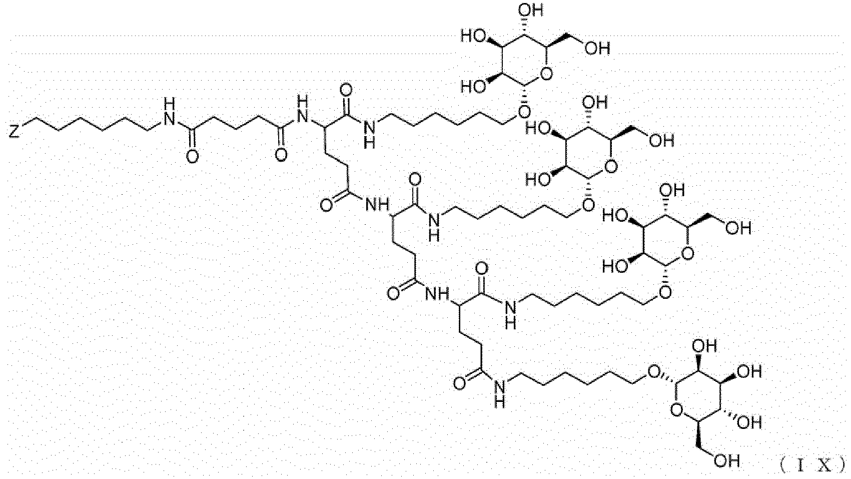 wherein Z is a group containing an oligonucleotide,
   or a pharmaceutically acceptable salt thereof.
[10] A nucleic acid complex represented by the formula (X): 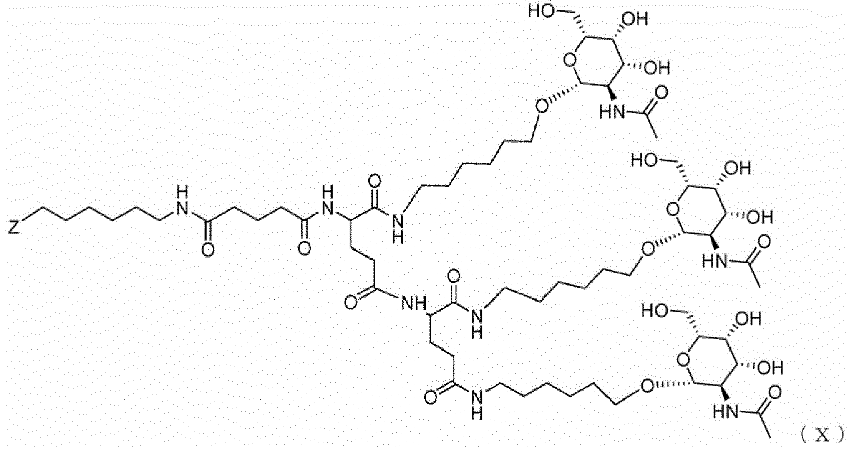 wherein Z is a group containing an oligonucleotide,
   or a pharmaceutically acceptable salt thereof.
[11] A nucleic acid complex represented by the formula (XI): 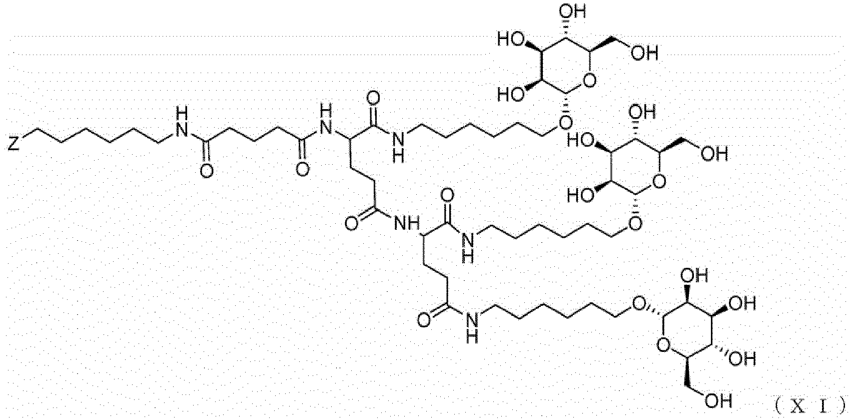 wherein Z is a group containing an oligonucleotide,
   or a pharmaceutically acceptable salt thereof.
[12] A nucleic acid complex represented by the formula (XII): 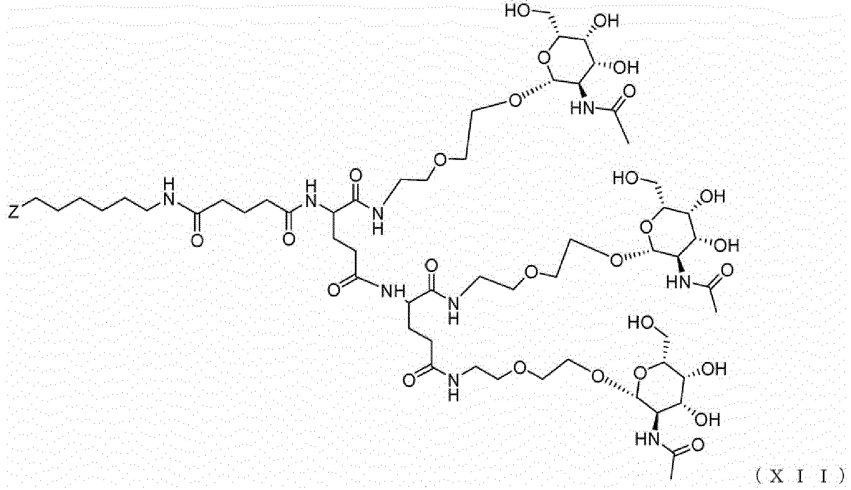 wherein Z is a group containing an oligonucleotide,
   or a pharmaceutically acceptable salt thereof.
[13] A nucleic acid complex represented by the formula (XIII): 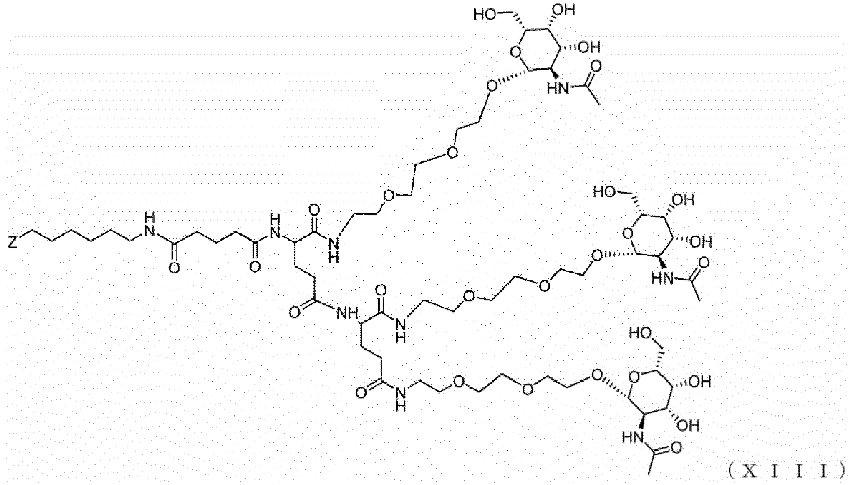 wherein Z is a group containing an oligonucleotide,
   or a pharmaceutically acceptable salt thereof.
[14] A nucleic acid complex represented by the formula (XIV): 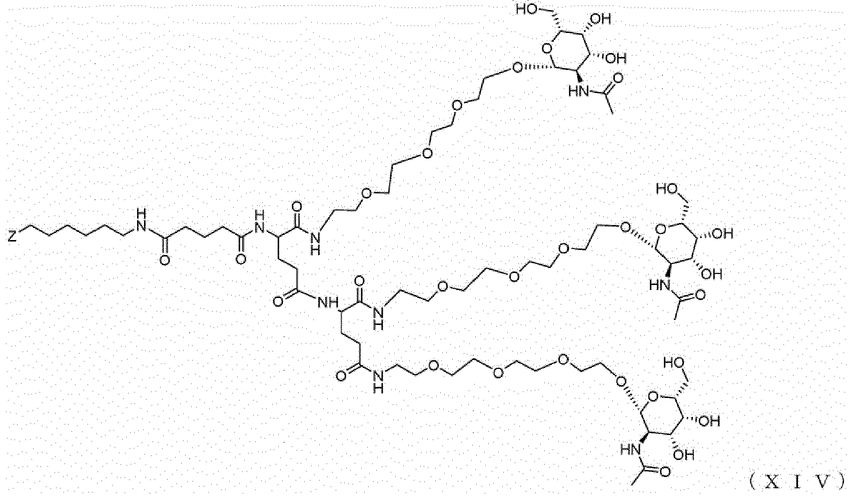 wherein Z is a group containing an oligonucleotide,
   or a pharmaceutically acceptable salt thereof.
[15] The nucleic acid complex according to any one of [1] to [14], wherein the oligonucleotide is single-stranded.
[16] The nucleic acid complex according to [15], wherein the oligonucleotide is bound via the 3' end.
[17] The nucleic acid complex according to [15], wherein the oligonucleotide is bound via the 5' end.
[18] The nucleic acid complex according to any one of [1] to [14], wherein the oligonucleotide is double-stranded.
[19] The nucleic acid complex according to [18], wherein the oligonucleotide is bound via the 3' end of one strand.
[20] The nucleic acid complex according to [18], wherein the oligonucleotide is bound via the 5' end of one strand.
[21] A pharmaceutical composition comprising the nucleic acid complex according to any one of [1] to [20].
[22] The pharmaceutical composition according to [21], wherein the pharmaceutical composition regulates expression of a target gene in a cell.
[23] The pharmaceutical composition according to [22], wherein the cell is a dendritic cell, a macrophage, or a liver parenchymal cell.
[24] The nucleic acid complex according to any one of [1] to [20], for use in a method for regulating expression of a target gene in a cell.
[25] The nucleic acid complex according to [24], wherein the cell is a dendritic cell, a macrophage, or a liver parenchymal cell.
[26] A method for regulating expression of a target gene in a cell of a subject, comprising:
   administering the nucleic acid complex according to any one of [1] to[20] or the pharmaceutical composition according to [21] to the subject.
[27] The method according to [26], wherein the cell is a dendritic cell, a macrophage, or a liver parenchymal cell.

### Advantageous Effects of Invention

As demonstrated in pharmacological tests shown later, the nucleic acid complex according to the present invention is selectively taken up by cells expressing the mannose receptor or ASGPR. The nucleic acid complex according to the present invention has a potential to cure various associated diseases through administration of a pharmaceutical composition comprising the nucleic acid complex according to the present invention to mammals (including humans).

### Description of Embodiments

Now, the contents of the present invention will be described in detail.

For a compound in the present specification, the structural formula occasionally represents a certain isomer for convenience, but the scope of the compound is not limited to the illustration of the formula for convenience, and includes all the structurally acceptable isomers including geometric isomers, optical isomers, rotamers, stereoisomers, and tautomers, and isomer mixtures, and the compound may be any one isomer or a mixture containing the isomers at any ratio. Accordingly, while optical isomers and a racemate may exist for a compound in the present specification, for example, the compound is not limited to any of them, and may be the racemate, or any of the optically active forms, or a mixture containing the optically active forms at any ratio.

While crystalline polymorphs may exist for a compound in the present specification, the compound is not limited to any of them, similarly, and may be a single substance of any of the crystal forms or a mixture thereof, and the scope of a compound in the present specification includes its amorphous forms, and the scope of a compound in the present specification encompasses its anhydride and solvates (in particular, hydrates).

The scope of a compound in the present specification also includes isotope-labeled forms of the compound. An isotope-labeled compound is the same as the original compound except that one or more atoms have been replaced with atoms each having an atomic mass or mass number differing from the atomic mass or mass number typically found in the natural world. Isotopes that can be incorporated in a compound in the present specification are isotopes of hydrogen, carbon, nitrogen, oxygen, fluorine, phosphorus, sulfur, iodine, and chlorine, and examples thereof include ²H, ³H, ¹¹C, ¹⁴C, ¹⁵N, ¹⁸O, ¹⁸F, and ³⁵S.

The "pharmaceutically acceptable salt" in the present specification is not limited as long as the pharmaceutically acceptable salt is formed by salt formation with a compound, and specific examples thereof include acid addition salts, metal salts, ammonium salts, organic amine addition salts, and amino acid addition salts.

Preferred examples of acid addition salts include inorganic acid salts such as hydrochloride, hydrobromide, sulfate, nitrate, and phosphate, and organic acid salts such as acetate, succinate, fumarate, maleate, tartrate, citrate, lactate, stearate, benzoate, methanesulfonate, p-toluenesulfonate, and benzenesulfonate.

Preferred examples of metal salts include alkali metal salts such as sodium salt and potassium salt, alkali earth metal salts such as magnesium salt and calcium salt, aluminum salt, and zinc salt.

Preferred examples of ammonium salts include salts of ammonium, tetramethylammonium, and the like.

Preferred examples of organic amine addition salts include addition salts of morpholine, piperidine, and the like.

Preferred examples of amino acid addition salts include addition salts of lysine, glycine, phenylalanine, aspartic acid, glutamic acid, and the like.

If a compound in the present specification is obtained as a free form, the compound can be converted into a state in which a salt that the compound may be forming or a hydrate thereof in accordance with a conventional method.

If a compound in the present specification is obtained as a salt or hydrate, the salt or hydrate can be converted into the free form in accordance with a conventional method.

Various isomers (e.g., geometric isomers, optical isomers, rotamers, stereoisomers, tautomers) obtained for a compound in the present specification can be purified and isolated by using a common separation means such as recrystallization, diastereomeric salt formation, enzymatic resolution, and various kinds of chromatography (e.g., thin-layer chromatography, column chromatography, gas chromatography, high-performance liquid chromatography).

The pharmaceutical composition according to the present invention can be produced by sufficiently mixing a pharmaceutically acceptable excipient with the nucleic acid complex or a pharmaceutically acceptable salt thereof. The pharmaceutical composition according to the present invention can be produced in accordance with a known method such as a method described in General Rules for Preparations, The Japanese Pharmacopeia, Seventeenth Edition.

The pharmaceutical composition according to the present invention can be administered to a patient in a proper manner according to the dosage form.

The dose of the nucleic acid complex according to the present invention depends on the degree of the symptoms, the age, sex, and body weight, the mode of administration and the type of the salt, the specific type of the disease, and so on, and a daily dose of approximately 30 µg to 10 g, preferably 100 µg to 1 g, in the case of oral administration, or a daily dose of approximately 1 µg to 1 g, preferably 100 µg to 300 mg, in the case of administration by injection, in terms of oligonucleotide is typically administered to an adult at once, or separately in several portions.

The nucleic acid complex according to the present invention is a nucleic acid complex comprising a sugar ligand bound to an oligonucleotide via a linker, wherein the sugar ligand has O-, N-, or C-linked, preferably O-linked mannose or GalNAc. That is, the nucleic acid complex according to the present invention has the structure (sugar ligand)-(linker)-(oligonucleotide).

In an embodiment, the nucleic acid complex comprises two or more sugar moieties, preferably three or four sugar moieties. In an embodiment, the nucleic acid complex comprises at least three mannose moieties or at least three GalNAc moieties, and the nucleic acid complex can be delivered to macrophages or liver parenchymal cells as the target.

### <Sugar Ligand>

The mannose receptor is highly expressed on cells highly expressing CD206, for example, specific cells including macrophages and dendritic cells. Mannose conjugates and mannosylated drug carriers exhibit high binding affinity with CD206, and are successfully used to deliver drug molecules such as oligonucleotides to cells including macrophages and dendritic cells.

ASGPR is highly expressed on specific cells including liver parenchymal cells. GalNAc conjugates and GalNAc-conjugated drug carriers exhibit high binding affinity with ASGPR, and are successfully used to deliver drug molecules such as oligonucleotides to cells including liver parenchymal cells.

The term sugar ligand refers to a group derived from a sugar capable of binding to a receptor expressed on target cells, and one of preferred modes of the sugar ligand in the nucleic acid complex according to the present invention may be, for example, the following structure. Each wavy line indicates a bond to the linker.

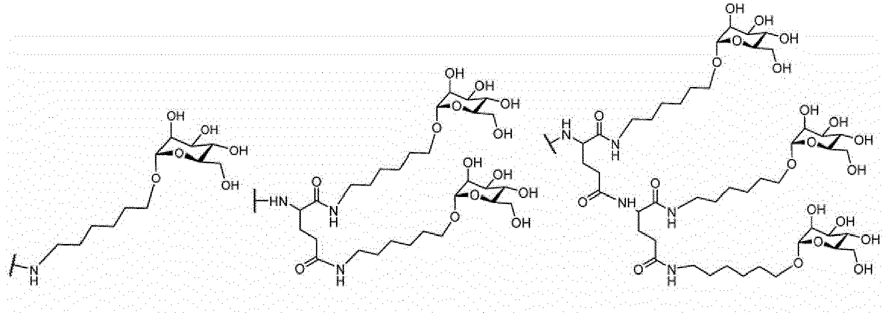

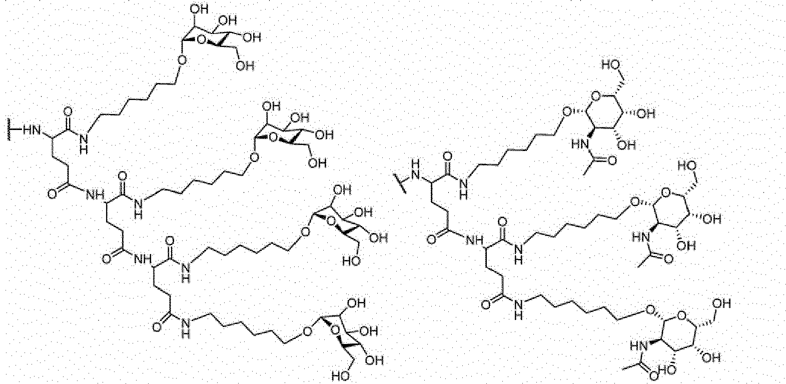

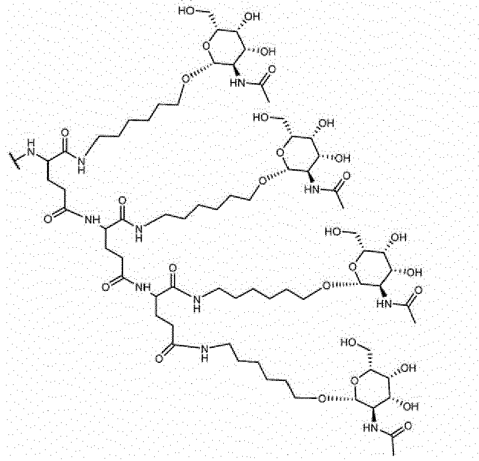

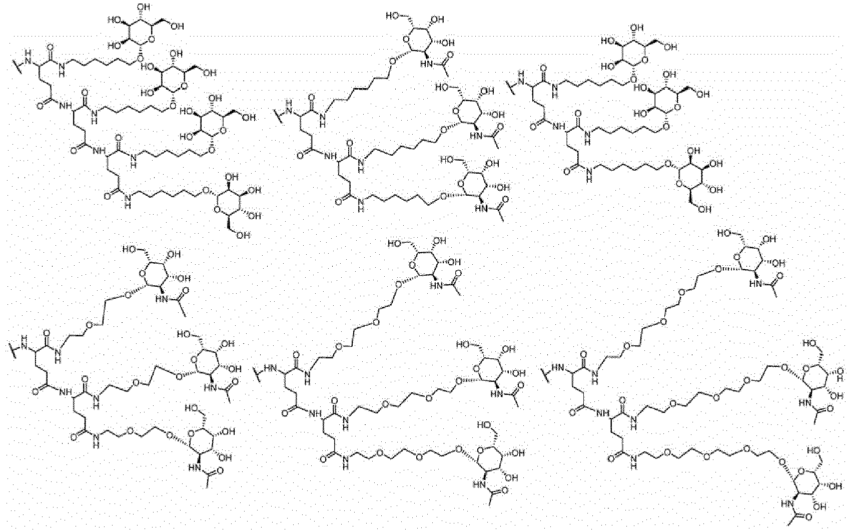

### <Oligonucleotide>

For the oligonucleotide in the nucleic acid complex according to the present invention, any oligonucleotide known to be applicable as a nucleic acid drug can be used. Herein, the term nucleic acid drug refers to nucleotides for use as an antisense drug, a decoy nucleic acid, a ribozyme, siRNA, miRNA, anti-miRNA, mRNA, or the like.

The oligonucleotide may be a single-stranded or double-stranded oligonucleotide.

The linker and the oligonucleotide in the nucleic acid complex according to the present invention may be bound together, for example, at a nucleotide of the oligonucleotide, and are bound together, for example, at the 3' end or 5' end of the oligonucleotide. If the oligonucleotide is double-stranded, it is preferable that the linker be bound to the 3' end or 5' end of the sense strand constituting the double-stranded nucleic acid; however, the binding is not limited to the mentioned one.

The number of linkers to which the oligonucleotide in the nucleic acid complex is bound is not limited to one, and may be two or more.

In some embodiments, for example, inclusion of a specific base in an overhang or inclusion of a modified nucleotide or nucleotide substitute in a single-stranded overhang (e.g., a 5' overhang or a 3' overhang, or both of them) is acceptable for enhancing the stability.

The oligonucleotide constituting the nucleic acid complex according to the present invention may be in any shape as long as the oligonucleotide has an ability to regulate expression of a target gene when being introduced into mammalian cells, and single-stranded oligonucleotide or double-stranded oligonucleotide is preferably used.

The oligonucleotide may be any molecule as long as the oligonucleotide is a polymer of nucleotides or molecules having functions comparable to those of nucleotide, and examples thereof include DNA, which is a polymer of deoxyribonucleotides, RNA, which is a polymer of ribonucleotides, and chimeric nucleic acid, which is a polymer of DNA and RNA. The DNA, RNA, and chimeric nucleic acid may be each a nucleotide polymer in which at least one nucleotide such as deoxyribonucleotide and ribonucleotide is substituted with a molecule having functions comparable to those of nucleotide. Uracil (U) in RNA is uniquely interpreted as thymine (T) in DNA.

Examples of the molecules having functions comparable to those of nucleotide include nucleotide derivatives obtained by modifying nucleotide, and a molecule or the like obtained by modifying deoxyribonucleotide or ribonucleotide, for example, to enhance or stabilize the nuclease resistance, increase the affinity with the complementary nucleic acid, or increase the cell permeability as compared with DNA and RNA, or for visualization, is preferably used.

Examples of the nucleotide derivatives include nucleotide in which at least one of the sugar moieties, the phosphodiester bond, and the base has been modified, such as nucleotide with the sugar moiety modified, nucleotide with the phosphodiester bond modified, and nucleotide with the base modified.

The nucleotide with the sugar moiety modified may be any nucleotide as long as the nucleotide is modified or substituted with any substituent or substituted with any atom partially or wholly in the chemical structure of the sugar of the nucleotide, and 2'-modified nucleotide is preferably used.

Examples of the 2'-modified nucleotide include 2'-modified nucleotide in which the 2'-OH group of the ribose is substituted with a substituent selected from the group consisting of OR, R, R'OR, SH, SR, NH₂, NHR, NR₂, N₃, CN, F, Cl, Br, and I (R is an alkyl or aryl, preferably an alkyl having one to six carbon atoms, and R' is an alkylene, preferably an alkylene having one to six carbon atoms), and preferred examples of the 2'- modification include substitution with F, substitution with a methoxy group, and substitution with an ethoxy group. Also acceptable is 2'-modified nucleotide in which the oxygen atom at the 2' position and the carbon at the 4' position in the ribose are crosslinked via methylene, that is, locked nucleic acid.

The nucleotide with the phosphodiester bond modified may be any nucleotide as long as the nucleotide is modified or substituted with any substituent or substituted with any atom partially or wholly in the chemical structure of the phosphodiester bond of the nucleotide, and examples thereof include nucleotide in which the phosphodiester bond is substituted with a phosphorothioate bond, nucleotide in which the phosphodiester bond is substituted with a phosphorodithioate bond, nucleotide in which the phosphodiester bond is substituted with an alkylphosphonate bond, and nucleotide in which the phosphodiester bond is substituted with a phosphoramidate bond, and a preferred example is nucleotide in which the phosphodiester bond is substituted with a phosphorothioate bond.

The scope of the oligonucleotide encompasses oligonucleotide in which some or all of the atoms in the molecule are substituted with an atom of different mass number (isotope).

In a mode, the oligonucleotide in the nucleic acid complex according to the present invention regulates expression of a target gene in cells.

In a mode, the oligonucleotide in the nucleic acid complex of the present invention binds to a ligand via a linker (also referred to as a linker-ligand).

### <Linker>

For the "linker" in the present invention, any linker that can be used for nucleic acid complexes can be used.

For example, any of the structures disclosed in WO 2009/073809, WO 2013/075035, and WO 2015/105083 can be employed as the linker structure.

In an embodiment, the linker comprises at least one cleavable linking group.

The term cleavable linking group refers to a group that is sufficiently stable outside of cells, but is cleaved upon the entry to a target cell and releases the moiety to which the linker is bound.

For example, a phosphate-based cleavable linking group is cleaved by an agent that decomposes or hydrolyzes a phosphate group. An example in which a phosphate group is cleaved in cells is an enzyme such as phosphatase. A preferred embodiment of the phosphate-based linking group is -O-P(O)(OH)-O- or O-P(S)(OH)-O-.

An embodiment of the linker is any one of the following structures, wherein X and Y, independently at each occurrence, X represents CH₂ or O, and Y represents a sugar ligand, respectively, Z represents a group containing oligonucleotide, and n is an integer of 1 to 8:

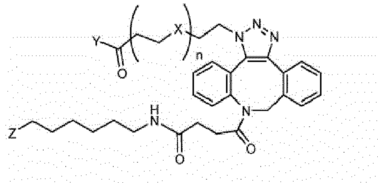

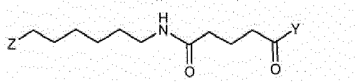

### Examples

The nucleic acid complex according to the present invention can be produced with any method described in production examples below, and the effects of the compound can be confirmed with methods described in Test Examples below. However, those are only examples, and the present invention is not limited to specific examples below in any case, and may be modified without departing from the scope of the present invention. Examples in the present invention can be produced by using synthetic chemistry techniques known to those skilled in the art.

Abbreviations used herein are conventional ones well known to those skilled in the art. In the present specification, the following abbreviations are used.
Cbz: benzyloxycarbonyl
CTC: 2-chlorotrityl chloride
DCE: 1,2-dichloroethane
DCM: dichloromethane
DIPEA: N,N-diisopropylethylamine
DMF: N,N-dimethylformamide
DMSO: dimethyl sulfoxide
EDCI: 1-ethyl-3-(3-dimethylaminopropyl)carbodiimide hydrochloride
EtOAc: ethyl acetate
ESI: electrospray ionization
Fmoc: 9-fluorenylmethyloxycarbonyl
HBTU: 1-[bis(dimethylamino)methylene]-1H-benzotriazolium 3-oxide hexafluorophosphate
HOBT: 1-hydroxybenzotriazole
IMS: industrial methylated spirit
MALDI-TOF-MS: matrix-assisted laser desorption ionization time-of-flight mass spectrometry
MeOH: methanol
MTBE: methyl tert-butyl ether
NaOMe: sodium methoxide
TFA: trifluoroacetic acid
¹H-NMR: proton nuclear magnetic resonance spectrometry
MS: mass spectrometry
HPLC: high-performance liquid chromatography

"Room temperature" in Examples and production examples below refers to approximately 10°C to approximately 35°C in normal cases. Unless otherwise specified, % indicates percent by weight or volume.

Chemical shifts in proton nuclear magnetic resonance spectra were recorded in δ units (ppm) with respect to tetramethylsilane, and coupling constants were recorded in Hertz (Hz). Patterns are s: singlet, d: doublet, t: triplet, q: quartet, quin: quintet, m: multiplet, br: broad, and br.s: broad singlet.

In silica gel column chromatography, Silica Gel 60 (70 to 230 mesh, or 230 to 400 mesh ASTM) manufactured by Merck KGaA, PSQ60B manufactured by FUJI SILYSIA CHEMICAL LTD., or a prepacked column (column: Hi-Flash (TM) Column (Silicagel) manufactured by YAMAZEN CORPORATION, or a Biotage (TM) SNAP Ultra Silica Cartridge manufactured by Biotage) was used as the silica gel.

For names of compounds, except common reagents, those displayed in "E-Notebook" version 12 or 13 (PerkinElmer, Inc.) or "MarvinSketch" version 16 (Chemaxon Ltd.) were used.

With reference to the following production examples, production can be performed with methods known to those skilled in the art.

### Synthesis Scheme for Compound 6

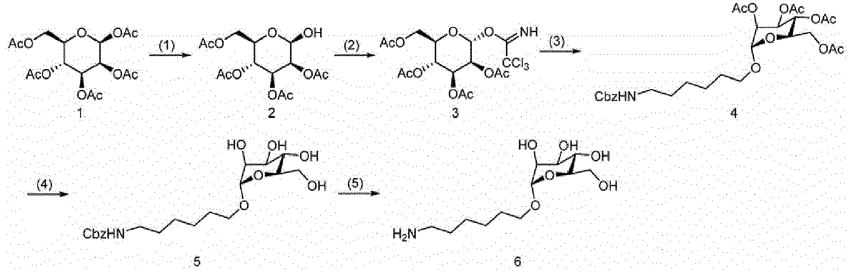

### Synthesis of Compound 2

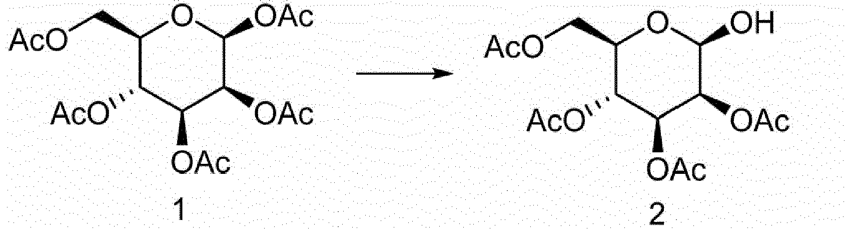

To hydrazine acetate (5.19 g, 56.4 mmol), DMF (200 mL) was added, and the reaction mixture was stirred at 55°C and then cooled to 15°C, to which 1-O,2-O,3-O,4-O,6-O-pentaacetyl-β-D-mannopyranose (compound 1) (20.0 g, 51.2 mmol) was added, and the resultant was stirred at 15°C for 16 hours. The reaction mixture was added to water, and extraction was performed with EtOAc. The organic layer combined was washed with brine, dried over anhydrous sodium sulfate, filtered, and then concentrated under reduced pressure to afford compound 2 (14.3 g).
¹H-NMR (400 MHz, CDCl₃) δ (ppm): 1.97-2.00 (m, 3H), 2.03 (s, 3H), 2.08 (s, 3H), 2.14 (s, 3H), 4.11-4.15 (m, 1H), 4.16-4.32 (m, 3H), 5.14-5.34 (m, 3H), 5.37-5.45 (m, 1H).

### Synthesis of Compound 3

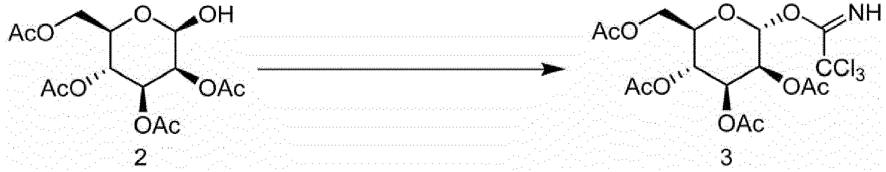

To DCM (50 mL), compound 2 (5.00 g, 14.4 mmol), 2,2,2-trichloroacetonitrile (20.7 g, 143 mmol), and Cs₂CO₃ (5.14 g, 15.8 mmol) were added, and the resultant was stirred at 25°C for 2 hours. The reaction mixture was filtered, and the filtrate was concentrated under reduced pressure. The residue was purified by silica gel column chromatography (50:1 to 20:1 petroleum ether/EtOAc) to afford compound 3 (2.70 g).
¹H-NMR (400 MHz, CDCl₃) δ (ppm): 2.01 (s, 3H), 2.04 (s, 2H), 2.08 (d, J=6.8Hz, 6H), 2.20 (s, 3H), 4.15-4.22 (m, 2H), 4.24-4.32 (m, 1H), 5.37-5.42 (m, 2H), 5.46-5.49 (m, 1H), 6.28 (d, J=1.8Hz, 1H), 8.79 (s, 1H).

### Synthesis of Compound 4

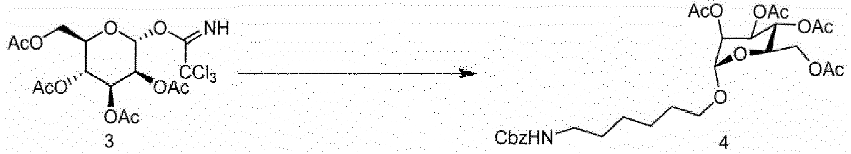

To DCM (140 mL), compound 3 (14.0 g, 28.4 mmol), 6-(Cbz-amino)-1-hexanol (8.57 g, 34.1 mmol), and molecular sieves 4A (10.0 g) were added, and the resultant was stirred at 0°C for 30 minutes. Subsequently, trimethylsilyl trifluoromethanesulfonate (6.32 g, 28.4 mmol) was added dropwise to the reaction mixture at -65°C, and the reaction mixture was stirred at 25°C for 16 hours. The reaction mixture was diluted with DCM and filtered, and the filtrate was then washed with saturated aqueous sodium hydrogen carbonate solution, water, and saline, dried over anhydrous sodium sulfate, filtered, and then concentrated under reduced pressure. The residue was purified by silica gel column chromatography (50:1 to 20:1 petroleum ether/EtOAc) to afford compound 4 (7.50 g).

### Synthesis of Compound 5

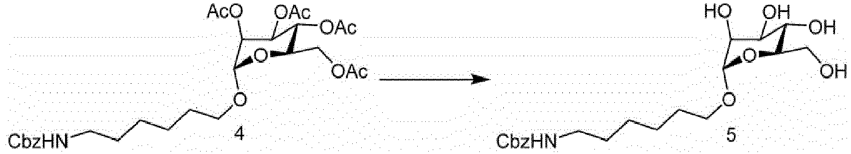

Compound 4 (7.50 g, 12.9 mmol) was dissolved in MeOH (300 mL), and NaOMe (2.79 g, 51.6 mmol) was added thereto and reacted at room temperature for 3 hours. The reaction mixture was partially concentrated and poured into cold water, to which acetic acid was added until pH = 5 was reached, and the resulting crude form was purified by silica gel column chromatography (50:1 to 20:1 DCMZMeOH) to afford compound 5 (3.90 g).
¹H-NMR (400 MHz, CDCl₃) δ (ppm): 1.26-1.63 (m, 8H) , 3.14 (d, J=6.3Hz, 2H), 3.34 (d, J=8.8Hz, 1H), 3.54-3.74(m, 2H) , 3.80-4.01 (m, 4H), 4.78 (s, 1H), 5.01-5.12 (m, 2H), 5.16-5.47 (m, 6H), 7.28-7.41 (m, 5H).

### Synthesis of Compound 6

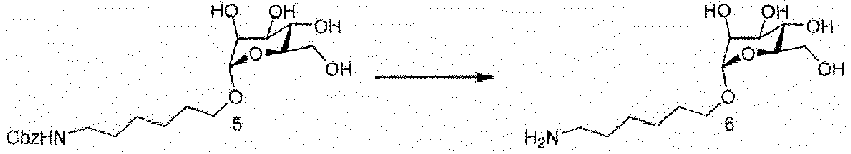

To dried 10% palladium-carbon (0.40 g), MeOH (40 mL) and compound 5 (3.90 g, 9.43 mmol) were added, and the resultant was stirred at 25°C under hydrogen pressure (50 psi) for 3 hours. The reaction mixture was filtered, and concentrated under reduced pressure to afford compound 6 (1.50 g).
¹H-NMR (400 MHz, MeOD) δ (ppm): 1.38-1.49 (m, 4H), 1.61 (dquin, J=13.3, 6.9, 6.9, 6.9, 6.9Hz, 4H), 2.73-2.88 (m, 2H), 3.31 (dt, J=3.3, 1.6Hz, 1H), 3.43 (dt, J=9.7, 6.1Hz, 1H), 3.48-3.89 (m, 8H).

### Synthesis of Compound 8

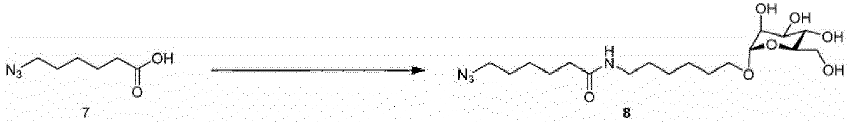

To a solution of 6-azidehexanoic acid (compound 7) (50.2 mg, 319 µmol) and compound 6 (268 mg, 959 µmol) in DMF (3 mL), HOBt (129 mg, 959 µmol), DIPEA (248 mg, 1.92 mmol), and EDCI (183 mg, 959 µmol) were added, and the resultant was stirred at 25°C for 3 hours. The reaction mixture was purified by preparative HPLC (TFA condition) to afford compound 8 (20.1 mg).
¹H-NMR (400 MHz, DMSO-d6) δ (ppm): 1.24-1.54 (m, 14H) 2.04 (t, J=7.40Hz, 2H) 3.00 (q, J=6.53Hz, 2H) 3.23-3.40 (m, 12H) 3.55-3.66 (m, 2H) 4.57 (s, 1H) 7.75 (br s, 1H)

### Synthesis Scheme for Compound 11

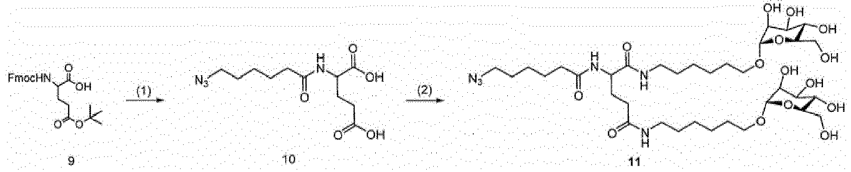

### Synthesis of Compound 10

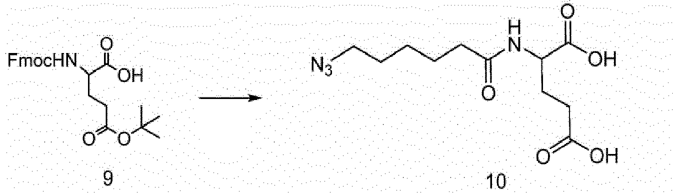

To a mixture containing CTC resin (0.50 g, 0.50 mmol, 1.00 mmol/g) and 5-tert-butyl N-[(9H-fluoren-9-ylmethoxy)carbonyl]-glutamate (compound 9) (212 mg, 0.50 mmol), DCM (30 mL) and DIPEA (258 mg, 2.00 mmol) were added, and the reaction mixture was stirred for 2 hours. MeOH (0.2 mL) was added to the reaction mixture, which was stirred for 30 minutes. To the reaction mixture, 6-azidehexanoic acid (compound 7) (118 mg, 0.75 mmol), DIPEA (258 mg, 2.00 mmol), HBTU (270 mg, 712 µmol), and DMF (3 mL) were added, and the resultant was stirred at 25° for 1 hour. To the reaction mixture, 90% TFA/10% DCM was added, and the resultant was stirred for 2 hours. Reaction mixture was filtered, and the filtrate was concentrated under reduced pressure to afford compound 10 (110 mg).

### Synthesis of Compound 11

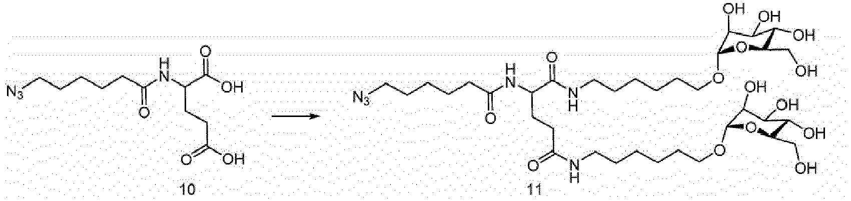

To a solution of compound 10 (50.0 mg, 174 µmol) and compound 6 (292 mg, 1.05 mmol) in DMF (3 mL), HOBt (141 mg, 1.05 mmol), DIPEA (270 mg, 2.10 mmol), and EDCI (200 mg, 1.05 mmol) were added. The reaction mixture was stirred at 25°C for 16 hours. Purification was performed by preparative HPLC (TFA condition) to afford compound 11 (18.1 mg).
¹H-NMR (400 MHz, DMSO-d₆) δ (ppm):
1.16-1.56 (m, 24H), 1.62-1.73 (m, 1H), 1.76-1.87 (m, 1H), 2.00-2.07 (m, 2H), 2.09-2.15 (m, 2H), 2.95-3.07 (m, 4H), 3.22-3.41 (m, 18H), 3.65 (br s, 6H), 4.10-4.19 (m, 1H), 4.57 (d, J=1.00Hz, 2H), 7.74-7.93 (m, 3H).

### Synthesis of Compound 12

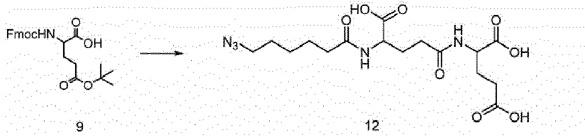

To CTC resin (1.00 g, 1.00 mmol, 1.00 mmol/g) and 5-tert-butyl N-[(9H-fluoren-9-ylmethoxy)carbonyl]-glutamate (compound 9) (511 mg, 1.20 mmol), DCM (20 mL) and DIPEA (516 mg, 4.00 mmol) were added, and the reaction mixture was stirred for 2 hours while bubbling with N₂ was performed. MeOH (1 mL) was added to the reaction mixture, which was stirred for 30 minutes. A solution of 20% piperidine in DMF (30 mL) was added to the reaction mixture, which was stirred for 30 minutes, and the resin was washed with DMF, a solution of 1-tert-butyl N-[(9H-fluoren-9-ylmethoxy)carbonyl]-glutamate (N-Fmoc-glutamic acid-1-tert-butyl ester) (851 mg, 2.00 mmol), HBTU (720 mg, 1.90 mmol), and DIPEA (516 mg, 4.00 mmol) inDMF (5 mL) was added thereto, and the resultant was stirred at 25°C for 1 hour. A solution of 20% piperidine in DMF (30 mL) was added thereto, the resultant was stirred for 30 minutes, and the resin was washed with DMF. Subsequently, a solution of 6-azidehexanoic acid (compound 7) (0.30 g, 1.91 mmol), HBTU (720 mg, 1.90 mmol), and DIPEA (516 mg, 4.00 mmol) in DMF (5 mL) was added thereto, the resultant was stirred at 25°C for 1 hour, and the resin was washed with DMF, washed with MeOH, and dried under reduced pressure. To the resulting residue (0.60 g), 50% TFA/50% DCM was added, the resultant was stirred for 2 hours, and the reaction mixture was concentrated under reduced pressure to afford compound 12 (240 mg).

### Synthesis of Compound 13

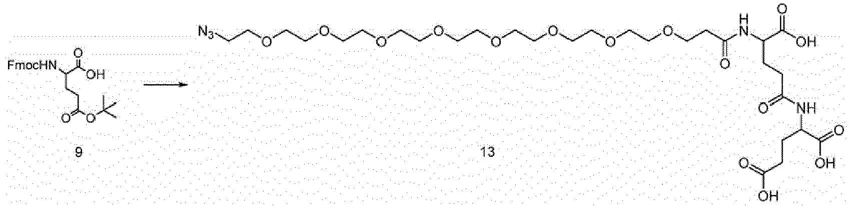

Synthesis of compound 13 was performed in accordance with the synthesis method for compound 12 with use of Azido-PEG8-acid (CAS No. 1214319-92-2) in place of 6-azidehexanoic acid to afford compound 13 (350 mg).
¹H-NMR (400 MHz, CDCl₃) δ (ppm):
2.01 (s, 2H), 2.18 (s, 2H), 2.32-2.63 (m, 6H), 3.40 (t, J=5.0Hz, 2H), 3.54-3.71 (m, 32H), 4.49 (s, 2H), 7.67-8.03 (m, 2H).

### Synthesis of Compound 14

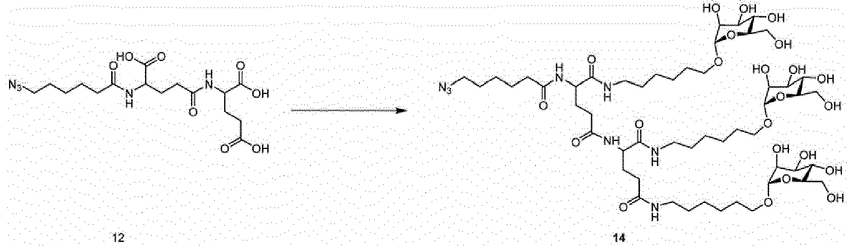

To a solution of compound 12 (0.20 g, 481 µmol) and compound 7 (1.08 g, 3.85 mmol) in DMF (0.5 mL), DIPEA (996 mg, 7.70 mmol), HOBt (520 mg, 3.85 mmol), and EDCI (738 mg, 3.85 mmol) were added. The reaction mixture was stirred at 25°C for 16 hours, and purified by preparative HPLC (TFA condition) to afford compound 14 (103 mg).
¹H-NMR (400 MHz, MeOD) δ (ppm):
1.26-1.72 (m, 32H), 1.82-1.97 (m, 2H), 2.03-2.13 (m, 2H), 2.22-2.41 (m, 6H), 3.14-3.24 (m, 6H), 3.37-3.47 (m, 3H), 3.49-3.55 (m, 3H), 3.60 (t, J=9.5Hz, 3H), 3.65-3.86 (m, 15H), 4.22-4.42 (m, 2H), 4.73 (s, 3H).

### Synthesis of Compound 15

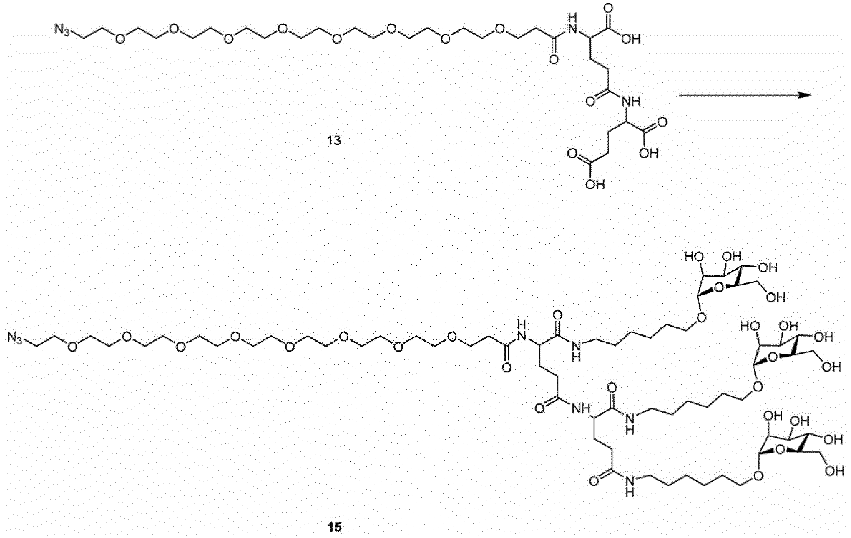

To a solution of compound 13 (0.20 g, 275 µmol) and compound 7 (616 mg, 2.20 mmol) in DMF (0.5 mL), DIPEA (570 mg, 4.41 mmol), HOBT (298 mg, 2.20 mmol), and EDCI (423 mg, 2.20 mmol) were added, and the reaction mixture was stirred at 25°C for 16 hours. The reaction mixture was purified by preparative HPLC (TFA condition) to afford compound 15 (127 mg).
¹H-NMR (400 MHz, MeOD) δ (ppm):
1.23-1.68 (m, 24H), 1.78-1.95 (m, 2H), 2.04-2.19 (m, 2H), 2.23-2.31 (m, 2H), 2.33-2.42 (m, 2H), 2.48-2.60 (m, 2H), 3.10-3.26 (m, 6H), 3.35-3.46 (m, 5H), 3.48-3.56 (m, 3H), 3.57-3.84 (m, 50H), 4.23-4.41 (m, 2H), 4.73 (s, 3H).

### Synthesis Scheme for Compound 17

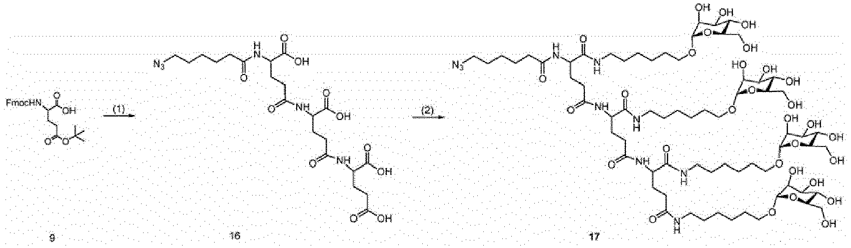

### Synthesis of Compound 16

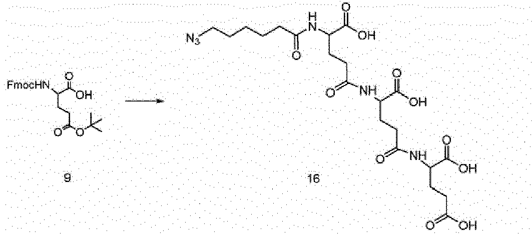

To CTC resin (0.50 mmol, 0.50 g, 1.00 mmol/g) and 5-tert-butyl N-[(9H-fluoren-9-ylmethoxy)carbonyl]-glutamate (compound 9) (212 mg, 0.50 mmol), DCM (30 mL) and DIPEA (258 mg, 2.00 mmol) were added. The reaction mixture was stirred for 2 hours, MeOH (0.2 mL) was then added thereto, and the resultant was stirred for 30 minutes. To the reaction mixture, a solution of HBTU (360 mg, 950 µmol) and DIPEA (258 mg, 2.00 mmol) in DMF (3 mL) and 1-tert-butyl N-[(9H-fluoren-9-ylmethoxy)carbonyl]-glutamate (N-Fmoc-glutamic acid-1-tert-butyl ester) (425 mg, 1.00 mmol) were added, the resultant was stirred at 25°C for 1 hour, and a solution of 1-tert-butyl N-[(9H-fluoren-9-ylmethoxy)carbonyl]-glutamate (N-Fmoc-glutamic acid-1-tert-butyl ester) (425 mg, 1.00 mmol), HBTU (360 mg, 950 µmol), and DIPEA (258 mg, 2.00 mmol) in DMF (3 mL) was added at 25°C over 1 hour. Next, 6-azidehexanoic acid (compound 7) (118 mg, 750 µmol), HBTU (360 mg, 950 µmol), and DIPEA (258 mg, 2.00 mmol) were added thereto, and the resultant was stirred for 1 hour. Thereto, 90% TFA/10% DCM was added, and the resultant was stirred at room temperature for 2 hours. The reaction mixture was filtered, and concentrated under reduced pressure to afford compound 16 (200 mg).

### Synthesis of Compound 17

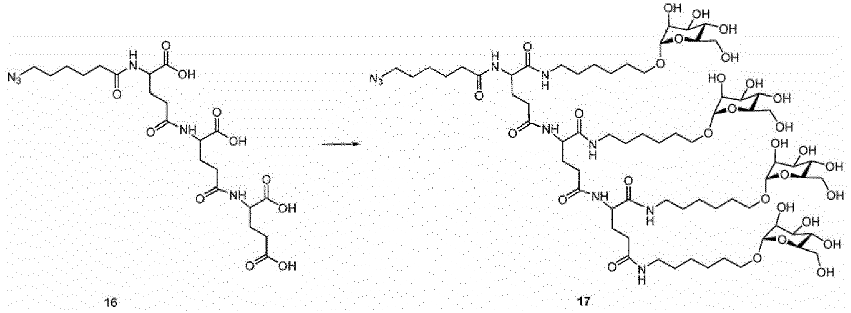

To a solution of compound 16 (50.0 mg, 91.8 µmol) and compound 7 (256 mg, 918 µmol) in DMF (3 mL), HOBt (124 mg, 918 µmol), DIPEA (237 mg, 1.84 mmol), and EDCI (176 mg, 918 µmol) were added, and the reaction mixture was stirred at 25°C for 16 hours. The reaction mixture was purified by preparative HPLC (TFA condition) to afford compound 17 (18.2 mg).
¹H-NMR (400 MHz, DMSO-d₆) δ (ppm):
1.19-1.56 (m, 40H), 1.66 (br s, 3H), 1.83 (br s, 3H), 2.00-2.15 (m, 7H), 2.94-3.05 (m, 8H), 3.29 (br d, J=5.02Hz, 24H), 3.52-3.71 (m, 24H), 4.13 (br s, 3H), 4.57 (s, 4H), 7.74-7.99 (m, 7H).

### Synthesis Scheme for Compounds 21 and 22

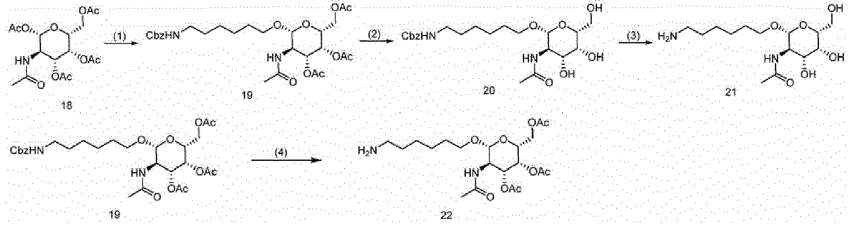

### Synthesis of Compound 19

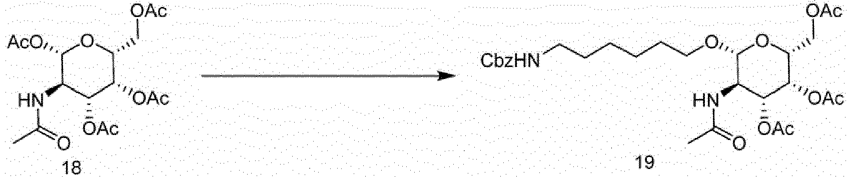

To a mixture of galactosamine pentaacetate compound 18 (12.0 g, 30.8 mmol), 6-(Cbz-amino)-1-hexanol (10.3 g, 41.1 mmol), and ZnCl₂ (5.60 g, 41.1 mmol) (dried under reduced pressure at 110°C for 1 hour before use), DCE (120 mL) was added, and the reaction mixture was stirred at 70°C for 3 hours. The reaction mixture was diluted with EtOAc and aqueous sodium hydrogen carbonate solution, and the mixture was stirred for 10 minutes, filtered through a Celite (Registered Trademark), and washed with EtOAc. The filtrate was washed with water and saline. The aqueous layer was subjected to extraction with EtOAc, and the organic layer was dried over sodium sulfate, filtered, and then concentrated under reduced pressure, and the resulting residue was purified by silica gel column chromatography (2:1 petroleum ether/EtOAc) to afford compound 19 (14.0 g).
¹H-NMR (400 MHz, CDCl₃) δ (ppm): 1.35 (d, J=3.8Hz, 4H), 1.45-1.66 (m, 4H), 1.77 (s, 2H), 1.94 (s, 3H),2.00 (s, 3H), 2.05 (s, 3H), 2.14 (s, 3H), 3.20 (qq, J=13.4, 6.8Hz, 2H), 3.48 (dt, J=9.6, 6.5Hz, 1H), 3.79-4.03 (m, 3H), 4.08-4.19 (m, 2H), 4.65 (d, J=8.3Hz, 1H), 4.90 (s, 1H), 5.04-5.17 (m, 2H), 5.26 (dd, J=11.2, 3.1Hz, 1H), 5.30 (s, 1H), 5.34 (d, J=2.8Hz, 1H), 5.94 (d, J=8.8Hz, 1H), 7.28-7.42 (m, 5H).

### Synthesis of Compound 20

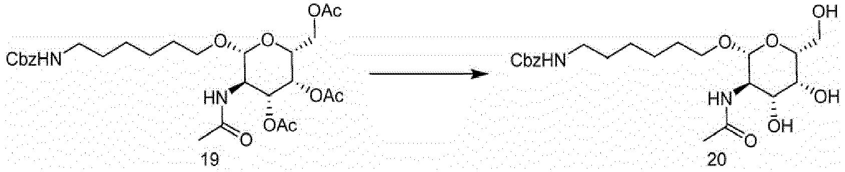

To compound 19 (7.50 g, 12.9 mmol), MeOH (300 mL) and NaOMe (2.79 g, 51.7 mmol) were added, and the resultant was stirred at room temperature for 3 hours. The reaction mixture was partially concentrated and poured into cold water, to which acetic acid was added until pH = 5 was reached. Next, the resulting crude product was purified by silica gel column chromatography (50:0 to 50:1 DCM/MeOH) to afford compound 20 (4.00 g).
¹H-NMR (400 MHz, MeOD) δ (ppm): 1.19-1.35 (m, 4H), 1.40-1.56 (m, 4H), 1.93 (s, 2H), 3.06 (t, J=7.0Hz, 2H), 3.27 (dt, J=3.3, 1.6Hz, 1H), 3.31 (s, 1H), 3.37-3.49 (m, 2H), 3.55 (dd, J=10.8, 3.3Hz, 1H), 3.65-3.76 (m, 2H), 3.78-3.94 (m, 3H), 4.31 (d, J=8.5Hz, 1H), 5.02 (s, 2H), 7.17-7.44 (m, 5H).

### Synthesis of Compound 21

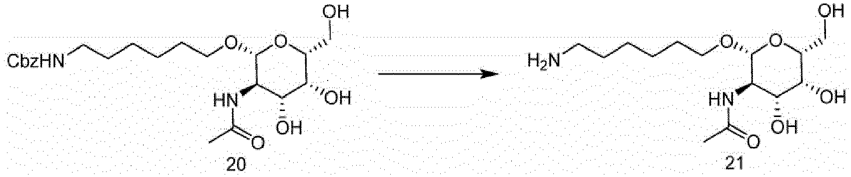

To dried 10% palladium-carbon (240 mg), MeOH (25 mL) and compound 20 (2.40 g, 5.28 mmol) were added in an argon atmosphere, and the resultant was stirred under hydrogen pressure (50 psi) at 25°C for 3 hours. The reaction mixture was filtered, and the filtrate was concentrated under reduced pressure to afford compound 21 (0.66 g).
¹H-NMR (400 MHz, MeOD) δ (ppm): 1.30-1.44 (m, 4H), 1.52-1.66 (m, 4H), 1.98 (s, 3H), 2.76-2.90 (m, 2H), 3.31 (dt, J=3.3, 1.6Hz, 2H), 3.44-3.51 (m, 2H), 3.58 (dd, J=10.8, 3.3Hz, 1H), 3.73-3.78 (m, 2H), 3.82-3.95 (m, 3H), 4.34 (d, J=8.5Hz, 1H).

### Synthesis of Compound 22

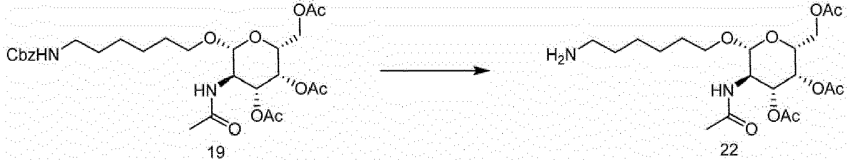

To compound 19 (202 mg, 0.35 mmol), ethanol (6 mL) and 10% palladium-carbon (50% wet product, 42 mg) were added, and the resultant was stirred in a hydrogen atmosphere at room temperature for 3 hours. The reaction mixture was filtered through a Celite (Registered Trademark), and washed with ethanol. The filtrate was concentrated under reduced pressure to afford compound 22 (159 mg).
¹H-NMR (600 MHz, DMSO-d6) δ (ppm): 0.64 (br s, 1H) 1.27 (br s, 5H) 1.33-1.52 (m, 4H) 1.67 (br s, 1H) 1.80 (s, 3H) 2.61 (br t, J=6.88Hz, 2H) 3.40-3.60 (m, 5H) 3.58-3.77 (m, 4H) 4.23 (br d, J=8.25Hz, 1H) 4.31-4.97 (m, 5H) 7.53-7.71 (m, 1H); MS (ESI+): m/z 321 [M+H]+.

### Synthesis of Compound 23

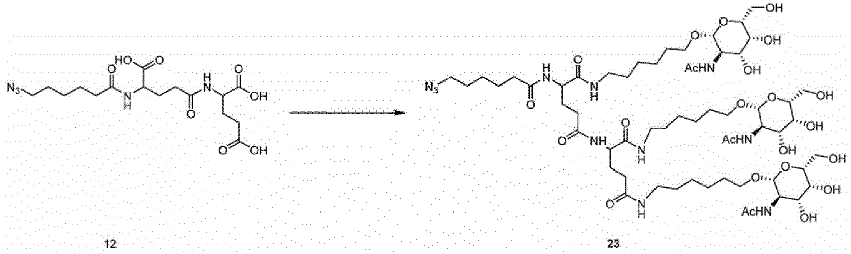

To a solution of compound 12 (602 mg, 1.88 mmol) and compound 21 (601 mg, 1.88 mmol) in DMF (0.5 mL), DIPEA (485 mg, 3.76 mmol), HOBt (254 mg, 1.88 mmol), and EDCI (360 mg, 1.88 mmol) were added, and the reaction mixture was stirred at 25°C for 16 hours. The residue was purified by preparative HPLC (TFA condition) to afford compound 23 (104 mg).
¹H-NMR (400 MHz, MeOD) δ (ppm): 1.29-1.70 (m, 32H), 1.82-2.01 (m, 11H), 2.08 (s, 2H), 2.22-2.38 (m, 8H), 3.18 (d, J=6.3Hz, 6H), 3.42-3.52 (m, 6H), 3.60 (d, J=10.5Hz, 3H), 3.71-3.95 (m, 14H), 4.28 (s, 1H), 4.36 (d, J=8.3Hz, 3H).

### Synthesis of Compound 24

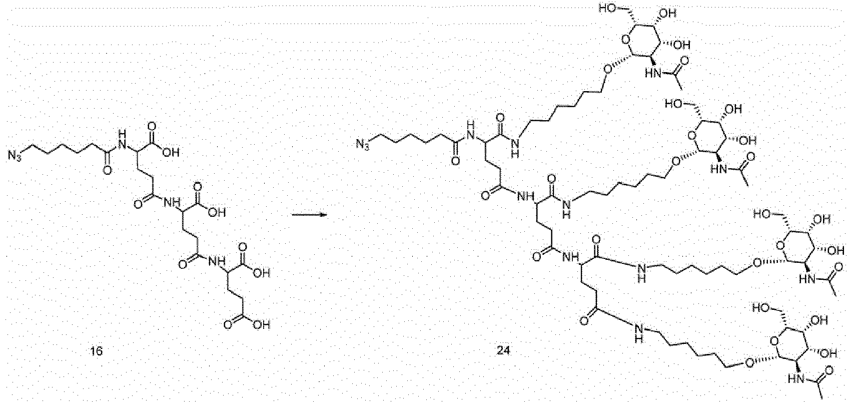

Compound 24 was obtained in the same manner as in Synthesis of Compound 23 with use of compound 16 in place of compound 12.
¹H-NMR (600 MHz, DMSO-d6) δ (ppm): 1.15-1.59 (m, 44H) 1.68 (br d, J=9.90Hz, 3H) 1.79 (s, 12H) 1.82-1.92 (m, 5H) 1.97-2.22 (m, 9H) 3.02 (br d, J=5.50Hz, 10H) 3.32-3.58 (m, 19H) 3.61-3.72 (m, 13H) 4.06-4.27 (m, 8H) 4.43 (br s, 4H) 4.46-4.59 (m, 9H) 7.59 (br d, J=8.80Hz, 4H) 7.74 (br s, 1H) 7.83 (br s, 3H) 7.89 (br s, 3H); MS (ESI+): m/z 1754 [M+H]+.

### Synthesis Scheme for Compound 28

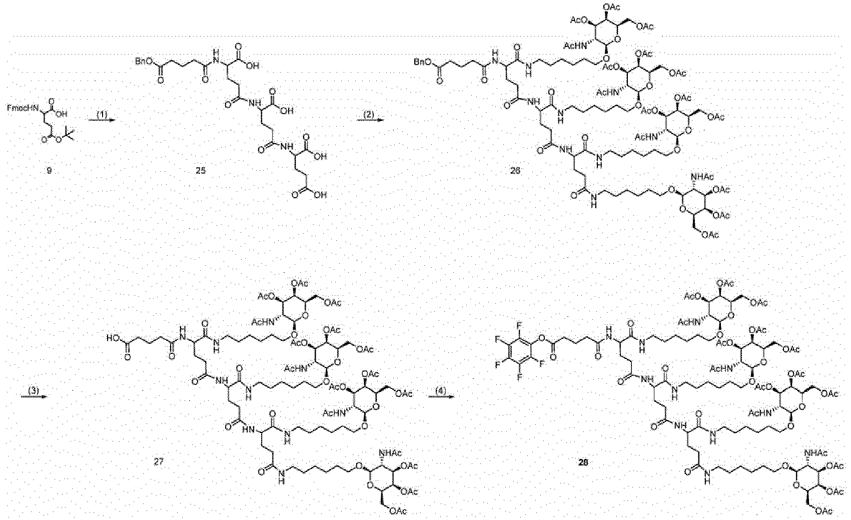

### Synthesis of Compound 25

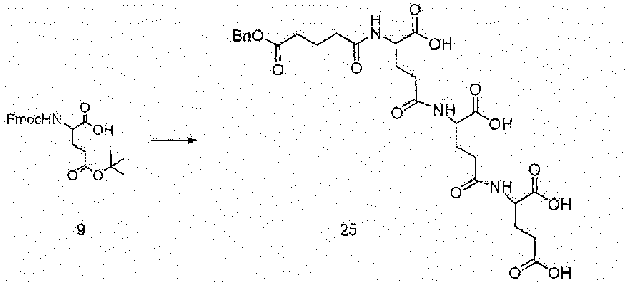

To a mixture containing CTC resin (1.33 mmol/g, 0.375 g, 0.50 mmol) and compound 9 (212 mg, 0.50 mmol), DCM(30 mL) was added, DIPEA (0.35 mL, 2.00 mmol) was added dropwise thereto, and the reaction mixture was stirred for 2 hours. MeOH (0.2 mL, 4.94 mmol) was added to the reaction mixture, which was stirred for 35 minutes. To the reaction mixture, a solution of 20% piperidine in DMF (15 mL) was added, and the resultant was stirred for 30 minutes. The reaction mixture was filtered, and the resulting solid was washed with DMF. Subsequently, 1-tert-butyl N-[(9H-fluoren-9-ylmethoxy)carbonyl]-D-glutamate (N-Fmoc-D-glutamic acid-1-tert-butyl ester) (213 mg, 0.499 mmol), 1-tert-butyl N-[(9H-fluoren-9-ylmethoxy)carbonyl]-L-glutamate (N-Fmoc-L-glutamic acid-1-tert-butyl ester) (213 mg, 0.499 mmol), HBTU (360 mg, 0.949 mmol), DMF (3 mL), and DIPEA (0.35 mL, 2.00 mmol) were added thereto, and the reaction mixture was stirred for 1 hour while bubbling with nitrogen was performed. A solution of 20% piperidine in DMF (15 mL) was added thereto, and the resultant was stirred for 30 minutes. Then, the resin was washed with DMF. Subsequently, 1-tert-butyl N-[(9H-fluoren-9-ylmethoxy)carbonyl]-D-glutamate (N-Fmoc-D-glutamic acid-1-tert-butyl ester) (213 mg, 0.499 mmol), 1-tert-butyl N-[(9H-fluoren-9-ylmethoxy)carbonyl]-L-glutamate (N-Fmoc-L-glutamic acid-1-tert-butyl ester) (213 mg, 0.499 mmol), HBTU (360 mg, 0.949 mmol), and DMF (3 mL) were added thereto, and DIPEA (0.35 mL, 2.00 mmol) was slowly added dropwise thereto. The reaction mixture was stirred for 80 minutes while bubbling with nitrogen was performed. Then, the resin was washed with DMF, washed with MeOH, and dried under reduced pressure. To the resulting residue, 50% TFA/50% DCM was added, the resultant was stirred for 2 hours and filtered, and the filtrate was then concentrated under reduced pressure to afford compound 25 (307.3 mg).
MS (ESI) M/Z: 610 [M+H]⁺
¹H-NMR (600 MHz, DMSO-d₆) δ (ppm):
1.77 (m, 5H), 1.94 (m, 3H), 2.12-2.23 (m, 6H), 2.26 (m, 2H), 2.38 (br t, J=7.34Hz, 2H), 4.13-4.21 (m, 3H), 5.09 (s, 2H), 7.30-7.40 (m, 5H), 8.05-8.15 (m, 3H).

### Synthesis of Compound 26

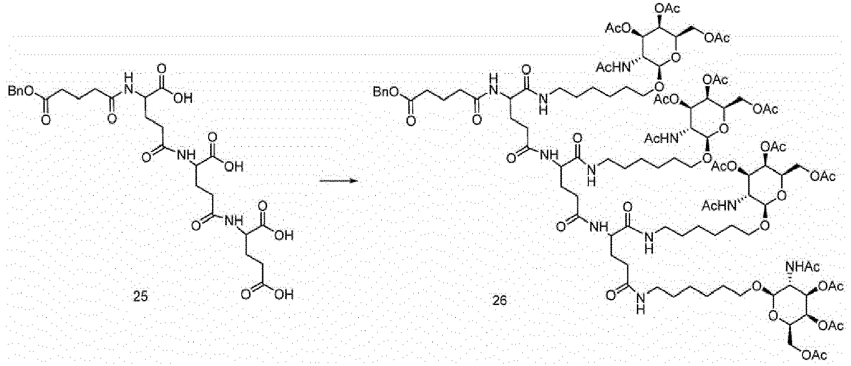

To a solution of compound 22 (154 mg, 0.344 mmol) and compound 25 (21.0 mg, 0.034 mmol) in DMF (2 mL), HOBt (52.8 mg, 0.344 mmol), DIPEA (0.12 mL, 0.689 mmol), and EDCI (66.0 mg, 0.344 mmol) were added. The reaction mixture was stirred at room temperature for 18 hours, and water, EtOAc, and a small amount of methanol were added thereto for extraction, and the organic layer was dried over sodium sulfate. The resultant was filtered, and then concentrated under reduced pressure. The residue was purified by preparative HPLC to afford compound 26 (16.7 mg).
MS (ESI) M/Z: 1162 [M+2H]⁺
¹H-NMR (600 MHz, DMSO-d₆) δ (ppm):
1.23 (br s, 16H), 1.31-1.41 (m, 8H), 1.41-1.48 (m, 8H), 1.60-1.73 (m, 4H), 1.77 (s, 12H), 1.78-1.87 (m, 4H), 1.89 (s, 12H), 1.99 (s, 12H), 2.01-2.07 (m, 2H), 2.10 (s, 12H), 2.11-2.21 (m, 6H), 2.33-2.37 (m, 2H), 2.95-3.07 (m, 8H), 3.37-3.43 (m, 4H), 3.65-3.72 (m, 4H), 3.82-3.90 (m, 4H), 3.98-4.05 (m, 12H), 4.09-4.20 (m, 3H), 4.48 (d, J=8.44Hz, 4H), 4.97 (dd, J=11.19, 3.12Hz, 4H), 5.08 (s, 2H), 5.21 (d, J=3.30Hz, 4H), 7.30-7.39 (m, 5H), 7.80 (m, 8H), 7.86-7.95 (m, 3H).

### Synthesis of Compound 27

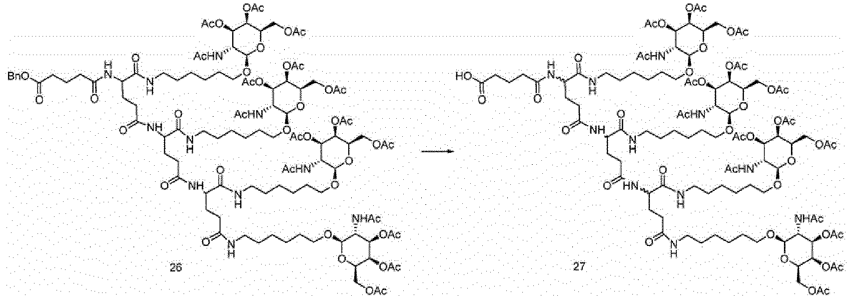

To a solution of compound 26 (19.0 mg, 8.18 µmol) in ethanol (3 mL), 10% palladium-carbon (50% wet product) (5.3 mg) was added. The reaction mixture was stirred in a hydrogen atmosphere at room temperature for 3 hours. The reaction mixture was filtered through a Celite (Registered Trademark), and washed with ethanol. The filtrate was concentrated under reduced pressure to afford compound 27 (19.6 mg).
¹H-NMR (600 MHz, DMSO-d₆) δ (ppm):
1.24 (br s, 22H), 1.30-1.40 (m, 8H), 1.45 (br s, 8H), 1.61-1.74 (m, 4H), 1.77 (s, 12H), 1.80-1.87 (m, 2H), 1.89 (s, 12H), 1.99 (s, 12H), 2.01-2.07 (m, 2H), 2.10 (s, 12H), 2.12-2.25 (m, 4H), 2.93-3.06 (m, 8H), 3.36-3.46 (m, 4H), 3.64-3.72 (m, 4H), 3.82-3.91 (m, 4H), 3.98-4.20 (m, 16H), 4.50 (br d, J=8.07Hz, 4H), 4.94-5.02 (m, 4H), 5.21 (d, J=2.93Hz, 4H) 7.73-8.03 (m, 11H).

### Synthesis of Compound 28

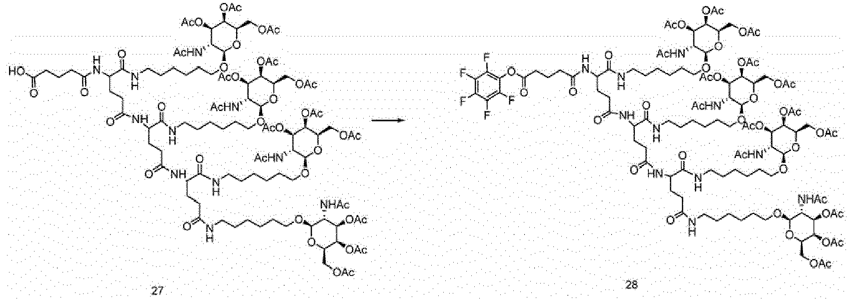

To a solution of compound 27 (17.4 mg, 7.79 µmol) in DMF (2 mL), triethylamine (8.7 µL, 62 µmol) and pentafluorophenyl trifluoroacetate (5.4 µL, 31 µmol) were added in a nitrogen atmosphere, and the reaction mixture was stirred at room temperature for 1.5 hours. Water was added to the reaction mixture, and extraction was performed with EtOAc. The organic layer was washed with saturated sodium hydrogen carbonate, sodium hydrogen sulfate, and brine. After concentration under reduced pressure, the residue was triturated with pentane, collected through filtration, and dried at 40°C under reduced pressure to afford compound 28 (11.72 mg).
MS (ESI) M/Z: 1200 [M+2H]⁺
¹H-NMR (600 MHz, DMSO-d₆) δ (ppm):
1.24 (br s, 20H), 1.31-1.40 (m, 8H), 1.41-1.49 (m, 8H), 1.61-1.72 (m, 2H), 1.77 (s, 12H), 1.79-1.86 (m, 2H), 1.89 (s, 12H), 1.99 (s, 12H), 2.01-2.07 (m, 2H), 2.10 (s, 12H), 2.12-2.21 (m, 4H), 2.25-2.31 (m, 2H), 2.80 (br t, J=7.34Hz, 2H), 2.95-3.08 (m, 8H), 3.37-3.43 (m, 4H), 3.65-3.72 (m, 4H), 3.82-3.90 (m, 4H), 3.97-4.06 (m, 12H), 4.10-4.22 (m, 3H), 4.48 (d, J=8.44Hz, 4H), 4.97 (dd, J=11.00, 2.93Hz, 4H), 5.21 (d, J=2.93Hz, 4H), 7.70-7.86 (m, 8H), 7.86-8.01 (m, 3H).

### Examples 1 to 7: Synthesis of Nucleic Acid Complexes 1 to 7

Synthesis of SEQ-1 and SEQ-2 was outsourced to Gene Design Inc. Sodium tetraborate buffer at pH 8.5 (final concentration: 40 mM) was added to SEQ-2 (1.3 µmol), DBCO-NHS ester (CAS No. 1353016-71-3, 60 µmol) dissolved in DMSO was added thereto, and the resultant was stirred at room temperature for 15 minutes. After adding water to the reaction mixture, gel filtration purification was performed with a PD-10 column (GE Healthcare). Further, purification and concentration were performed with an Amicon Ultra 3K (Millipore) to afford a crude product (SEQ-3). To the crude product (40 nmol), triethylammonium acetate at pH 7.0 (final concentration: 50 mM) was added, and compound 8, 11, 14, 15, 17, 23, or 24 (400 nmol) dissolved in water was added thereto respectively, and the resultant was stirred at room temperature for 15 minutes. The reaction mixture was subjected to gel filtration purification with a PD-10 column (GE Healthcare). Further, purification and concentration were performed with an Amicon Ultra 3K (Millipore), thus providing nucleic acid complexes, specifically, nucleic acid complexes 1 to 7 corresponding to compounds 8, 11, 14, 15, 17, 23, and 24, respectively.

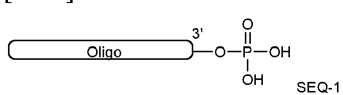

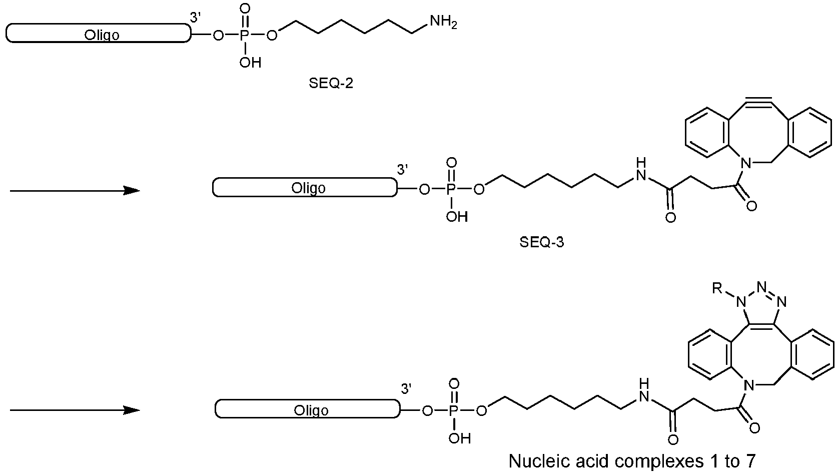

### Nucleic Acid Complex 1

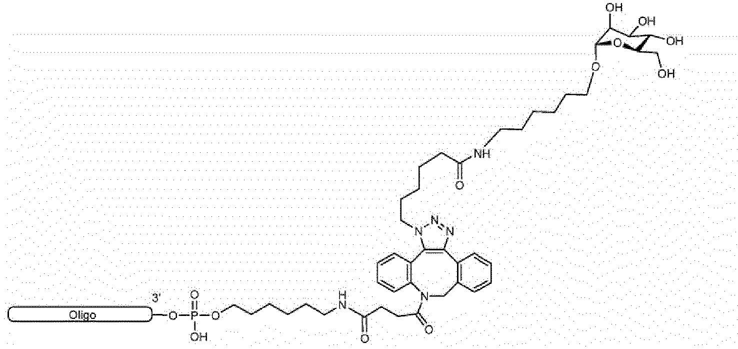

### Nucleic Acid Complex 2

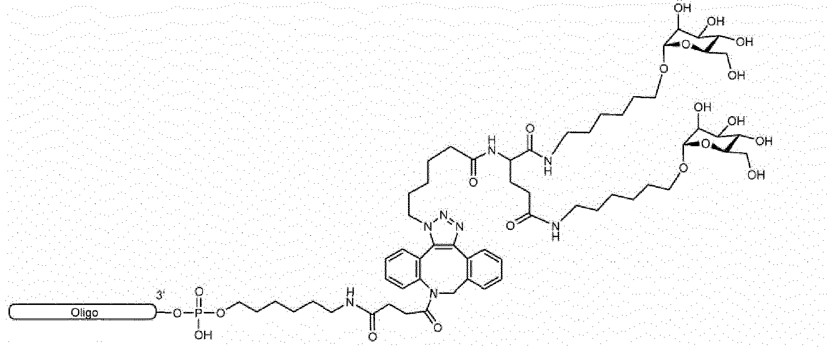

### Nucleic Acid Complex 3

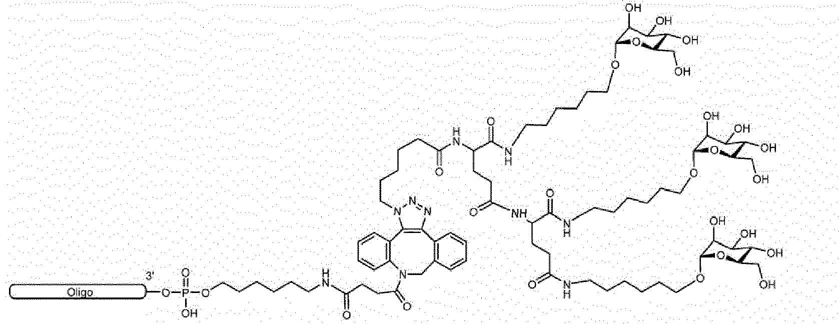

### Nucleic Acid Complex 4

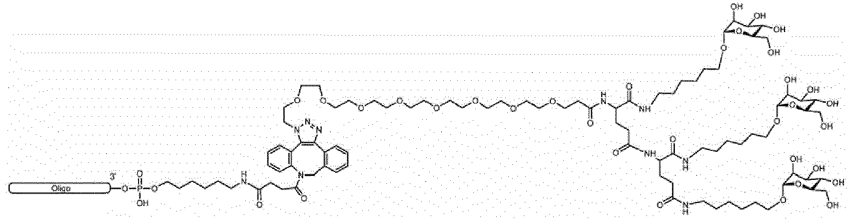

### Nucleic Acid Complex 5

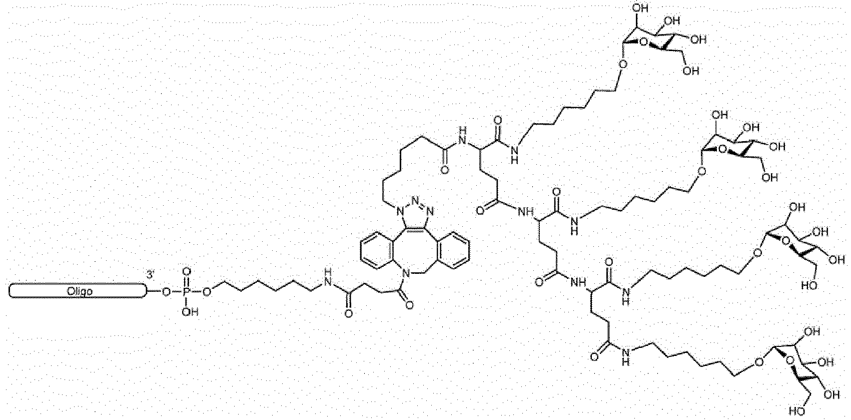

### Nucleic Acid Complex 6

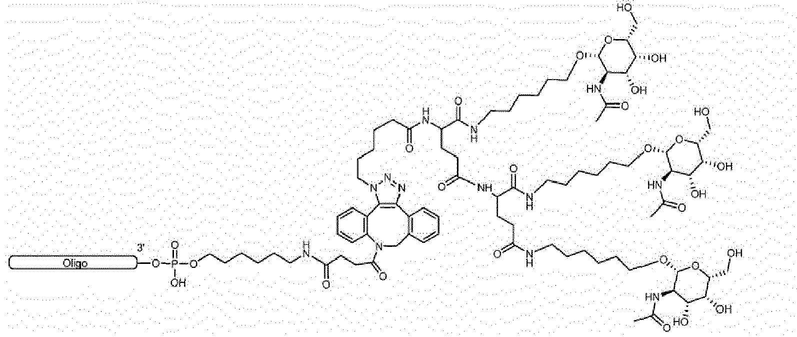

### Nucleic Acid Complex 7

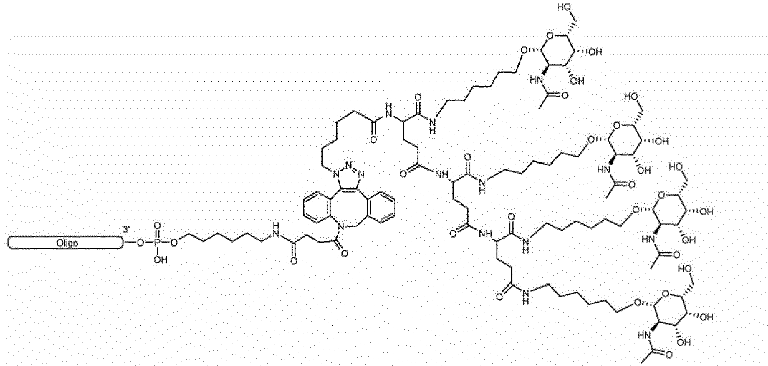

### Example 8: Synthesis of Nucleic Acid Complex 8

To SEQ-2 (26.6 nmol), sodium tetraborate buffer at pH 8.5 and compound 28 (120 nmol) dissolved in DMSO were added, and the resultant was stirred at room temperature. After adding water to the reaction mixture, purification was performed with an Amicon Ultra 3K (Millipore). To the crude product obtained, 28% aqueous ammonia in a volume five times that of the crude product was added, and the resultant was left to stand at room temperature for 3 hours. After adding water to the reaction mixture, purification was performed with an Amicon Ultra 3K (Millipore). Further, purification was performed by reversed-phase HPLC to afford nucleic acid complex 8.

### Nucleic Acid Complex 8

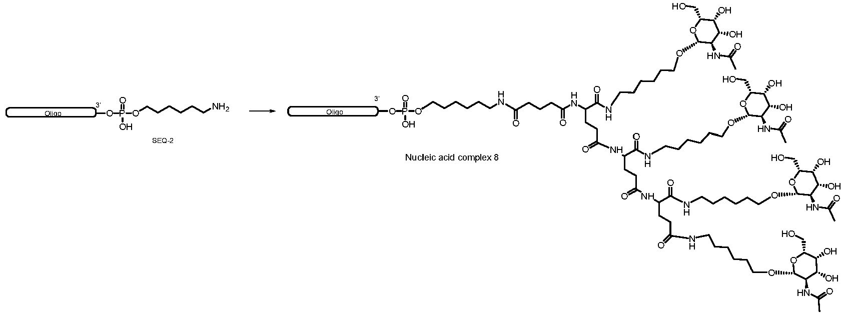

### Description of Nucleic Acid Sequence

The sequence of the nucleic acid (5'-3') used in Examples shown here is
A(F)^G(M)^G(F)A(M)C(F)U(M)G(F)G(M)U(F)C(M)U(F)U(F)U(M)C(F)U (M)A(F)U(M)A(F)U(M)^C(F)^U(M), wherein capital alphabets represent RNA, (M) indicates 2'-O-methylated RNA, (F) indicates 2'-fluoroRNA, and ^ indicates phosphorothioate linkage. The cyanine dye Cy3 (excitation wavelength: 555 nm, fluorescence wavelength: 570 nm), which is a fluorescent dye, is bound to the 5' end of each oligonucleotide. SEQ-1, 2 and the nucleic acid complexes synthesized in Examples shown here were subjected to measurement of molecular weight by MALDI-TOF-MS, and Table 1 shows the results.

**[Table 1]**

| Compound | Theoretical molecular weight | Found m/z [M-H]- |
|---|---|---|
| SEQ-1 | 7346.1 | 7345.5 |
| SEQ-2 | 7525.3 | 7526.4 |
| Nucleic acid complex 1 | 8231.1 | 8227.8 |
| Nucleic acid complex 2 | 8621.5 | 8620.1 |
| Nucleic acid complex 3 | 9011.9 | 9009.4 |
| Nucleic acid complex 4 | 9322.3 | 9321.1 |
| Nucleic acid complex 5 | 9402.4 | 9397.6 |
| Nucleic acid complex 6 | 9135.1 | 9131.1 |
| Nucleic acid complex 7 | 9236.2 | 9235.5 |
| Nucleic acid complex 8 | 9566.6 | 9566.0 |

### <Test Example 1>

### Evaluation of in vitro Uptake Activity of Human CD206-Expressing Lenti-X 293T Cells for Nucleic Acid Complexes

The nucleic acid complexes synthesized in Examples were each introduced into human CD206-expressing Lenti-X 293T cells (Clontech Laboratories, Inc.) with a method shown below, and the uptakes of them were evaluated. Human CD206-expressing Lenti-X 293T cells (Clontech Laboratories, Inc.) were seeded in a 96-well PDL-coating plate (Coming Incorporated) at 2 × 10⁴ cells/100 µL/well, and cultured in a 5% CO₂ incubator at 37°C for 2 days. SEQ-1 or any one of the nucleic acid complexes synthesized was added to reach a final concentration of 100 nmol/L, and incubation was performed in a 5% CO₂ incubator at 37°C for 2 hours. Thereto, 4% paraformaldehyde/PBS (Wako Pure Chemical Industries, Ltd.) containing 10 µg/mL Hoechst (Life Technologies) was added, and the cells were fixed at normal temperature for 30 minutes, and washed four times with PBS. Fluorescence imaging analysis was performed with an IN Cell Analyzer 2200 (GE Healthcare), wherein Cy3 fluorescence intensity in each well was corrected with the number of nuclei, and the average Cy3 fluorescence intensity per cell was calculated. At that time, the fluorescence intensity in the well with addition of SEQ-1, which is a nucleic acid without modification with a sugar ligand, was defined as 1, and fluorescence intensity in each well with addition of any one of the nucleic acid complexes was calculated as a relative value.

Table 2 shows the results. It was revealed from the results that, as compared with SEQ-1, which contained no sugar ligand, nucleic acid complex 6, which had GalNAc, was not effectively taken up by CD206-expressing cells, whereas nucleic acid complexes 1 to 5, nucleic acid complexes having mannose, were effectively taken up by CD206-expressing cells.

**[Table 2]**

| Specimen No. | SEQ-1 | Nucleic acid complex | | | | | |
|---|---|---|---|---|---|---|---|
| | | 1 | 2 | 3 | 4 | 5 | 6 |
| Ligand | - | Mannose | Mannose | Mannose | Mannose | Mannose | GalNAc |
| Valency | - | 1 | 2 | 3 | 3 | 4 | 3 |
| Relative value of fluorescence intensity (SEQ-1 = 1.0) | 1 | 7.5 | 12.6 | 13.8 | 15 | 14.5 | 1.1 |

### <Test Example 2>

### Evaluation of in vitro Uptake Activity of Human ASGR1-Expressing Lenti-X 293T Cells for Nucleic Acid Complexes

The nucleic acid complexes synthesized in Examples were each introduced into human ASGR1-expressing Lenti-X293T cells (Clontech Laboratories, Inc.) with a method shown below, and the uptake activity for them was evaluated. Human ASGR1-expressing Lenti-X293T cells (Clontech Laboratories, Inc.) were seeded in a 96-well PDL-coating plate (Corning Incorporated) at 2 × 10⁴ cells/100 µL/well, and cultured in a 5% CO₂ incubator at 37°C for 1 day or 2 days. SEQ-1 or any one of the nucleic acid complexes synthesized was added to reach a final concentration of 100 nmol/L, and incubation was performed in a 5% CO₂ incubator at 37°C for 2 hours. Subsequent fluorescence imaging analysis was performed with the same method as in Test Example 1.

Table 3 shows the results. It was revealed from the results that, as compared with SEQ-1, which contained no sugar ligand, nucleic acid complexes 1 to 5, which had mannose, were not effectively taken up by ASGR1-expressing cells, whereas nucleic acid complexes 6 to 8, which had GalNAc, were effectively taken up by ASGR1-expressing cells.

**[Table 3]**

| Specimen No. | SEQ-1 | Nucleic acid complex | | | | | | | |
|---|---|---|---|---|---|---|---|---|---|
| | | 1 | 2 | 3 | 4 | 5 | 6 | 7 | 8 |
| Ligand | - | Mannose | Mannose | Mannose | Mannose | Mannose | GalNAc | GalNAc | GalNAc |
| Valency | - | 1 | 2 | 3 | 3 | 4 | 3 | 4 | 4 |
| Relative value of fluorescence intensity (SEQ-1 = 1.0) | 1 | 2.1 | 1.4 | 1.3 | 0.9 | 1.1 | 44.9 | 64.5 | 53.3 |

### <Test Example 3>

### Evaluation of Uptake of Nucleic Acid Complexes in Mouse Liver Parenchymal Cells and Kupffer Cells (in vivo Evaluation)

SEQ-1, nucleic acid complex 3 and nucleic acid 6 were each subcutaneously administered to BALB/c mice (n = 3) at 1 mg/kg. Four hours after the administration, the liver was perfused and liver parenchymal cells and Kupffer cells were separated through a flow cytometer, and fluorescence intensity was measured. At that time, the fluorescence intensity for the group with administration of SEQ-1 was defined as 1, and fluorescence intensity for each group with administration of any one of the nucleic acid complexes was calculated as a relative value.

Table 4 shows the results. It was revealed from the results that, as compared with SEQ-1, which contained no sugar ligand, nucleic acid complex 3, which had mannose, was efficiently taken up by mouse Kupffer cells, which have been reported to be CD206-positive, and nucleic acid complex 6, which had GalNAc, was efficiently taken up by mouse liver parenchymal cells, which have been reported to be ASGR1-positive.

**[Table 4]**

| Specimen No. | SEQ-1 | Nucleic acid complex | |
|---|---|---|---|
| | | 3 | 6 |
| Ligand | - | Mannose | GalNAc |
| Valency | - | 3 | 3 |
| Liver parenchymal cells | 1 | 1.6 | 5.3 |
| Kupffer cells | 1 | 2.2 | 0.7 |

### Synthesis Scheme for Compound 37

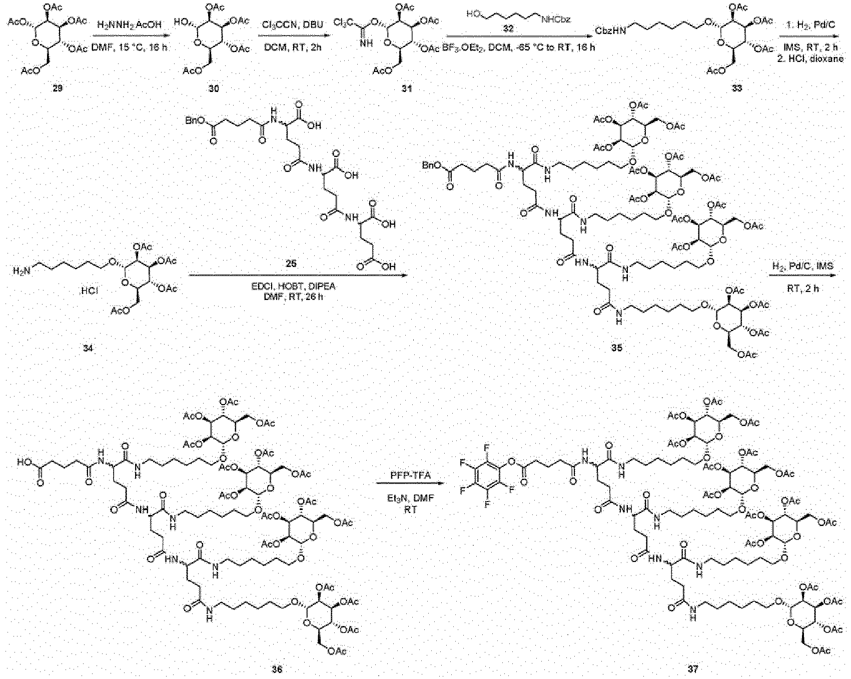

### Synthesis of Compound 30

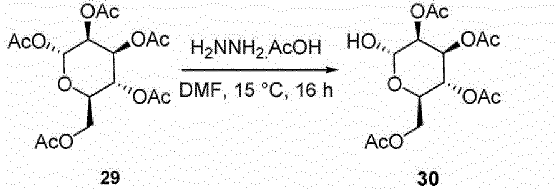

A mixture of hydrazine acetate (1.29 g, 14.06 mmol) and DMF (50 mL) was heated at 50°C. Subsequently, this was cooled in a water bath at 15°C, and α-D-mannose pentaacetate (4.99 g, 12.8 mmol) was added thereto. The resulting solution was stirred in a water bath at 15 to 19°C for 16 hours. To the mixture, water and EtOAc were added for extraction, and the aqueous layer was further subjected to extraction with EtOAc. The organic layer combined was washed with brine, dried over anhydrous sodium sulfate, filtered, and concentrated under reduced pressure. The residue was purified by column chromatography using a Biotage Isolera (100 g KP-Sil, 20 to 100% EtOAc/cyclohexane) to afford compound 30 (4.19 g) as a 9:1 mixture of α and β diastereomers.
α isomer
¹H NMR (600 MHz, CDCl₃) δ ppm:
2.00 (s, 3H), 2.05 (s, 3H), 2.11 (s, 3H), 2.16 (s, 3H), 3.00 (d, J=4.03Hz, 1H), 4.15 (br d, J=10.64Hz, 1H), 4.22-4.28 (m, 2H), 5.23-5.33 (m, 3H), 5.43 (br dd, J=10.27, 3.30Hz, 1H)

### Synthesis of Compound 31

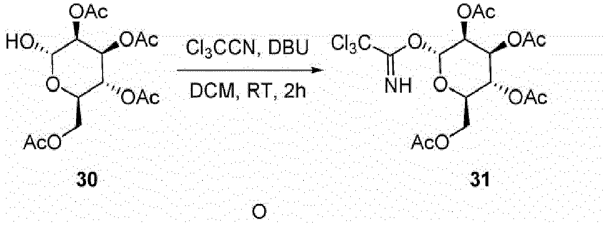

To a solution of compound 30 (3.64 g, 10.5 mmol) in DCM (218 mL), trichloroacetonitrile (10.48 mL, 104.5 mmol) and then DBU (0.158 mL, 1.05 mmol) were added dropwise in a nitrogen atmosphere. The mixture was stirred in a nitrogen atmosphere at room temperature for 2.25 hours. Concentration was performed under reduced pressure, and the residue was purified by column chromatography using a Biotage Isolera (100 g KP-Sil, 0 to 50% EtOAc/cyclohexane) to afford compound 31 (4.567 g).
¹H NMR (600 MHz, CDCl₃) δ ppm:
2.01 (s, 3H), 2.07 (s, 3H), 2.08 (s, 3H), 2.20 (s, 3H), 4.14-4.23 (m, 2H), 4.28 (dd, J=12.47, 4.77Hz, 1H), 5.38-5.43 (m, 2H), 5.47 (br s, 1H), 6.28 (s, 1H), 8.79(s, 1H)

### Synthesis of Compound 33

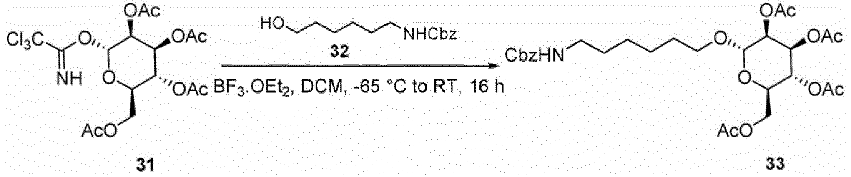

A solution of compound 31 (2.45 g, 4.96 mmol) and N-Cbz-6-amino-hexan-1-ol (1.87 g, 7.45 mmol) in DCM (90 mL) was cooled to 2°C (internal temperature), and boron trifluoride diethyl etherate (0.126 mL, 0.993 mmol) was added dropwise thereto in a nitrogen atmosphere. The resulting mixture was stirred at 2°C for 25 minutes, and then stirred at room temperature for 1 hour 20 minutes. To the mixture, saturated aqueous sodium hydrogen carbonate solution was added for extraction, and the aqueous layer was subjected to extraction with DCM. The organic layer combined was washed with brine, filtered through hydrophobic filter paper, and concentrated under reduced pressure. The residue was purified by column chromatography using a Biotage Isolera (100 g KP-Sil, 0 to 100% EtOAc/cyclohexane) to afford compound 33 (1.37 g).
¹H NMR (600 MHz, CDCl₃) δ ppm:
1.32-1.43 (m, 4H), 1.49-1.53 (m, 2H), 1.56-1.64 (m, 2H), 1.99 (s, 3H), 2.04 (s, 3H), 2.10 (s, 3H), 2.15 (s, 3H), 3.20 (q, J=6.72Hz, 2H), 3.40-3.48 (m, 1H), 3.64-3.71 (m, 1H), 3.97 (ddd, J=9.90, 5.32, 2.38Hz, 1H), 4.08-4.12 (m, 1H), 4.28 (dd, J=12.29, 5.32Hz, 1H), 4.79 (d, J=1.47Hz, 2H), 5.10 (s, 2H), 5.22 (dd, J=3.30, 1.83Hz, 1H), 5.28 (t, J=9.54Hz, 1H), 5.34 (dd, J=10.64, 3.67Hz, 1H), 7.29-7.33 (m, 1H), 7.36 (d, J=4.03Hz, 4H)

### Synthesis of Compound 34

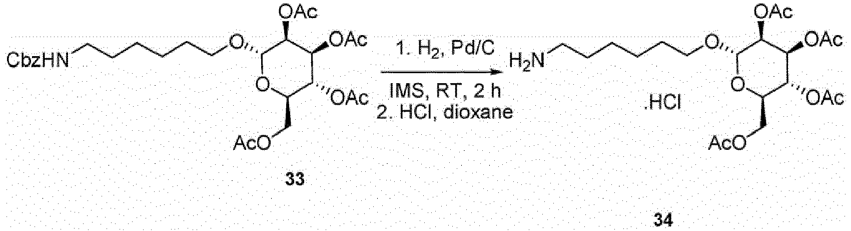

To a solution of compound 33 (1.35 g, 2.31 mmol) in IMS (38 mL), 10% palladium-carbon (0.293 g, 50% wet product) was added in a nitrogen atmosphere, and the resultant was stirred in a hydrogen atmosphere at room temperature for 1.5 hours. The mixture was filtered through a Celite (Registered Trademark), and washed with IMS. To the filtrate, 1,4-dioxane solution of 4 N hydrogen chloride (0.78 mL, 3.12 mmol) was added, and the resultant was concentrated under reduced pressure to afford compound 34 (1.06 g).
¹H NMR (600 MHz, DMSO-d₆) δ ppm: 1.30-1.37 (m, 4H), 1.51-1.61 (m, 4H), 1.94 (s, 3H), 2.03 (s, 6H), 2.11 (s, 3H), 2.72-2.80 (m, 2H), 3.42-3.50 (m, 1H), 3.63 (dt, J=9.72, 6.88Hz, 1H), 3.88-3.96 (m, 1H), 4.06 (dd, J=12.10, 2.57Hz, 1H), 4.15 (dd, J=12.29, 5.32Hz, 1H), 4.87 (s, 1H), 5.04-5.15 (m, 3H), 7.80 (br s, 3H)

### Synthesis of Compound 35

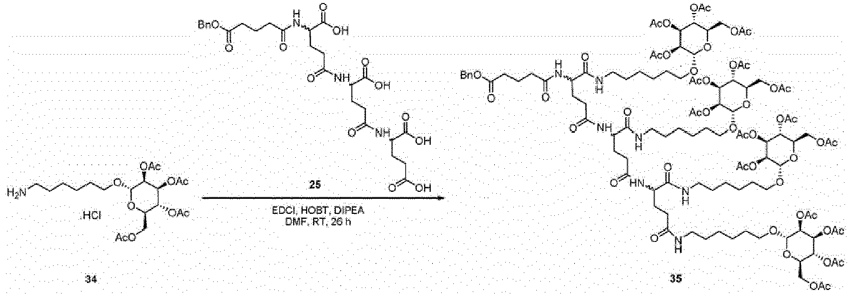

To a solution of compound 34 (510 mg, 1.05 mmol) and compound 25 (128.4 mg, 0.21 mmol) in DMF (3 mL), HOBt (161 mg, 1.05 mmol) and EDCI (202 mg, 1.05 mmol) were sequentially added. The resulting mixture was stirred at room temperature for 10 minutes, and DIPEA (0.18 mL, 1.05 mmol) was then added thereto. The resulting solution was stirred at room temperature for 22 hours. A solution of compound 34 (505.9 mg, 1.04 mmol) inDMF (1.5 mL), HOBt (161 mg, 1.05 mmol), and EDCI (202 mg, 1.05 mmol) were additionally added, and DIPEA (0.18 mL, 1.05 mmol) was added after 10 minutes. The mixture was further stirred for 3 hours. The mixture was purified by preparative HPLC to afford compound 34 (46.3 mg).
¹H NMR (600 MHz, DMSO-d₆) δ ppm:
1.21-1.45 (m, 24H), 1.49-1.59 (m, 8H), 1.61-1.89 (m, 8H), 1.93 (s, 12H), 2.01 (s, 24H), 2.10 (s, 12H), 2.07-2.23 (m, 8H), 2.33-2.37 (m, 2H), 2.96-3.08 (m, 7H), 3.41-3.48 (m, 4H), 3.57-3.65 (m, 4H), 3.88-3.94 (m, 4H), 4.05 (dd, J=12.10, 1.83Hz, 4H), 4.10-4.20 (m, 8H), 4.85 (s, 4H), 5.06-5.13 (m, 14H), 7.31-7.40 (m, 6H), 7.69-7.94 (m, 6H);
LCMS (ESI+): m/z 1164.58 [M+2H]²⁺.

Synthesis of Compound 36

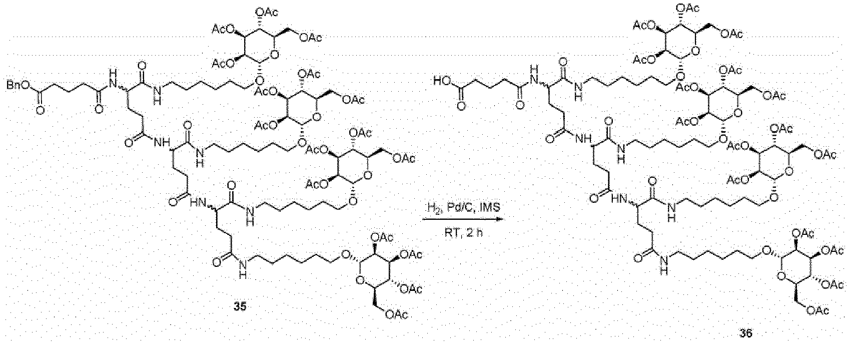

To a solution of compound 35 (40.7 mg, 0.02 mmol) in IMS (6 mL), 10% palladium-carbon (11.5 mg, 50% wet product) was added in a nitrogen atmosphere, and the resultant was stirred in a hydrogen atmosphere at room temperature for 2 hours. The mixture was filtered through a Celite (Registered Trademark), and washed with IMS. The filtrate combined was concentrated under reduced pressure to afford compound 36 (43.8 mg).
¹H NMR (600 MHz, DMSO-d₆) δ ppm:
1.20-1.44 (m, 24H), 1.51-1.60 (m, 8H), 1.61-1.88 (m, 8H), 1.93 (s, 12H), 2.02 (s, 12H), 2.02 (s, 12H), 2.10 (s, 12H), 2.12-2.27 (m, 10H), 2.96-3.08 (m, 7H), 3.41-3.48 (m, 4H), 3.58-3.65 (m, 4H), 3.88-3.94 (m, 4H), 4.04 (br d, J=10.27Hz, 4H), 4.09-4.29 (m, 8H), 4.85 (s, 4H), 5.04-5.14 (m, 12H), 7.71-8.01 (br m, 7H)

### Synthesis of Compound 37

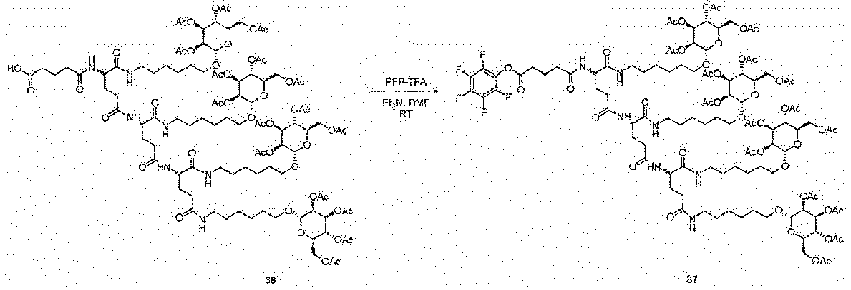

To a solution of compound 36 (32.8 mg, 0.015 mmol) in DMF (3 mL), trimethylamine (16.3 µL, 0.12 mmol) was added, and pentafluorophenyl trifluoroacetate (10.0 µL, 0.06 mmol) was then added dropwise, and the resulting mixture was stirred at room temperature for 1.25 hours. The reaction mixture was poured into water, brine was added thereto, and extraction was performed with EtOAc. The organic layer was washed sequentially with saturated aqueous sodium hydrogen carbonate solution, 1 M aqueous sodium hydrogen sulfate solution, and brine. The organic layer was dried over anhydrous sodium sulfate, filtered, and concentrated under reduced pressure. The residue was triturated with pentane. Subsequently, the solid was dried at 40°C under reduced pressure to afford compound 37 (36.7 mg).
¹H NMR (600 MHz, DMSO-d₆) δ ppm:
1.20-1.47 (m, 24H), 1.51-1.61 (m, 8H), 1.62-1.91 (m, 8H), 1.93 (s, 12H), 2.01 (s, 24H), 2.10 (s, 12H), 2.12-2.33 (m, 8H), 2.80 (br t, J=6.79Hz, 2H), 2.96-3.09 (m, 7H), 3.41-3.50 (m, 4H), 3.58-3.65 (m, 4H), 3.91 (br s, 4H), 4.04 (br d, J=12.10Hz, 4H), 4.10-4.28 (m, 8H), 4.85 (br s, 4H), 5.06-5.15 (m, 12H), 7.68-8.03 (m, 7H);
LCMS (ESI+): m/z 1202.46 [M+2H]²⁺.

### Synthesis Scheme for Compound 44

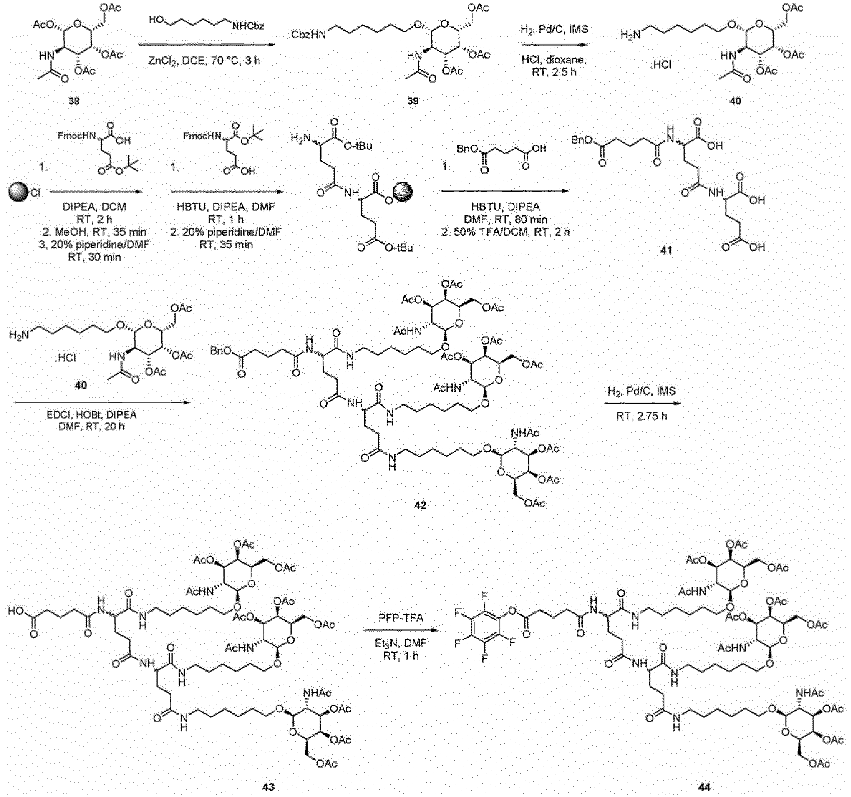

### Synthesis of Compound 39

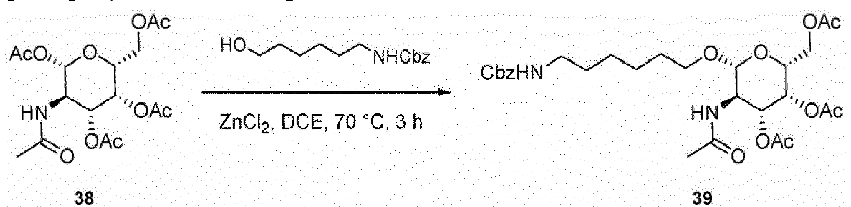

Anhydrous zinc chloride (3.65 g, 26.8 mmol) was weighed in a 250-mL three-necked flask under argon. The flask was dried under reduced pressure at 110°C for 1 hour, and then allowed to cool under reduced pressure overnight. Subsequently, the flask was filled with argon, and ((2S,3R,4R,5R,6R)-3-acetamide-6-(acetoxymethyl)tetrahydro-2H-pyran-2,4,5-triyltriacetate (7.85 g, 20.2 mmol), N-Cbz-6-amino-hexan-1-ol (6.74 g, 26.8 mmol), and DCE (85 mL) were added thereto. After the mixture was heated under argon at 70°C (external temperature) for 3 hours, the mixture was cooled, and then diluted with ethyl acetate and saturated aqueous sodium hydrogen carbonate solution. The mixture was stirred for 20 minutes, filtered through a Celite (Registered Trademark), and washed with ethyl acetate. The filtrate combined was partitioned, and the organic layer was washed with water and then with brine. The organic layer was dried over anhydrous sodium sulfate, filtered, and concentrated under reduced pressure. The residue was purified by column chromatography (330 g PuriFlash Si, 20 to 100% EtOAc/cyclohexane) using a CombiFlash to afford compound 39 (9.42 g).
¹H NMR (400 MHz, CDCl₃) δ ppm:
1.30-1.43 (m, 4H), 1.45-1.65 (m, 4H), 1.94 (s, 3H), 2.00 (s, 3H), 2.05 (s, 3H), 2.13 (s, 3H), 3.12-3.29 (m, 2H), 3.48 (dt, J=9.63, 6.50Hz, 1H), 3.79-4.02 (m, 3H), 4.06-4.21 (m, 2H), 4.65 (br d, J=8.19Hz, 1H), 4.78-4.93 (m, 1H), 5.06-5.18 (m, 2H), 5.27 (dd, J=11.25, 3.06Hz, 1H), 5.34 (d, J=2.81Hz, 1H), 5.77 (br d, J=8.44Hz, 1H), 7.29-7.42 (m, 5H)
LCMS (m/z 581 [M+H]⁺)

### Synthesis of Compound 40

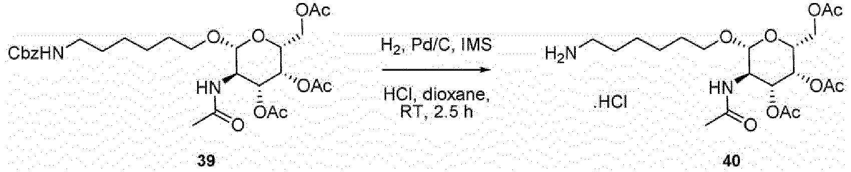

To a solution of compound 39 (9.23 g, 15.9 mmol) in IMS (275 mL), dioxane solution of 4 M hydrogen chloride (5.17 mL, 20.7 mmol) and 10% palladium-carbon (1.895 g; 50% wet product) were added in a nitrogen atmosphere, and the resultant was stirred in a hydrogen atmosphere at room temperature for 2.5 hours. The mixture was filtered through a Celite (Registered Trademark), and washed with IMS. The filtrate was concentrated under reduced pressure to afford compound 40 (8.07 g).
¹H NMR (400 MHz, DMSO-d₆) δ ppm:
1.22-1.35 (m, 4H), 1.39-1.62 (m, 4H), 1.78 (s, 3H), 1.89 (s, 3H), 2.00 (s, 3H), 2.10 (s, 3H), 2.69-2.82 (m, 2H), 3.41-3.47 (m, 2H), 3.64-3.77 (m, 1H), 3.81-3.93 (m, 1H), 3.95-4.12 (m, 2H), 4.49 (d, J=8.56Hz, 1H), 4.97 (dd, J=11.25, 3.42Hz, 1H), 5.22 (d, J=3.42Hz, 1H), 7.62-7.80 (m, 3H), 7.86 (d, J=9.29Hz, 1H)
LCMS (m/z 447 [M+H]⁺)

### Synthesis of Compound 41

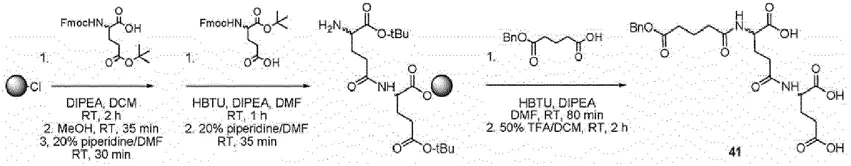

To a mixture of CTC resin (1.33 mmol/g, 1.31 g, 1.74 mmol), Fmoc-Glu(OtBu)-OH (0.370 g, 0.87 mmol), Fmoc-D-Glu(OtBu)-OH (0.370 g, 0.87 mmol), and DCM (105 mL) in a 250-mL Quickfit (Registered Trademark) Erlenmeyer flask, DIPEA (1.215 mL, 6.96 mmol) was added dropwise. The flask was plugged, equipped with a plate shaker, and shaken at 300 rpm for 2 hours. MeOH (0.7 mL, 17.3 mmol) was added thereto, and the mixture was further shaken for 35 minutes. The mixture was filtered through a 70-mL plastic phase separation cartridge, and treated with a solution of 20% piperidine in DMF (50 mL) for 30 minutes while bubbling with nitrogen was performed. The solution was discharged, and the resin was washed with DMF.

To the resin in the cartridge, a solution of Fmoc-D-Glu-OtBu (0.740 g, 1.74 mmol), N-Fmoc-L-glutamic acid-1-t-butyl ester (0.740 g, 1.74 mmol), and HBTU (1.253 g, 3.30 mmol) in DMF (10 mL) was added, and DIPEA (1.22 mL, 6.96 mmol) was then added dropwise. The mixture was mixed for 1 hour while bubbling with nitrogen was performed. Subsequently, the solution was removed of the solvent under nitrogen gas flow, and the resin was treated with a solution of 20% piperidine in DMF (50 mL) for 35 minutes while bubbling with nitrogen was performed. The solution was discharged, and the resin was washed with DMF.

To the residual resin in the cartridge, a solution of 1,5-pentanedioic acid monobenzyl ester (0.580 g, 2.61 mmol) and HBTU (1.253 g, 3.30 mmol) in DMF (10 mL) and then DIPEA (1.215 mL, 6.96 mmol) were added dropwise. The mixture was mixed for 80 minutes while bubbling with nitrogen was performed. The solution was discharged, and the resin was washed with DMF and then with MeOH. Subsequently, the resin was dried by suction. The resin was transferred into a test tube of 25 × 150 mm, dried under reduced pressure for 30 minutes, then treated with DCM (5 mL) and TFA (5 mL), and shaken with a plate shaker for 2 hours. Subsequently, the mixture was filtered through a phase separation cartridge, and washed with DCM. The filtrate was concentrated under reduced pressure to afford compound 41 (0.5859 g).
¹H NMR (400 MHz, DMSO-d₆) δ ppm:
1.68-1.84 (m, 4H), 1.86-2.01 (m, 2H), 2.13-2.23 (m, 4H), 2.23-2.29 (m, 2H), 2.38 (t, J=7.52Hz, 2H), 4.11-4.22 (m, 2H), 5.09 (s, 2H), 7.29-7.42 (m, 5H), 8.06-8.17 (m, 2H)
LCMS (m/z 481 [M+H]⁺)

### Synthesis of Compound 42

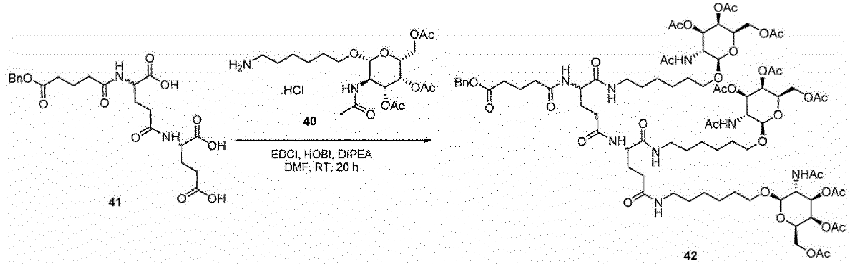

To a solution of compound 40 (4.06 g, 8.41 mmol) and compound 41 (0.5821 g, 1.21 mmol) in DMF (8 mL), HOBt (1.392 g, 9.09 mmol), EDCI (1.742 g, 9.09 mmol), and then DIPEA (1.587 mL, 9.09 mmol) were sequentially added. The mixture was stirred at room temperature for 20 hours. The mixture was purified by preparative HPLC to afford compound 42 (0.48 g).
¹H NMR (600 MHz, DMSO-d₆) δ ppm:
1.23 (br s, 12H), 1.29-1.40 (m, 6H), 1.40-1.48 (m, 6H), 1.60-1.71 (m, 2H), 1.76 (s, 9H), 1.76-1.87 (m, 4H), 1.89 (s, 9H), 1.99 (s, 9H), 2.00-2.07 (m, 2H), 2.10 (s, 9H), 2.11-2.15 (m, 2H), 2.18 (br t, J=7.15Hz, 2H), 2.34-2.38 (m, 2H), 2.94-3.07 (m, 6H), 3.36-3.43 (m, 3H), 3.65-3.73 (m, 3H), 3.82-3.91 (m, 3H), 3.98-4.06 (m, 9H), 4.10-4.20 (m, 2H), 4.48 (d, J=8.44Hz, 3H), 4.97 (dd, J=11.19, 3.12Hz, 3H), 5.08 (s, 2H), 5.21 (d, J=3.30Hz, 3H), 7.30-7.39 (m, 5H), 7.71-7.76 (m, 1H), 7.77-7.85 (m, 5H), 7.87-7.93 (m, 2H)
LCMS (m/z 1766 [M+H]⁺)

### Synthesis of Compound 43

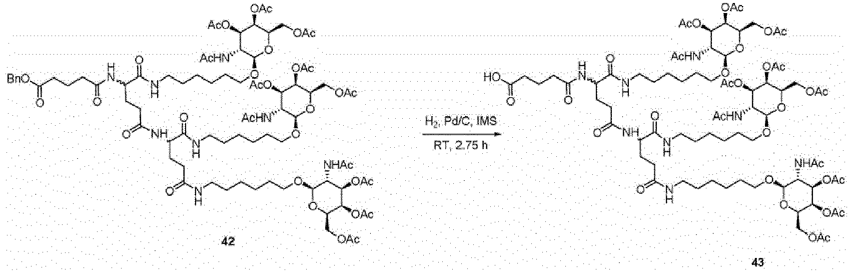

To a solution of compound 42 (0.43 g, 0.243 mmol) in IMS (85 mL), 10% palladium-carbon (0.158 g, 0.074 mmol; 50% wet product) was added in a nitrogen atmosphere, and the resultant was stirred in a hydrogen atmosphere at room temperature for 2.75 hours. The mixture was filtered through a Celite (Registered Trademark), and washed with IMS. The filtrate was concentrated under reduced pressure, and the residue was dissolved in DCM/EtOH (2:1), filtered through a Celite (Registered Trademark), and washed with DCM-EtOH (2:1). The filtrate was concentrated under reduced pressure to afford compound 43 (0.4252 g).
¹H NMR (600 MHz, DMSO-d₆) δ ppm:
1.18-1.29 (m, 12H), 1.31-1.41 (m, 6H), 1.41-1.49 (m, 6H), 1.62-1.75 (m, 4H), 1.77 (s, 9H), 1.81-1.87 (m, 2H), 1.89 (s, 9H), 1.99 (s, 9H), 2.01-2.08 (m, 2H),
   2.10 (s, 9H), 2.12-2.24 (m, 6H), 2.95-3.07 (m, 6H), 3.37-3.45 (m, 3H), 3.66-3.73 (m, 3H), 3.82-3.92 (m, 3H), 3.98-4.06 (m, 9H), 4.09-4.20 (m, 2H), 4.49 (d, J=8.44Hz, 3H), 4.97 (dd, J=11.19, 2.38Hz, 3H), 5.21 (d, J=3.30Hz, 3H), 7.73-8.04 (m, 8H), 11.81-12.20 (m, 1H).
LCMS (m/z 1676 [M+H]⁺).

### Synthesis of Compound 44

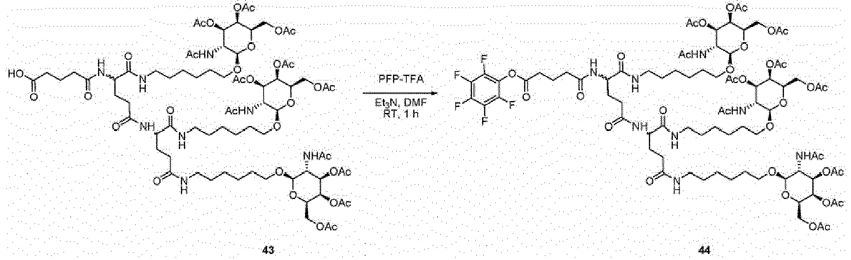

To a solution of compound 43 (0.4239 g, 0.253 mmol) in DMF (50 mL), triethylamine (0.282 mL, 2.024 mmol) and then pentafluorophenyl trifluoroacetate (0.174 mL, 1.012 mmol) were added dropwise in a nitrogen atmosphere. The mixture was stirred at room temperature for 1 hour, and poured into water. Brine was added to the aqueous layer, and extraction was performed with EtOAc. The organic layer combined was washed sequentially with saturated aqueous sodium hydrogen carbonate solution, 1 M aqueous sodium hydrogen sulfate solution, and brine. The organic layer was dried over anhydrous sodium sulfate, filtered, and then concentrated under reduced pressure, and EtOAc was added to the resulting residue, which was washed with 1 M aqueous sodium hydrogen sulfate solution and then with brine. The organic layer was dried over anhydrous sodium sulfate, filtered, and concentrated under reduced pressure to afford compound 44 (0.4513 g).
¹H NMR (600 MHz, DMSO-d₆) δ ppm:
1.24 (br s, 12H), 1.31-1.40 (m, 6H), 1.41-1.48 (m, 6H), 1.61-1.73 (m, 2H), 1.76 (s, 9H), 1.79-1.93 (m, 13H), 1.99 (s, 9H), 2.01-2.06 (m, 2H), 2.10 (s, 9H), 2.11-2.18 (m, 2H), 2.27 (br t, J=7.34Hz, 2H), 2.80 (t, J=6.97Hz, 2H), 2.96-3.07 (m, 6H), 3.37-3.43 (m, 3H), 3.69 (dt, J=9.63, 6.19Hz, 3H), 3.82-3.91 (m, 3H), 3.98-4.06 (m, 9H), 4.09-4.22 (m, 2H), 4.48 (d, J=8.44Hz, 3H), 4.97 (dd, J=11.37, 3.30Hz, 3H), 5.21 (d, J=3.30Hz, 3H), 7.73 (br t, J = 5.14Hz, 1H), 7.77-7.85 (m, 5H), 7.91 (br dd, J = 11.19, 7.89Hz, 1H), 7.98 (br d, J = 8.07Hz, 1H)
LCMS (m/z 1843.56 [M+H]⁺)

### Synthesis Scheme for Compound 47

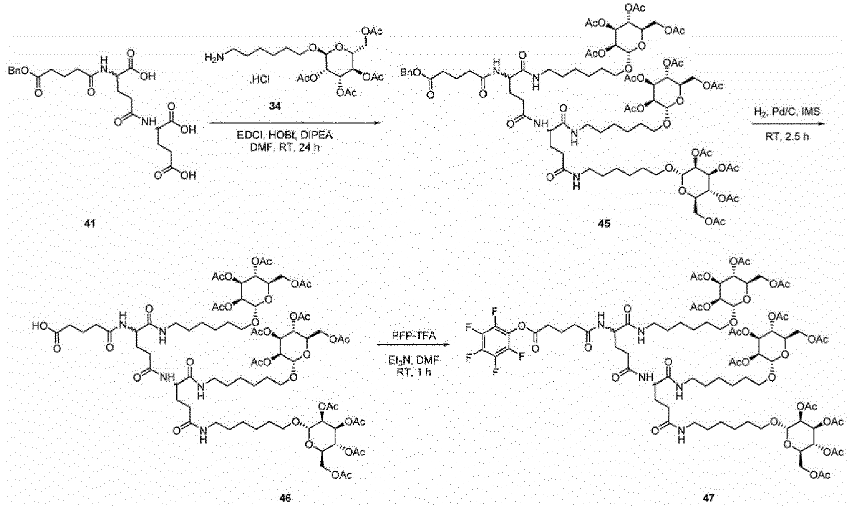

### Synthesis of Compound 45

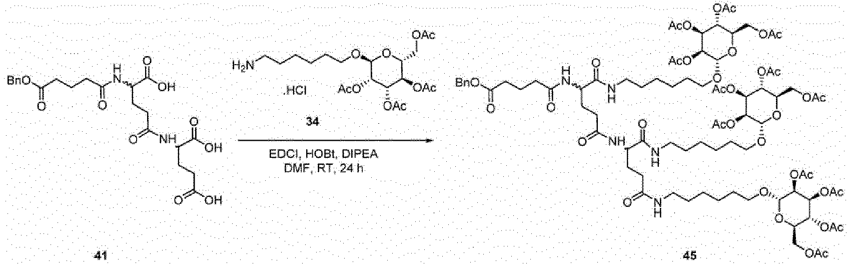

To a solution of compound 34 (1383 mg, 2.86 mmol) and compound 41 (183.1 mg, 0.381 mmol) in DMF (3.0 mL), HOBt (438 mg, 2.86 mmol), EDCI (548 mg, 2.86 mmol), and DIPEA (0.50 mL, 2.86 mmol) were sequentially added. The resulting mixture was stirred at room temperature for 24 hours. The mixture was purified by preparative HPLC to afford compound 45 (133.4 mg).
¹H NMR (600 MHz, DMSO-d₆) δ ppm:
1.23-1.34 (m, 12H), 1.34-1.43 (m, 6H), 1.50-1.59 (m, 6H), 1.62-1.72 (m, 2H), 1.74-1.80 (m, 2H), 1.81-1.89 (m, 2H), 1.93 (s, 9H), 2.01 (s, 18H), 2.03-2.07 (m, 2H), 2.10 (s, 9H), 2.11-2.15 (m, 2H), 2.16-2.21 (m, 2H), 2.33-2.38 (m, 2H), 2.97-3.07 (m, 6H), 3.41-3.48 (m, 3H), 3.57-3.64 (m, 3H), 3.87-3.94 (m, 3H), 4.05 (dd, J=12.10, 2.57Hz, 3H), 4.10-4.19 (m, 5H), 4.85 (s, 3H), 5.0-5.13 (m, 11H), 7.29-7.39 (m, 5H), 7.71 (br t, J=4.95Hz, 1H), 7.76-7.84 (m, 2H), 7.85-7.91 (m, 2H)
LCMS (m/z 1769 [M+H]⁺)

### Synthesis of Compound 46

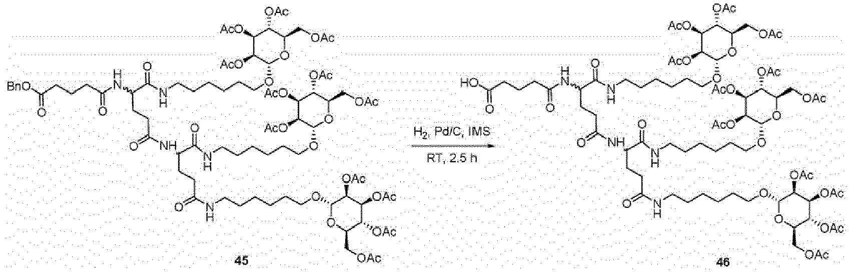

To a solution of compound 45 (101.9 mg, 0.058 mmol) in IMS (20 mL), 10% palladium-carbon (37.4 mg; 50% wet product) was added in a nitrogen atmosphere. The mixture was stirred in a hydrogen atmosphere at room temperature for 2.5 hours. The mixture was filtered through a Celite (Registered Trademark), and then washed with IMS. The filtrate combined was concentrated under reduced pressure to afford compound 46 (95.9 mg).
¹H NMR (600 MHz, DMSO-d₆) δ ppm:
1.28 (br s, 12H), 1.34-1.44 (m, 6H), 1.49-1.60 (m, 6H), 1.61-1.76 (m, 4H), 1.81-1.90 (m, 2H), 1.93 (s, 9H), 2.02 (s, 9H), 2.02 (s, 9H), 2.04-2.08 (m,2H), 2.10 (s, 9H), 2.12-2.23 (m, 6H), 2.96-3.07 (m, 6H), 3.40-3.48 (m, 3H), 3.57-3.65 (m, 3H), 3.86-3.94 (m, 3H), 4.05 (dd, J=12.10, 2.38Hz, 3H), 4.09-4.18 (m, 5H), 4.85 (s, 3H), 5.01-5.14 (m, 9H), 7.62-8.17 (m, 5H), 11.84-12.15 (m, 1H).
LCMS (m/z 1679 [M+H]⁺).

### Synthesis of Compound 47

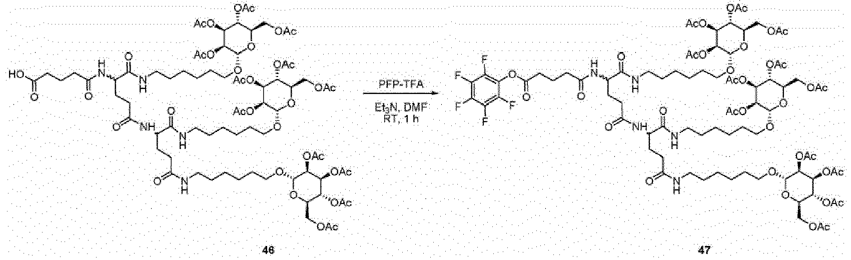

To a solution of compound 46 (94.6 mg, 0.056 mmol) in DMF (9 mL), triethylamine (62.8 µL, 0.451 mmol) was added in a nitrogen atmosphere, and pentafluorophenyl trifluoroacetate (38.7 µL, 0.225 mmol) was added dropwise. The resulting mixture was stirred at room temperature for 1 hour. The mixture was poured into water, brine was added thereto, and extraction was performed with EtOAc. The organic layer combined was washed sequentially with saturated aqueous sodium hydrogen carbonate solution, 1 M aqueous sodium hydrogen sulfate solution, and brine. The organic layer was dried over anhydrous sodium sulfate, filtered, and then concentrated under reduced pressure to afford compound 47 (109.0 mg).
¹H NMR (600 MHz, DMSO-d₆) δ ppm:
1.23-1.33 (m, 12H), 1.34-1.43 (m, 6H), 1.50-1.60 (m, 6H), 1.62-1.77 (m, 2H), 1.80-1.91 (m, 4H), 1.93 (s, 9H), 2.01 (s, 18H), 2.03-2.07 (m, 2H), 2.10 (s, 9H), 2.12-2.20 (m, 2H), 2.25-2.30 (m, 2H), 2.77-2.82 (m, 2H), 2.96-3.08 (m, 6H), 3.41-3.48 (m, 3H), 3.53-3.68 (m, 3H), 3.86-3.95 (m, 3H), 4.00-4.07 (m, 3H), 4.10-4.23 (m, 5H), 4.85 (s, 3H) 5.04-5.13 (m, 9H), 7.71 (br t, J=5.32Hz, 1H), 7.78-7.85 (m, 2H), 7.89 (dd, J=12.84, 8.07Hz, 1H), 7.96 (d, J=8.07Hz, 1H)
LCMS (m/z 1845.47 [M+H]⁺)

### Synthesis Scheme for Compounds 53a, 53b, and 53c

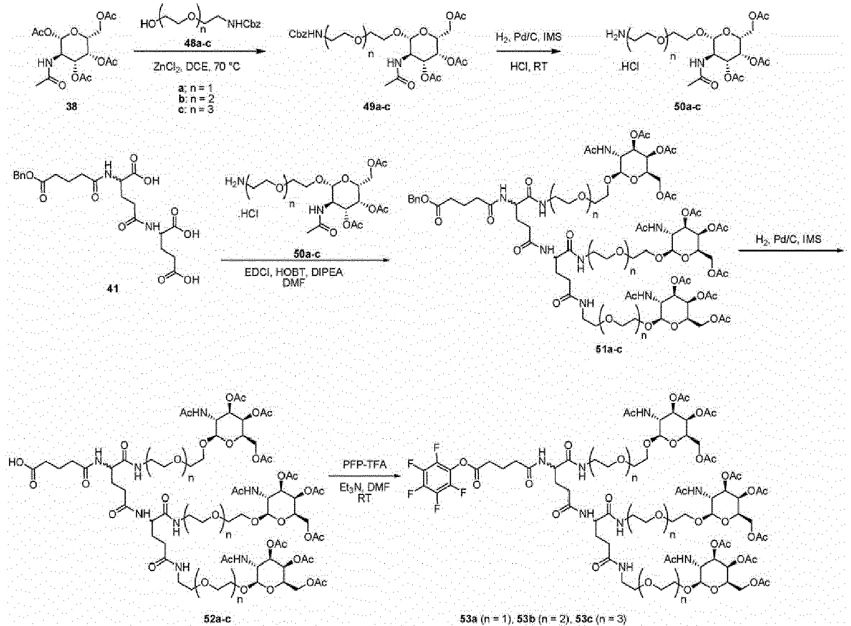

### Synthesis of Compound 49a

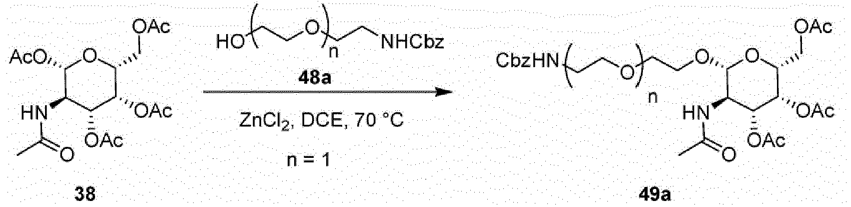

Zinc chloride (285 mg, 2.09 mmol) put in a 50-mL three-necked flask was dried under reduced pressure at 110°C for 1 hour, and allowed to cool under reduced pressure overnight. The flask was purged with nitrogen, and a solution of compound 38 (611 mg, 1.57 mmol) and compound 48a (500 mg, 2.09 mmol) in DCE (5 mL) was added thereto. The mixture was heated in a nitrogen atmosphere at 70°C (external temperature) for 3 hours. The mixture was allowed to cool, and diluted with ethyl acetate (10 mL)/saturated aqueous sodium hydrogen carbonate solution (5 mL). The mixture was stirred for 10 minutes, filtered through a Celite (Registered Trademark), and washed with ethyl acetate. The filtrate combined was washed with water and brine. The organic layer was filtered through hydrophobic filter paper, and concentrated under reduced pressure. The residue was purified by column chromatography using a Biotage Isolera (50 g KP-Sil, 0 to 50% (3:1 EtOAc-IMS)/MTBE) to afford compound 49a (599.4 mg).
¹H NMR (600 MHz, CDCl₃) δ ppm:
1.90 (s, 3H), 2.00 (s, 3H), 2.04 (s, 3H), 2.13 (s, 3H), 3.26-3.86 (m, 8H), 3.87-4.00 (m, 2H), 4.05-4.19 (m, 2H), 4.76 (br d, J=7.70Hz, 1H), 5.10-5.20 (m, 2H), 5.26 (br dd, J=6.60, 3.30Hz, 1H), 5.32 (br s, 1H), 5.43 (br s, 1H), 5.75 (br d, J=7.34Hz, 1H), 7.28-7.42 (m, 5H).
LCMS (m/z 569 [M+H]⁺).

### Synthesis of Compound 50a

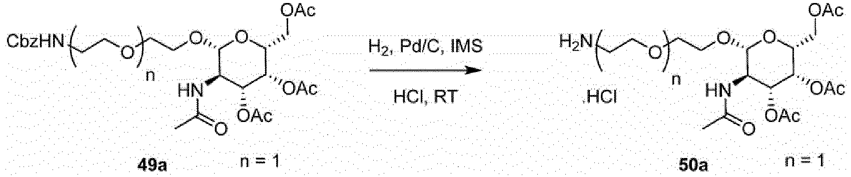

To a solution of compound 49a (594.2 mg, 1.05 mmol) in IMS (18 mL), 1,4-dioxane solution of 4 M hydrogen chloride (0.34 mL, 1.36 mmol) and 10% palladium-carbon (50% wet product, 125 mg) were added in a nitrogen atmosphere. The mixture was stirred in a hydrogen atmosphere at room temperature for 2.5 hours. The mixture was filtered through a Celite (Registered Trademark), and washed with IMS. The filtrate combined was concentrated under reduced pressure to afford compound 50a (507.9 mg).
¹H NMR (600 MHz, DMSO-d₆) δ ppm:
1.77-1.84 (m, 3H), 1.86-1.92 (m, 3H), 1.97-2.03 (m, 3H), 2.06-2.13 (m, 3H), 2.92-3.00 (m, 2H), 3.52-3.75 (m, 5H), 3.83 (ddd, J=11.28, 5.41, 3.85Hz, 1H), 3.86-3.93 (m, 1H), 3.95-4.17 (m, 3H), 4.57 (d, J=8.44Hz 1H), 4.99 (dd,J=11.19, 3.48Hz, 1H), 5.23 (d, J=3.67Hz, 1H), 7.81 (br s, 3H), 7.85-7.92 (m, 1H)
LCMS (m/z 435 [M+H]⁺)

### Synthesis of Compound 51a

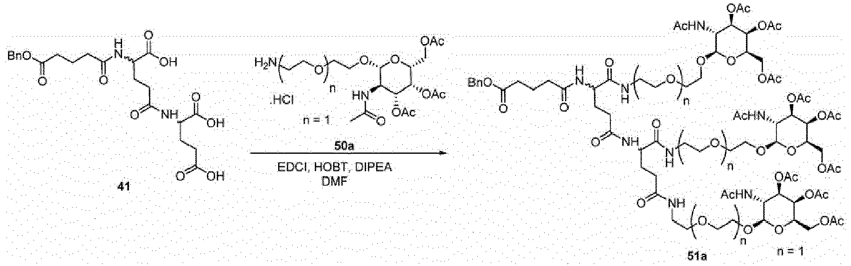

To a solution of compound 50a (506.5 mg, 1.08 mmol) and compound 41 (68.9 mg, 0.14 mmol) in DMF (2.0 mL), HOBt (165 mg, 1.08 mmol), EDCI (206 mg, 1.08 mmol), and DIPEA (188 µL, 1.08 mmol) were sequentially added. The resulting mixture was stirred at room temperature for 20 hours. The mixture was purified by preparative HPLC to afford compound 51a (40.0 mg).
¹H NMR (600 MHz, DMSO-d₆) δ ppm 1.62-1.73 (m, 2H), 1.77 (s, 9H), 1.80-1.87 (m, 2H), 1.89 (s, 9H), 1.99 (s, 9H), 2.02-2.08 (m, 2H), 2.10 (s, 9H), 2.11-2.24 (m, 4H), 2.34-2.38 (m, 2H), 3.13-3.22 (m, 4H), 3.34-3.42 (m, 7H), 3.44-3.54 (m, 7H), 3.55-3.61 (m, 3H), 3.73-3.80 (m, 3H), 3.84-3.91 (m, 3H), 3.97-4.08 (m, 9H), 4.13-4.25 (m, 2H), 4.55 (dd, J=8.07, 3.67Hz, 3H), 4.99 (br d, J=11.00Hz, 3H), 5.07-5.10 (m, 2H), 5.22 (d, J=3.30Hz, 3H), 7.31-7.40 (m, 5H), 7.60-7.97 (m, 8H)
LCMS (m/z 1730/1731 [M+H]⁺)

### Synthesis of Compound 52a

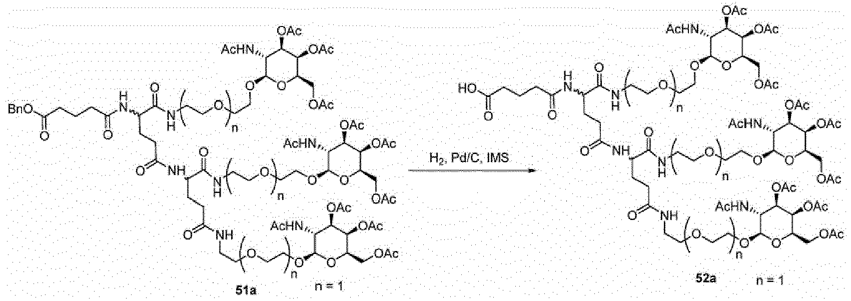

To a solution of compound 51a (42.7 mg, 0.03 mmol) in IMS (8 mL), 10% palladium-carbon (50% wet product; 16.0 mg) was added in a nitrogen atmosphere. The mixture was stirred in a hydrogen atmosphere at room temperature for 2.5 hours. The mixture was filtered through a Celite (Registered Trademark), and washed with IMS. The filtrate was concentrated under reduced pressure to afford compound 52a (41.2 mg).
¹H NMR (600 MHz, DMSO-d₆) δ ppm 1.62-1.75 (m, 4H), 1.78 (s, 9H), 1.81-1.87 (m, 2H), 1.89 (s, 9H), 2.00 (s, 9H), 2.05-2.32 (m, 17H), 3.12-3.23 (m, 4H), 3.36-3.43 (m, 7H), 3.45-3.54 (m, 7H), 3.55-3.61 (m, 3H), 3.74-3.81 (m, 3H), 3.83-3.92 (m, 3H), 3.98-4.08 (m, 9H), 4.11-4.23 (m, 2H), 4.51-4.59 (m, 3H), 4.99 (br d, J=11.00Hz, 3H), 5.22 (d, J=3.30Hz, 3H), 7.62-8.04 (m, 8H)
LCMS (m/z 1640/1641 [M+H]⁺)

### Synthesis of Compound 53a

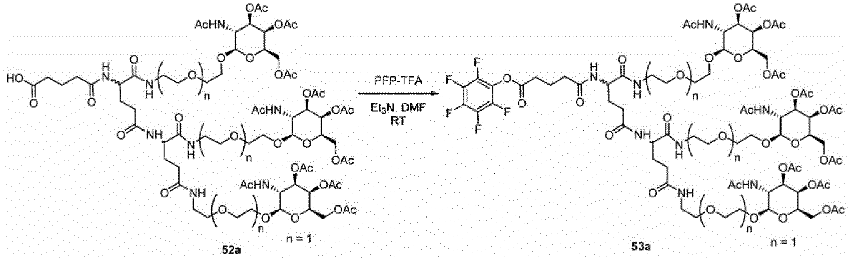

To a solution of compound 52a (41.6 mg, 0.03 mmol) in DMF (4 mL), triethylamine (28 µL, 0.20 mmol) and then pentafluorophenyl trifluoroacetate (17 µL, 0.10 mmol) were added dropwise in a nitrogen atmosphere. The resulting mixture was stirred at room temperature for 1.5 hours and poured into water, brine was added thereto, and extraction was performed with EtOAc. The organic layer combined was washed sequentially with saturated aqueous sodium hydrogen carbonate solution, 1 M aqueous sodium hydrogen sulfate solution, and brine. The organic layer was dried over anhydrous sodium sulfate, filtered, and concentrated under reduced pressure. The residue was transferred into a sample vial containing DCM/MeOH, the solvent was evaporated, and then dried under reduced pressure at 40°C for 2 hours to afford compound 52c (27.1 mg).
¹H NMR (600 MHz, DMSO-d₆) δ ppm:
1.57-1.75 (m, 4H), 1.77 (s, 9H), 1.86-1.95 (m, 11H), 2.00 (s, 9H), 2.05-2.33 (m, 15H), 2.77-2.88 (m, 2H), 3.11-3.24 (m, 4H), 3.41-3.54 (m, 14H), 3.55-3.62 (m, 3H), 3.74-3.81 (m, 3H), 3.84-3.91 (m, 3H), 3.98-4.07 (m, 9H), 4.11-4.23 (m, 2H), 4.55 (br dd, J=8.07, 3.67Hz, 3H), 4.99 (br d, J=11.00Hz, 3H), 5.22 (d, J=3.30Hz, 3H), 7.65-8.04 (m, 8H)
LCMS (m/z 1806.83 [M+H]⁺)

### Synthesis of Compound 49b

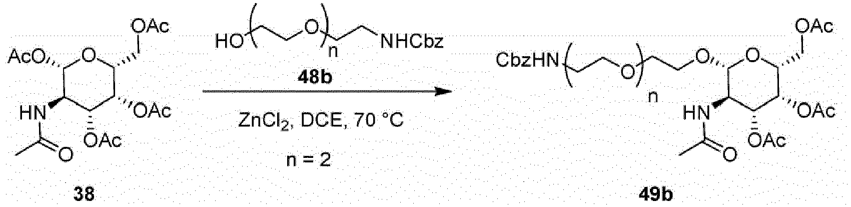

Zinc chloride (0.466 g, 3.42 mmol) put in a 50-mL three-necked flask was dried under reduced pressure at 110°C for 95 minutes, and allowed to cool under reduced pressure overnight. The flask was purged with nitrogen, and a solution of compound 38 (1.00 g, 2.57 mmol) and compound 48b (0.97 g, 3.42 mmol) in DCE (10 mL) was added thereto. The mixture was heated in a nitrogen atmosphere at 70°C (external temperature) for 3 hours. The mixture was allowed to cool, and diluted with ethyl acetate (20 mL)/saturated aqueous sodium hydrogen carbonate solution (10 mL). The mixture was stirred for 15 minutes, filtered through a Celite (Registered Trademark), and washed with ethyl acetate. The filtrate was washed with water and brine. The organic layer was filtered through hydrophobic filter paper, and concentrated under reduced pressure. The residue was purified by column chromatography using a Biotage Isolera (100 g Sfar Duo Si, 0 to 50% (3:1 EtOAc-IMS)/MTBE) to afford compound 49b (1.14 g). ¹H NMR (600 MHz, CDCl₃) δppm:
1.92 (s, 3H), 1.98 (s, 3H), 2.03 (s, 3H), 2.14 (s, 3H), 3.32-3.50 (m, 2H), 3.53-3.73 (m, 8H), 3.77-3.94 (m, 3H), 4.02-4.22 (m, 3H), 4.74 (br d, J=8.44Hz, 1H), 5.03-5.08 (m, 1H), 5.11 (br s, 2H), 5.26 (br s, 1H), 5.40-5.53 (m, 1H), 6.11-6.21 (m, 1H), 7.30-7.40 (m, 5H).
LCMS (m/z 613 [M+H]⁺).

### Synthesis of Compound 50b

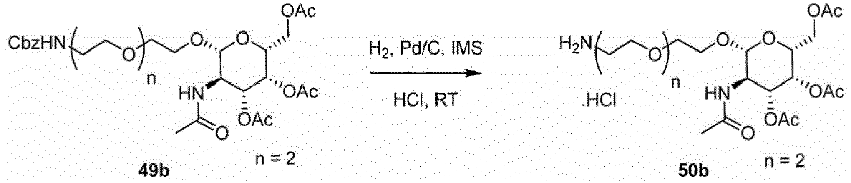

To a solution of compound 49b (1.14 g, 1.86 mmol) in IMS (32 mL), 1,4-dioxane solution of 4 M hydrogen chloride (0.60 mL, 2.42 mmol) and 10% palladium-carbon (50% wet product; 0.221 g) were added in a nitrogen atmosphere. The mixture was stirred in a hydrogen atmosphere at room temperature for 2.5 hours. The mixture was filtered through a Celite (Registered Trademark), and washed with IMS. The filtrate combined was concentrated under reduced pressure to afford compound 50b (0.97 g).
¹H NMR (600 MHz, DMSO-d₆) δ ppm 1.79 (s, 3H), 1.89 (s, 3H), 2.00 (s, 3H,) 2.11 (s, 3H), 2.94-3.02 (m, 2H), 3.50-3.62 (m, 9H), 3.77-3.84 (m, 1H), 3.86-3.94 (m, 1H), 3.98-4.10 (m, 3H), 4.54 (d, J=8.44Hz, 1H), 4.97 (dd, J=11.19, 3.48Hz, 1H), 5.22 (d, J=3.67Hz, 1H), 7.70-7.93 (m, 4H)
LCMS (m/z 479 [M+H]⁺)

### Synthesis of Compound 51b

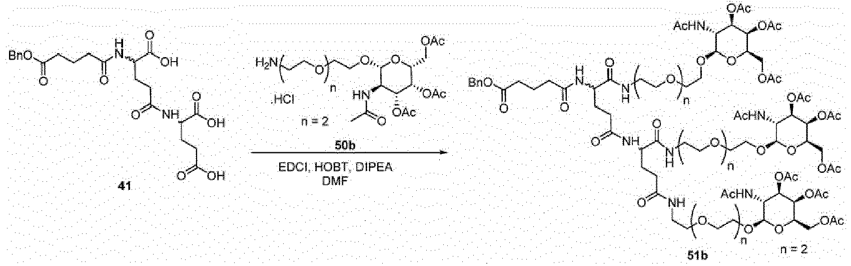

To a solution of compound 50b (556 mg, 1.08 mmol) and compound 41 (74.1 mg, 0.15 mmol) in DMF (3 mL), HOBt (165 mg, 1.08 mmol), EDCI (207 mg, 1.08 mmol), and DIPEA (189 µL, 1.08 mmol) were sequentially added. The resulting mixture was stirred at room temperature for 19 hours. The mixture was purified by preparative HPLC to afford compound 51b (67.5 mg).
¹H NMR (600 MHz, DMSO-d₆) δ ppm 1.61-1.74 (m, 2H), 1.77 (s, 9H), 1.80-1.85 (m, 2H), 1.89 (s, 9H), 2.00 (s, 9H), 2.03-2.08 (m, 2H), 2.10 (s, 9H), 2.12-2.24 (m, 4H), 2.35-2.38 (m, 2H), 3.10-3.23 (m, 4H), 3.36-3.42 (m, 7H), 3.44-3.63 (m, 22H), 3.74-3.81 (m, 3H), 3.83-3.91 (m, 3H), 3.99-4.08 (m, 9H), 4.13-4.25 (m, 2H), 4.55 (d, J=8.44Hz, 3H), 4.95-5.00 (m, 3H), 5.09 (s, 2H), 5.21 (d, J=3.30Hz, 3H), 7.31-7.41 (m, 5H), 7.72-7.86 (m, 4H), 7.86-7.99 (m, 4H)
LCMS (m/z 1862/1863 [M+H]⁺)

### Synthesis of Compound 52b

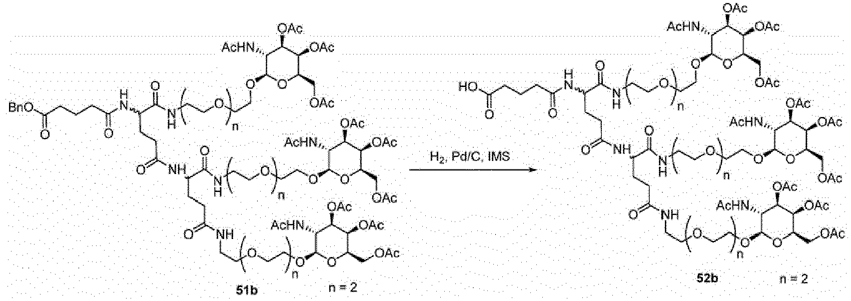

To a solution of compound 51b (66.1 mg, 0.04 mmol) in IMS (13 mL), 10% palladium-carbon (50% wet product; 23.1 mg) was added in a nitrogen atmosphere. The mixture was stirred in a hydrogen atmosphere at room temperature for 2.5 hours. The mixture was filtered through a Celite (Registered Trademark), and washed with IMS. The filtrate combined was concentrated under reduced pressure to afford compound 52b (61.3 mg).
¹H NMR (600 MHz, DMSO-d₆) δ ppm 1.63-1.75 (m, 4H), 1.77 (s, 9H), 1.81-1.87 (m, 2H), 1.89 (s, 9H),2.00 (s, 9H), 2.03-2.24 (m, 17H), 3.09-3.23 (m, 4H), 3.35-3.43 (m, 7H), 3.44-3.64 (m, 22H), 3.74-3.81 (m, 3H), 3.84-3.91 (m, 3H), 3.98-4.07 (m, 9H), 4.10-4.24 (m, 2H), 4.56 (br d, J=8.80Hz, 3H), 4.98 (br d, J=11.00Hz, 3H), 5.22 (d, J=2.93Hz, 3H), 7.70-8.06 (m, 8H).
LCMS (m/z 1772/1773 [M+H]⁺)

### Synthesis of Compound 53b

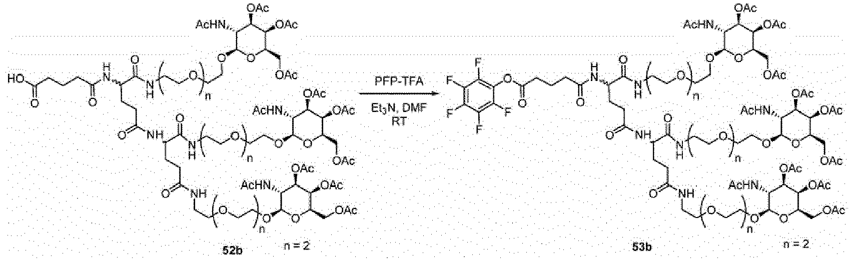

To a solution of compound 52b (60.7 mg, 0.03 mmol) in DMF (6 mL), triethylamine (38 µL, 0.27 mmol) and then pentafluorophenyl trifluoroacetate (24 µL, 0.14 mmol) were added dropwise in a nitrogen atmosphere. The resulting mixture was stirred at room temperature for 77 minutes and poured into water, brine was added thereto, and extraction was performed with EtOAc. The organic layer combined was washed sequentially with saturated aqueous sodium hydrogen carbonate solution, 1 M aqueous sodium hydrogen sulfate solution, 4% aqueous lithium chloride solution, and brine. The organic layer was dried over anhydrous sodium sulfate, filtered, then concentrated, and dried under reduced pressure at 40°C for 4 hours to afford compound 53b (29.3 mg). ¹H NMR (600 MHz, DMSO-d₆) δ ppm:
1.62-1.75 (m, 2H), 1.77 (s, 9H), 1.85-1.95 (m, 13H), 2.00 (s, 9H), 2.10 (s, 9H), 2.12-2.33 (m, 6H), 2.78-2.86 (m, 2H), 3.12-3.24 (m, 4H), 3.35-3.44 (m, 7H), 3.45-3.64 (m, 22H), 3.75-3.81 (m, 3H), 3.83-3.92 (m, 3H), 3.99-4.06 (m, 9H), 4.12-4.26 (m, 2H), 4.55 (d, J=8.44Hz, 3H), 4.98 (dd, J=11.19, 2.02Hz, 3H), 5.21 (d, J=3.67Hz, 3H), 7.64-8.03 (m, 8H)
LCMS (m/z 1939.01 [M+H]⁺)

### Synthesis of Compound 49c

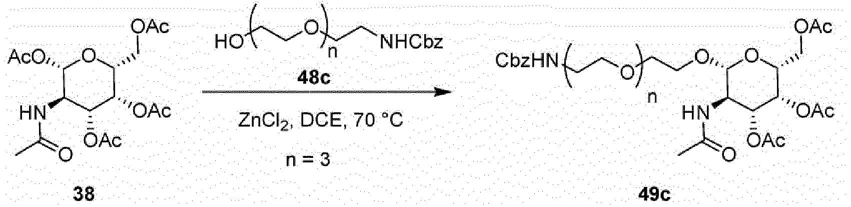

Zinc chloride (210 mg, 1.54 mmol) put in a 50-mL three-necked flask was dried under reduced pressure at 110°C for 75 minutes, and allowed to cool under reduced pressure overnight. The flask was purged with nitrogen, and a solution of compound 38 (450.2 mg, 1.16 mmol) and compound 48c (503.4 mg, 1.54 mmol) in DCE (5 mL) was added thereto. The mixture was heated in an argon atmosphere at 70°C (external temperature) for 3 hours. The mixture was allowed to cool, and diluted with ethyl acetate (10 mL)/saturated aqueous sodium hydrogen carbonate solution (5 mL). The mixture was stirred for 15 minutes, filtered through a Celite (Registered Trademark), and washed with ethyl acetate. The filtrate was washed with water and brine. The organic layer was filtered through hydrophobic filter paper, and concentrated under reduced pressure. The residue was purified by column chromatography using a Biotage Isolera (50 g Sfar Si, 0 to 50% (3:1 EtOAc-IMS)/MTBE) to afford compound 49c (469.0 mg).
¹H NMR (600 MHz, CDCl₃) δ ppm:
1.95 (s, 3H), 1.96 (s, 3H), 2.04 (s, 3H), 2.15 (s, 3H), 3.39 (br d, J=4.77Hz, 2H), 3.52-3.72 (m, 12H), 3.79-3.94 (m, 3H), 4.09-4.14 (m, 1H), 4.14-4.18 (m, 1H), 4.18-4.25 (m, 1H), 4.77 (br d, J=8.44Hz, 1H), 4.98-5.06 (m, 1H), 5.10 (br s, 2H), 5.31 (br s, 1H), 5.34-5.45 (m, 1H), 6.29-6.39 (m, 1H), 7.29-7.33 (m, 1H), 7.36 (d, J=4.40Hz, 4H)
LCMS (m/z 657 [M+H]⁺)

### Synthesis of Compound 50c

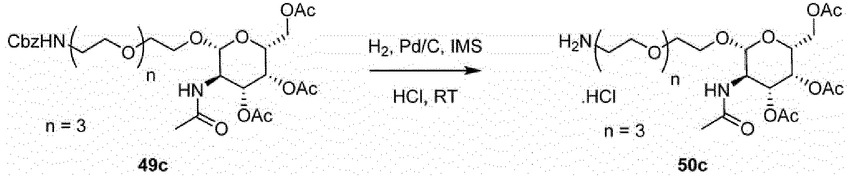

To a solution of compound 49c (0.45 g, 0.69 mmol) in IMS (12 mL), 1,4-dioxane solution of 4 M hydrogen chloride (225 µL, 0.90 mmol) and 10% palladium-carbon (50% wet product; 0.083 g) were added in a nitrogen atmosphere. The mixture was stirred in a hydrogen atmosphere at room temperature for 2.5 hours. The mixture was filtered through a Celite (Registered Trademark), and washed with IMS. The filtrate was concentrated under reduced pressure to afford compound 50c (0.40 g).
¹H NMR (600 MHz, DMSO-d₆) δ ppm 1.78 (s, 3H), 1.89 (s, 3H), 2.00 (s, 3H), 2.11 (s, 3H), 2.94-3.01 (m, 2H), 3.48-3.62 (m, 13H), 3.76-3.82 (m, 1H), 3.85-3.93 (m, 1H), 3.99-4.08 (m, 3H), 4.55 (d, J=8.44Hz, 1H), 4.94-5.01 (m, 1H), 5.22 (d, J=3.67Hz, 1H), 7.75-7.88 (m, 4H)
LCMS (m/z 423 [M+H]⁺)

### Synthesis of Compound 51c

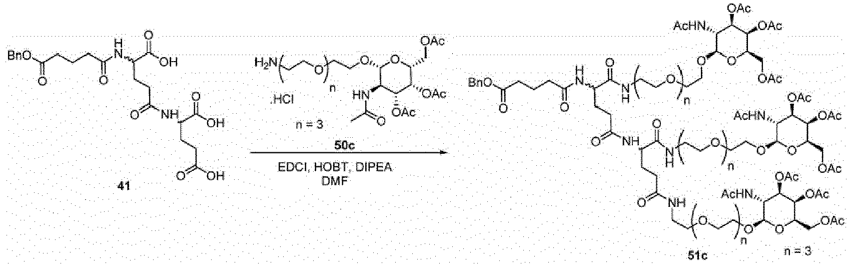

To a solution of compound 50c (379.8 mg, 0.679 mmol) and compound 41 (47.2 mg, 0.10 mmol) in DMF (2 mL), HOBt (104 mg, 0.68 mmol), EDCI (130 mg, 0.68 mmol), and DIPEA (119 µL, 0.68 mmol) were sequentially added. The resulting mixture was stirred at room temperature for 19 hours. The mixture was purified by preparative HPLC to afford compound 51c (53.8 mg).
¹H NMR (600 MHz, DMSO-d₆) δ ppm 1.61-1.73 (m, 2H), 1.77 (s, 9H), 1.79-1.85 (m, 2H), 1.89 (s, 9H), 2.00 (s, 9H), 2.03-2.08 (m, 2H), 2.10 (s, 9H), 2.12-2.22 (m, 4H), 2.33-2.37 (m, 2H), 3.07-3.23 (m, 4H), 3.34-3.42 (m, 7H), 3.43-3.55 (m, 30H), 3.55-3.62 (m, 4H), 3.75-3.81 (m, 3H), 3.85-3.92 (m, 3H), 3.99-4.07 (m, 9H), 4.13-4.24 (m, 2H), 4.56 (d, J=8.80Hz, 3H), 4.97 (dd, J=11.00, 3.30Hz, 3H), 5.06-5.11 (m, 2H), 5.21 (d, J=3.30Hz, 3H), 7.30-7.39 (m, 5H), 7.62-7.84 (m, 5H), 7.85-7.95 (m, 3H)
LCMS (m/z 1994/1995 [M+H]⁺)

### Synthesis of Compound 52c

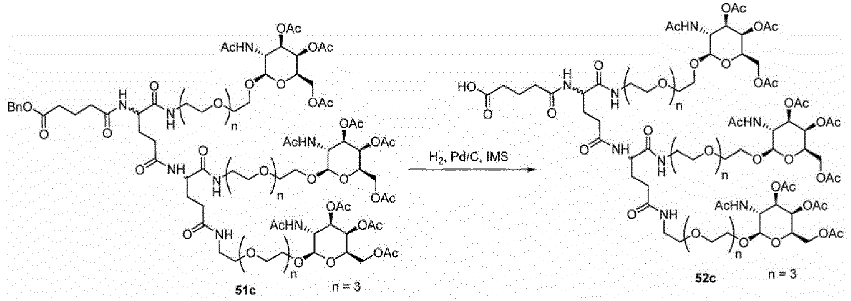

To a solution of compound 51c (84 mg, 0.04 mmol) in IMS (17 mL), 10% palladium-carbon (50% wet product; 27.3 mg) was added in a nitrogen atmosphere. The mixture was stirred in a hydrogen atmosphere at room temperature for 2.5 hours. The mixture was filtered through a Celite (Registered Trademark), and washed with IMS. The filtrate was concentrated under reduced pressure to afford compound 52c (78.9 mg).
¹H NMR (600 MHz, DMSO-d₆) δ ppm 1.60-1.75 (m, 4H), 1.78 (s, 9H), 1.80-1.87 (m, 2H), 1.89 (s, 9H), 2.00 (s, 9H), 2.03-2.32 (m, 17H), 3.12-3.23 (m, 4H), 3.36-3.42 (m, 7H), 3.43-3.63 (m, 34H), 3.74-3.81 (m, 3H), 3.84-3.92 (m, 3H), 3.99-4.07 (m, 9H), 4.09-4.22 (m, 2H), 4.57 (br d, J=8.44Hz, 3H), 4.98 (dd, J=11.19, 3.12Hz, 3H), 5.21 (d, J=3.30Hz, 3H), 7.61-8.05 (m, 8H), 12.01 (br s, 1H)
LCMS (m/z 1904/1905 [M+H]⁺)

### Synthesis of Compound 53c

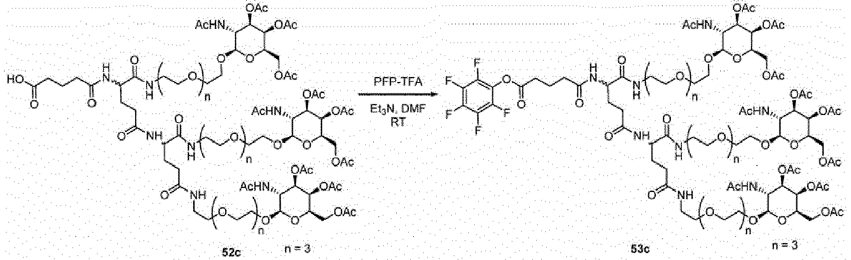

To a solution of compound 52c (77.3 mg, 0.04 mmol) in DMF (3 mL), triethylamine (45 µL, 0.33 mmol) and then pentafluorophenyl trifluoroacetate (27 µL, 0.16 mmol) were added dropwise in a nitrogen atmosphere. The resulting mixture was stirred at room temperature for 1.5 hours and poured into water, brine was added thereto, and extraction was then performed with EtOAc. The organic layer was washed sequentially with saturated aqueous sodium hydrogen carbonate solution, 1 M aqueous sodium hydrogen sulfate solution, and then brine. The organic layer was dried over anhydrous sodium sulfate, filtered, and then concentrated under reduced pressure. The residue was transferred into a sample vial containing DCM/MeOH, the solvent was evaporated, and the resultant was dried under reduced pressure at 40°C for 2 hours to afford a residue. This was dissolved in EtOAc (20 mL), washed with 4% aqueous lithium chloride solution (5 mL) and then with brine (5 mL), dried over anhydrous sodium sulfate, filtered, and then subjected to evaporation. The residue was transferred into a sample vial containing EtOAc, subjected to evaporation, and dried under reduced pressure at 40°C. DCM was added to the residue, the resultant was concentrated under reduced pressure, and the residue was dried under reduced pressure at 40°C for 4 hours to afford compound 53c (19.4 mg).
¹H NMR (600 MHz, DMSO-d₆) δ ppm:
1.59-1.73 (m, 2H), 1.77 (s, 9H), 1.85-1.95 (m, 13H), 2.00 (s, 9H), 2.10 (s, 9H), 2.12-2.34 (m, 6H), 2.77-2.89 (m, 2H), 3.11-3.23 (m, 4H), 3.35-3.44 (m, 7H), 3.45-3.63 (m, 34H), 3.75-3.80 (m, 3H), 3.84-3.91 (m, 3H), 4.00-4.07 (m, 9H), 4.16-4.26 (m, 2H), 4.56 (d, J=8.80Hz, 3H), 4.97 (dd, J=11.37, 3.30Hz, 3H), 5.21 (d, J=3.67Hz, 3H), 7.62-8.03 (m, 8H).
QC LCMS (m/z 1035.97 [M+2H]²⁺)

### Examples 9 to 12: Synthesis of Nucleic Acid Complexes 9 to 12

To the sense strand (215 nmol) of B2M (β2-microglobulin)-targeting siRNA, sodium tetraborate buffer at pH 8.5 and compound 28 or 44 (1000 nmol) dissolved in DMSO were added, and the resultant was stirred at room temperature. After adding water to the reaction mixture, purification was performed with a Vivaspin 3K (Sartorius AG). To the crude product obtained, 28% aqueous ammonia in a volume five times that of the crude product was added, and the resultant was left to stand at room temperature for 2 hours. After adding water to the reaction mixture, purification was repeatedly performed with a Vivaspin 3K (Sartorius AG) to afford nucleic acid complexes 9 to 12. The molecular weights of the nucleic acid complexes synthesized in Examples shown here were determined by ESI-MS or MALDI-TOF-MS. Table 5 shows the sequences of the sense strands used, the sequence of the antisense strand, and their molecular weights.

**[Table 5]**

| Name of sequence | Sequence (5'-3') | Theoretical molecular weight | Found |
|---|---|---|---|
| siB2M-s | A(F)^G(M)^G(F)A(M)C(F)U(M)G(F) G(M)U(F)C(M)U(F)U(F)U(M)C(F) U(M)A(F)U(M)A(F)U(M)^C(F)^U(M) | 6838.5 | 6838.9 |
| siB2M-s_3'NH2 | A(F)^G(M)^G(F)A(M)C(F)U(M)G(F) G(M)U(F)C(M)U(F)U(F)U(M)C(F) U(M)A(F)U(M)A(F)U(M)^C(F)^U(M)-NH2 | 7017.7 | 7018.5 |
| siB2M-s_5'NH2 | NH2-A(F)^G(M)^G(F)A(M)C(F)U(M) G(F)G(M)U(F)C(M)U(F)U(F)U(M)C(F) U(M)A(F)U(M)A(F)U(M)^C(F)^U(M) | 7017.7 | 7018.9 |
| siB2M-s^_3'NH2 | A(F)^G(M)^G(F)A(M)C(F)U(M)G(F) G(M)U(F)C(M)U(F)U(F)U(M)C(F)U(M) A(F)U(M)A(F)U(M)^C(F)^U(M)^-NH2 | 7033.7 | 7035.6 |
| siB2M-as | A(F)^G(M)^A(F)U(M)A(F)U(M)A(F) G(M)A(F)A(M)A(M)G(F)A(M)C(F)C(M) A(F)G(M)U(F)C(M)C(F)U(M)^U(F)^G(M) | 7633.1 | 7634.3 |

Table 6 shows the sequences and molecular weights of the nucleic acid complexes.

**[Table 6]**

| Compound | Sequence (5'-3') | Theoretical molecular weight | Found |
|---|---|---|---|
| Nucleic acid complex 9 | siB2M-s-Compound 28 | 8728.6 | 8729.2 |
| Nucleic acid complex 10 | siB2M-s-Compound 44 | 8297.1 | 8299.8 |
| Nucleic acid complex 11 | Compound 44-siB2M-s | 8297.1 | 8297.2 |
| Nucleic acid complex 12 | siB2M-s^-Compound 44 | 8313.2 | 8311.0 |

### Nucleic Acid Complex 9

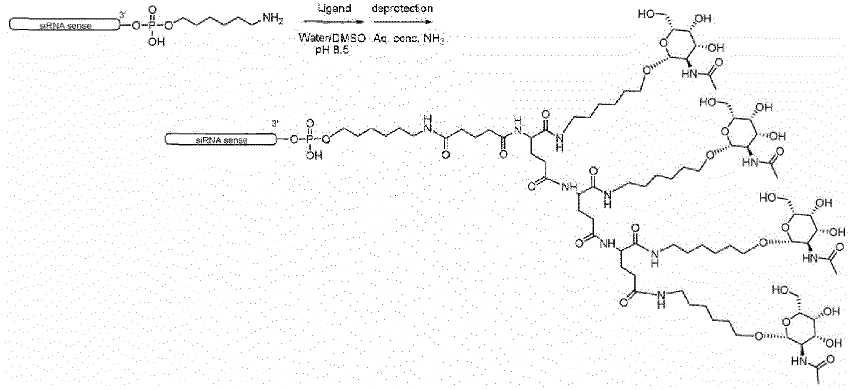

### Nucleic Acid Complex 10

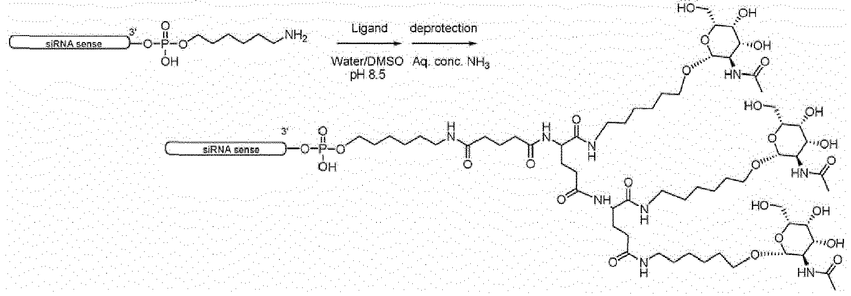

### Nucleic Acid Complex 11

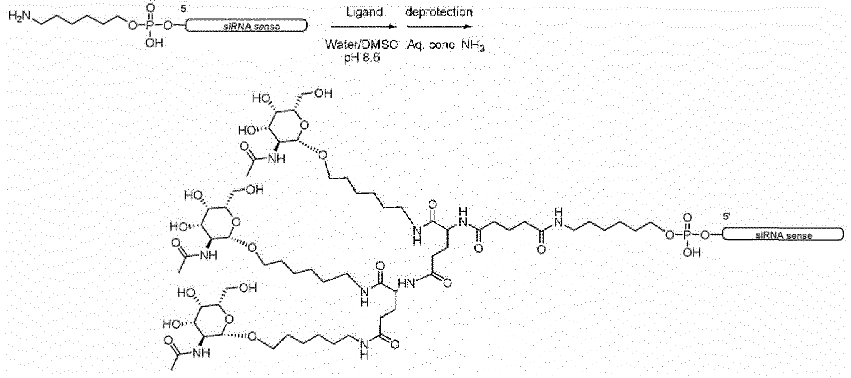

### Nucleic Acid Complex 12

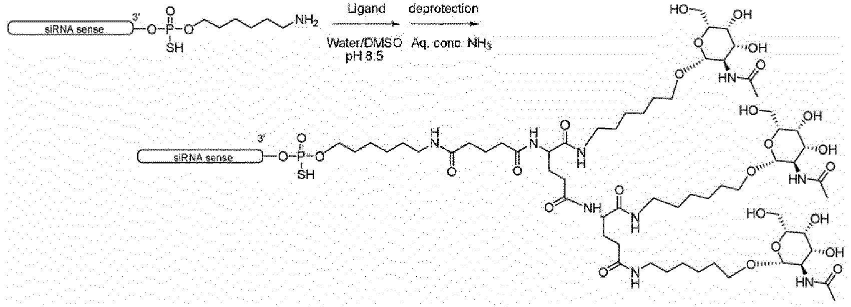

### Examples 13 to 16: Preparation of Nucleic Acid Complexes 13 to 16 (si-RNA)

By adding the antisense strand (siB2M-as) to an equimolar amount of each of nucleic acid complexes 9 to 12, si-RNAs targeting B2M were prepared. Table 7 shows the sense strands and antisense strands of the si-RNAs.

**[Table 7]**

| siRNA | Sense strand | Antisense strand |
|---|---|---|
| siRNA-1 | siB2M-s | siB2M-as |
| siRNA-2 (Nucleic acid complex 13) | Nucleic acid complex 9 | siB2M-as |
| siRNA-3 (Nucleic acid complex 14) | Nucleic acid complex 10 | siB2M-as |
| siRNA-4 (Nucleic acid complex 15) | Nucleic acid complex 11 | siB2M-as |
| siRNA-5 (Nucleic acid complex 16) | Nucleic acid complex 12 | siB2M-as |

### <Test Example 4>

The nucleic acid complexes (si-RNA) prepared in Examples 13 to 16 were each subcutaneously administered to BALB/c mice (three mice per group) at 1.0 mg/kg or 5.0 mg/kg, and the liver was collected 7 days after the administration. The mRNA expression levels of liver B2M and GAPDH (glyceraldehyde-3-phosphate dehydrogenase) as an internal control were measured through qPCR with use of a TaqMan Probe (Applied Biosystems). The B2M mRNA expression level in the mouse liver for the untreated group (Control) was defined as 100%, and the B2M mRNA expression levels (relative values) for the groups with administration of any one of the nucleic acid complexes were calculated. Table 8 shows the results.

**[Table 8]**

| siRNA | Dose (mg/kg) | B2M mRNA expression level |
|---|---|---|
| Untreated | - | 100% |
| siRNA-1 | 5.0 | 98% |
| siRNA-2 | 1.0 | 35% |
| siRNA-3 | 1.0 | 21% |
| siRNA-4 | 1.0 | 24% |
| siRNA-5 | 1.0 | 24% |
| siRNA-2 | 5.0 | 14% |
| siRNA-3 | 5.0 | 16% |
| siRNA-4 | 5.0 | 13% |
| siRNA-5 | 5.0 | 13% |

As is clear from the results shown in Table 8, siRNA-2, siRNA-3, siRNA-4, and siRNA-5 exhibited dose-dependent gene silencing effect. While almost no gene silencing effect was found for siRNA-1, which contained no sugar ligand, even at 5.0 mg/kg, the siRNAs containing a sugar ligand exhibited high gene silencing effect even at 1.0 mg/kg.

### Examples 17 to 19: Synthesis of Nucleic Acid Complexes 17 to 19

Nucleic acid complexes were prepared with the same method as in Examples 9 to 11, except that Gapmer-type LNA antisense having a 3-10-3 motif (ASO; ISIS-549148) was used as a nucleic acid and compound 44, 47, or 53a was used. Table 9 shows the sequence of the sense strand used, the sequence of the antisense strand, and their molecular weights.

**[Table 9]**

| Name of sequence | Sequence (5'-3') | Theoretical molecular weight | Found |
|---|---|---|---|
| ASO_3'Cy3 | G(L)^G(L)^5(L)^t^a^5(x)^t^a^5(x)^g^5(x) ^5(x)^g^T(L)^5(L)^A(L)-Cy3 | 6109.4 | 6110.4 |
| ASO_5'NH2_3'Cy3 | 5'NH2_G(L)^G(L)^5(L)^t^a^5(x)^t^a^5(x) ^g^5(x)^5(x)^g^T(L)^5(L)^A(L)-Cy3 | 6288.5 | 6290.8 |

| | | | |
|---|---|---|---|
| 5(L):LNA 5-Methylcytosine, 5(x)= DNA 5-Methylcytosine | | | |

Table 10 shows the sequences and molecular weights of the nucleic acid complexes.

**[Table 10]**

| Compound | Sequence (5'-3') | Theoretical molecular weight | Found |
|---|---|---|---|
| Nucleic acid complex 17 | Compound 44-ASO_3'Cy3 | 7569.9 | 7586.8 |
| Nucleic acid complex 18 | Compound 47-ASO_3'Cy3 | 7446.7 | 7463.5 |
| Nucleic acid complex 19 | Compound 53a-ASO_3'Cy3 | 7533.7 | 7549.2 |

### Nucleic Acid Complex 17

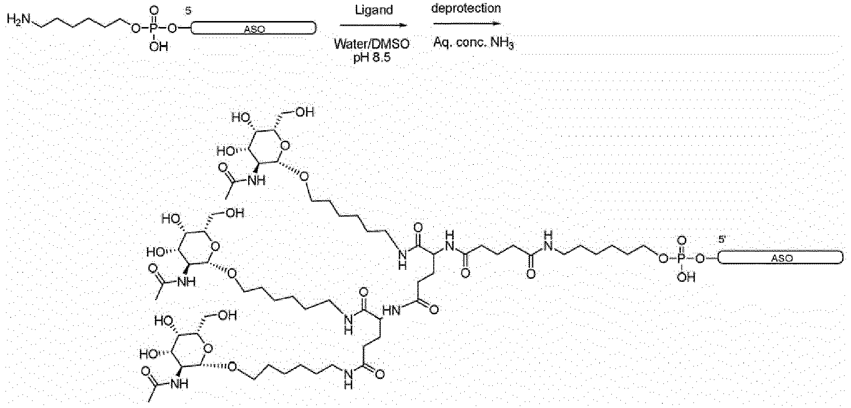

### Nucleic Acid Complex 18

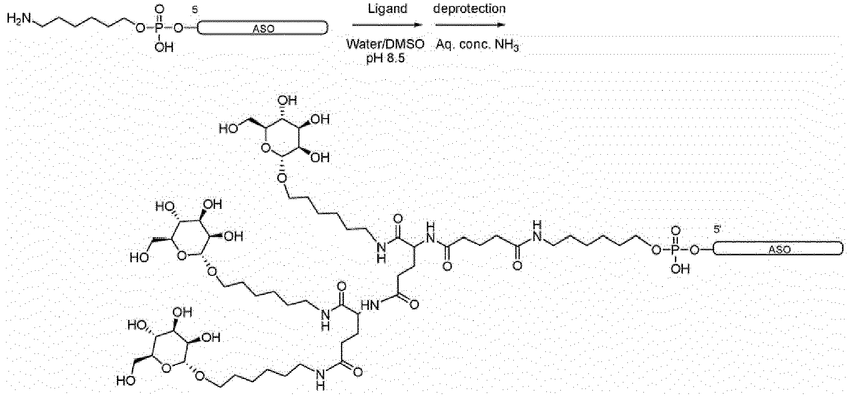

### Nucleic Acid Complex 19

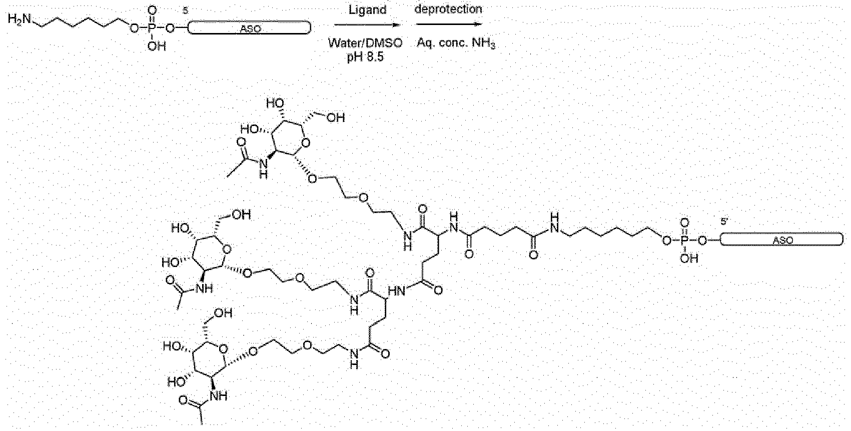

### <Test Example 5>

### Evaluation of in vitro Uptake Activity of Human CD206-Expressing Lenti-X 293T Cells for Nucleic Acid Complexes

Evaluation of nucleic acid complexes 17 to 19 was carried out in the same manner as in Test Example 1. In Test Example 5, the fluorescence intensity in a well with addition of ASO_3'Cy3, which is a nucleic acid without modification with a sugar ligand, was defined as 1, and fluorescence intensity in each well with addition of any one of the nucleic acid complexes was calculated as a relative value.

Table 11 shows the results. It was revealed from the results that, as compared with ASO_3'Cy3, which is a nucleic acid containing no sugar ligand, nucleic acid complexes 17 and 19, which had GalNAc, were not effectively taken up by CD206-expressing cells, whereas nucleic acid complex 18, which had mannose, was effectively taken up by CD206-expressing cells.

**[Table 111**

| Specimen No. | ASO_3'Cy3 | Nucleic acid complex | | |
|---|---|---|---|---|
| | | 17 | 18 | 19 |
| Ligand | - | GalNAc | Mannose | GalNAc |
| Relative value of fluorescence intensity (ASO 3'Cy3 = 1.0) | 1.0 | 1.1 | 15.8 | 1.0 |

### <Test Example 6>

### Evaluation of in vitro Uptake Activity of Human ASGR1-Expressing Lenti-X 293T Cells for Nucleic Acid Complexes

Evaluation of nucleic acid complexes 17 to 19 was carried out in the same manner as in Test Example 2. In Test Example 6, the fluorescence intensity in a well with addition of ASO_3'Cy3, which is a nucleic acid without modification with a sugar ligand, was defined as 1, and fluorescence intensity in each well with addition of any one of the nucleic acid complexes was calculated as a relative value.

Table 12 shows the results. It was revealed from the results that, as compared with ASO_3'Cy3, which is a nucleic acid without modification with a sugar ligand, nucleic acid complex 18, which had mannose, was not effectively taken up by ASGR1-expressing cells, whereas nucleic acid complexes 17 and 19, which had GalNAc, were effectively taken up by ASGR1-expressing cells.

**[Table 12]**

| Specimen No. | ASO 3'Cy3 | Nucleic acid complex | | |
|---|---|---|---|---|
| | | 17 | 18 | 19 |
| Ligand | - | GalNAc | Mannose | GalNAc |
| Relative value of fluorescence intensity (ASO_3'Cy3 = 1.0) | 1.0 | 24.6 | 0.6 | 10.7 |

## Claims

1. A nucleic acid complex represented by the formula (I): 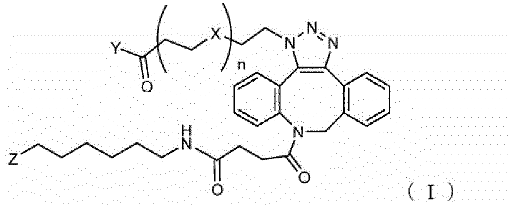 wherein X is CH₂ or O;
Y is a sugar ligand having mannose or GalNAc;
n is an integer of 1 to 8; and
Z is a group comprising an oligonucleotide,
or a pharmaceutically acceptable salt thereof.

2. A nucleic acid complex represented by the formula (II): 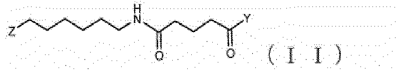 wherein Y is a sugar ligand having mannose or GalNAc; and
Z is a group comprising an oligonucleotide,
or a pharmaceutically acceptable salt thereof.

3. A nucleic acid complex represented by the formula (III): 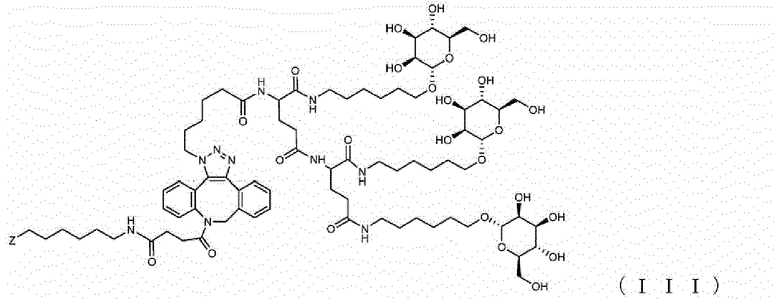 wherein Z is a group comprising an oligonucleotide,
or a pharmaceutically acceptable salt thereof.

4. A nucleic acid complex represented by the formula (IV): 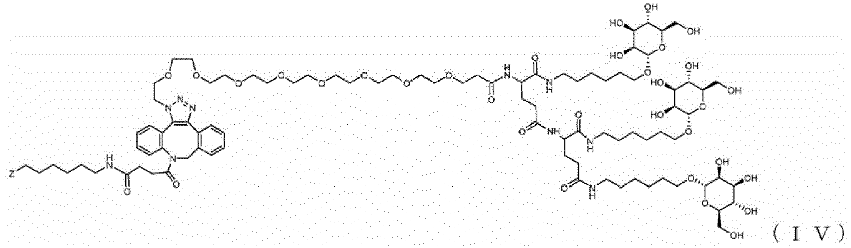 wherein Z is a group comprising an oligonucleotide,
or a pharmaceutically acceptable salt thereof.

5. A nucleic acid complex represented by the formula (V): 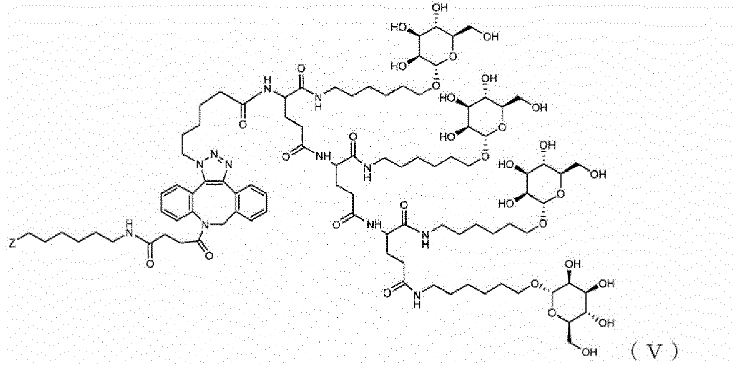 wherein Z is a group comprising an oligonucleotide,
or a pharmaceutically acceptable salt thereof.

6. A nucleic acid complex represented by the formula (VI): 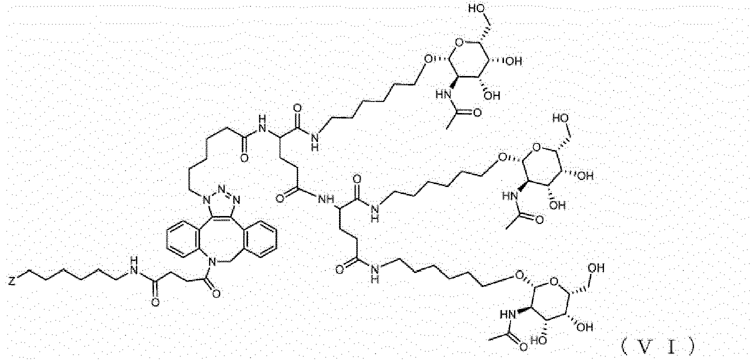 wherein Z is a group comprising an oligonucleotide,
or a pharmaceutically acceptable salt thereof.

7. A nucleic acid complex represented by the formula (VII): 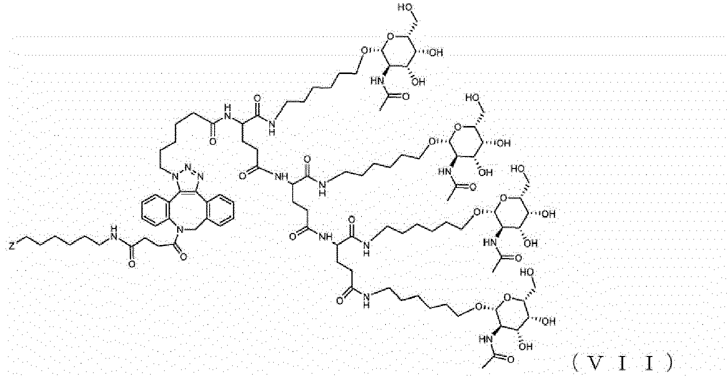 wherein Z is a group comprising an oligonucleotide,
or a pharmaceutically acceptable salt thereof.

8. A nucleic acid complex represented by the formula (VIII): 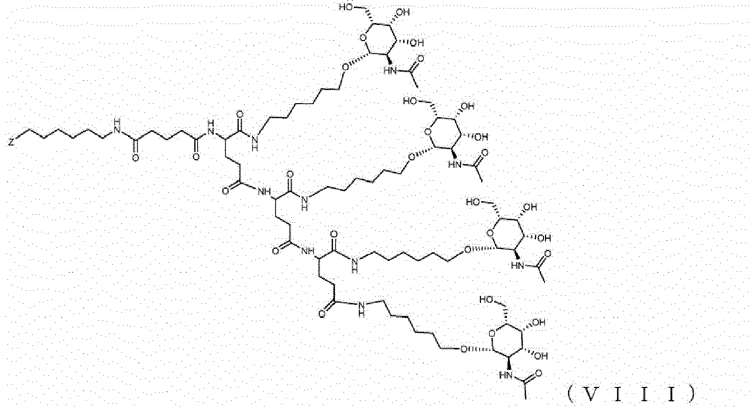 wherein Z is a group comprising an oligonucleotide,
or a pharmaceutically acceptable salt thereof.

9. A nucleic acid complex represented by the formula (IX): 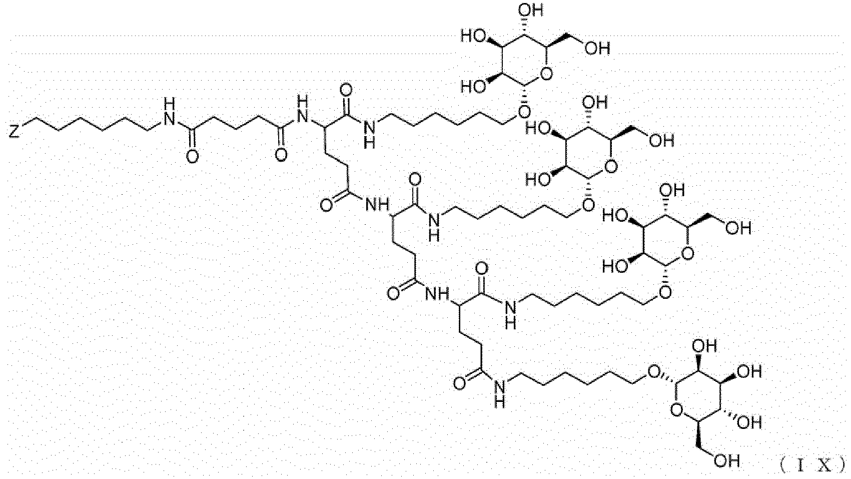 wherein Z is a group comprising an oligonucleotide,
or a pharmaceutically acceptable salt thereof.

10. A nucleic acid complex represented by the formula (X): 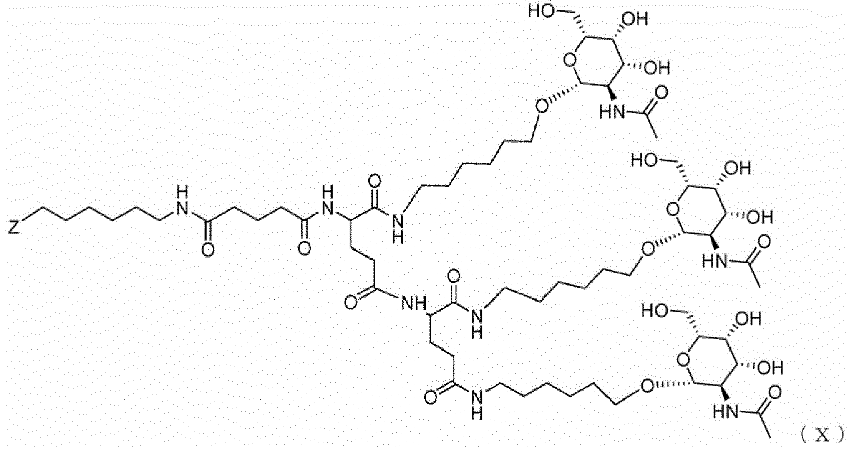 wherein Z is a group comprising an oligonucleotide,
or a pharmaceutically acceptable salt thereof.

11. A nucleic acid complex represented by the formula (XI): 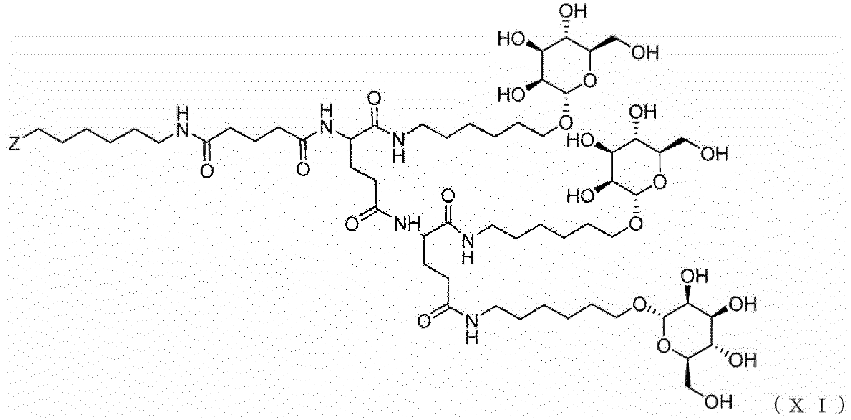 wherein Z is a group comprising an oligonucleotide,
or a pharmaceutically acceptable salt thereof.

12. A nucleic acid complex represented by the formula (XII): 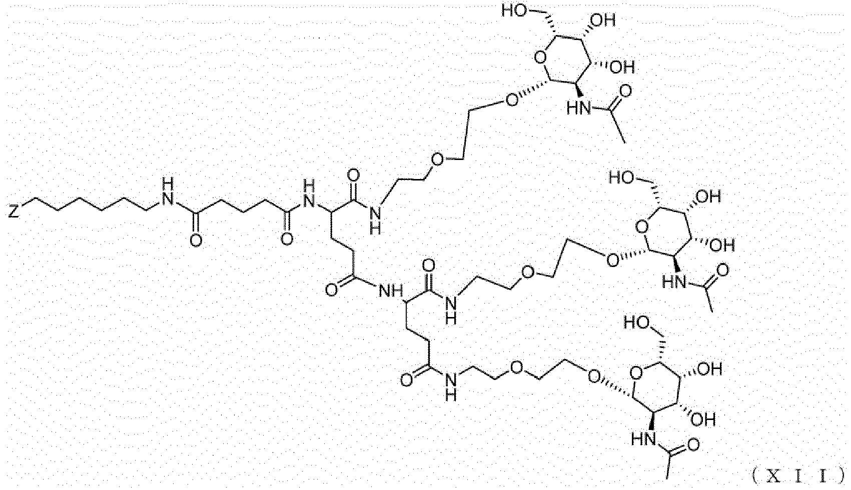 wherein Z is a group comprising an oligonucleotide,
or a pharmaceutically acceptable salt thereof.

13. A nucleic acid complex represented by the formula (XIII): 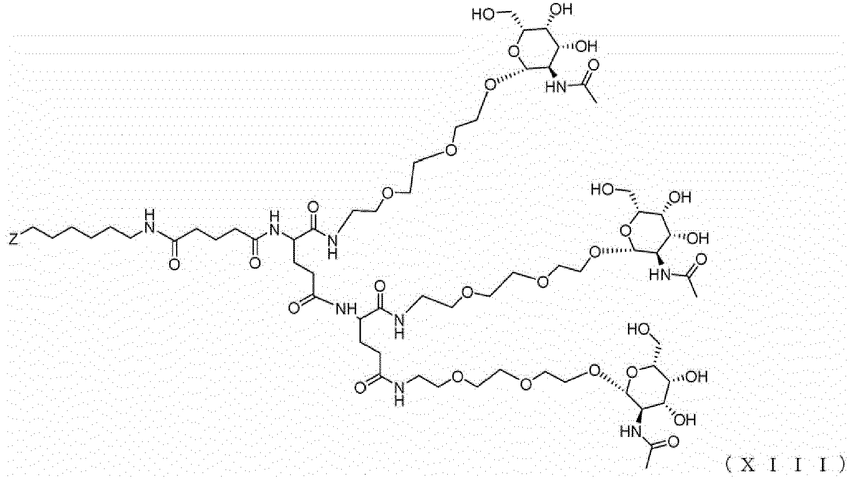 wherein Z is a group comprising an oligonucleotide,
or a pharmaceutically acceptable salt thereof.

14. A nucleic acid complex represented by the formula (XIV): 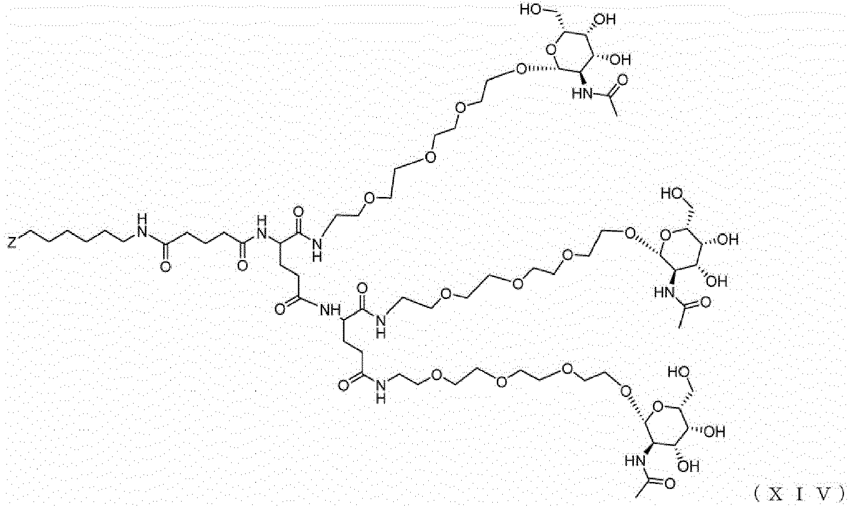 wherein Z is a group comprising an oligonucleotide,
or a pharmaceutically acceptable salt thereof.

15. The nucleic acid complex according to any one of claims 1 to 14, wherein the oligonucleotide is single-stranded.

16. The nucleic acid complex according to claim 15, wherein the oligonucleotide is bound via a 3' end.

17. The nucleic acid complex according to claim 15, wherein the oligonucleotide is bound via a 5' end.

18. The nucleic acid complex according to any one of claims 1 to 14, wherein the oligonucleotide is double-stranded.

19. The nucleic acid complex according to claim 18, wherein the oligonucleotide is bound via a 3' end of one strand.

20. The nucleic acid complex according to claim 18, wherein the oligonucleotide is bound via a 5' end of one strand.

21. A pharmaceutical composition comprising the nucleic acid complex according to any one of claims 1 to 20.

22. The pharmaceutical composition according to claim 21, wherein the pharmaceutical composition regulates expression of a target gene in a cell.

23. The pharmaceutical composition according to claim 22, wherein the cell is a dendritic cell, a macrophage, or a liver parenchymal cell.


SEQUENCE LISTING

<110>  Eisai R&D Management Co.,Ltd.

<120>  NUCLEIC ACID COMPLEX AND PHARMACEUTICAL COMPOSITION CONPRISING
       THE SAME

<130>  FP20-1030-00

<150>  JP 2020-013997
<151>  2020-01-30

<160>  3

<170>  PatentIn version 3.5

<210>  1
<211>  21
<212>  RNA
<213>  Artificial Sequence

<220>
<223>  siB2M-s

<400>  1
aggacugguc uuucuauauc u                                                21


<210>  2
<211>  23
<212>  RNA
<213>  Artificial Sequence

<220>
<223>  siB2M-as

<400>  2
agauauagaa agaccagucc uug                                              23


<210>  3
<211>  16
<212>  DNA
<213>  Artificial Sequence

<220>
<223>  ASO

<400>  3
ggctactacg ccgtca                                                      16